# EUROPÄISCHE PATENTSCHRIFT

(11) **EP 0 549 943 B1**
(45) Veröffentlichungstag und Bekanntmachung des Hinweises auf die Patenterteilung: **22.05.1996**
(21) Anmeldenummer: 92121239.5
(22) Anmeldetag: 14.12.1992
(51) Int. Cl.: C07D 401/12, A01N 43/52, C07D 417/12, C07D 403/12

(54) **Benzimidazolsulfonsäure-Derivate als Mikrobizide**
Benzimidazole sulfonic acid derivatives as microbicides
Dérivés d'acides sulphoniques de benzimidazole comme microbizides

(30) Priorität: 16.12.1991 CH 3706/91
(43) Veröffentlichungstag der Anmeldung: 07.07.1993
(73) Patentinhaber: CIBA-GEIGY AG, CH-4002 Basel (CH)
(72) Erfinder: Hubele, Adolf, Dr., CH-4312 Magden (CH); Hostettler, Bernhard, Dr., CH-8044 Zürich (CH); Sutter, Marius, Dr., CH-4102 Binningen (CH); Müller, Urs, Dr., CH-4142 Münchenstein (CH)
(74) Vertreter: Zumstein, Fritz, Dr.

(56) Entgegenhaltungen:
- EP-A- 0 087 375
- EP-A- 0 152 360
- EP-A- 0 181 826
- FR-A- 2 607 811

## Beschreibung

Die vorliegende Erfindung betrifft neue Benzimidazolsulfonsäure-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung oder Verhütung eines Befalls von Pflanzen durch phytopathogene Mikroorganismen, vorzugsweise Fungi.

Die erfindungsgemässe Verbindungen entsprechen der allgemeinen Formel I 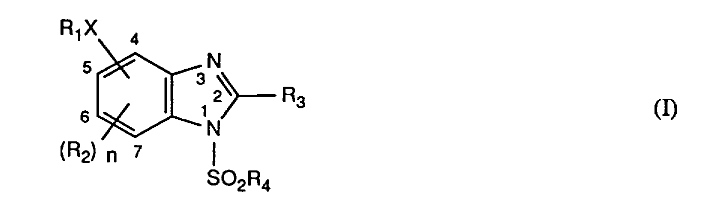 worin die R₄SO₂-Gruppe die 1- oder 3-Position besetzt und im Verhältnis zu den Substituenten R₁X- und R₂ reine oder gemischte Stellungsisomere bildet, und worin die Substituenten folgende Bedeutung haben:
- R₁ =: ungesättigter 5-gliedriger Heterocyclus mit maximal zwei Heteroatomen N und/oder S, oder ein ungesättigter 6-gliedriger Heterocyclus mit maximal zwei N-Atomen, wobei jeder der Heterocyclen unsubstituiert oder durch mindestens einen der Substituenten Halogen, C₁-C₃-Alkyl, C₁-C₃-Alkoxy, C₁-C₃-Haloalkyl, C₁-C₃-Haloalkoxy, COOC₁-C₃-Alkyl, C₃-C₆-Cycloalkyl, CN und Nitro substituiert sein kann;
- R₂ =: gleich oder verschieden Halogen, C₁-C₃-Alkyl, C₁-C₃-Alkoxy, C₁-C₃-Haloalkyl, C₁-C₃-Haloalkoxy, Nitro;
- R₃ =: Cyano, -CS-NH₂ oder -C(SR')=NH, wobei R'C₁-C₄-Alkyl, C₁-C₄-Haloalkyl, unsubstituiertes oder durch Halogen und/oder CF₃ substituiertes Benzyl darstellt;
- R₄ =: C₁-C₄-Alkyl, C₃-C₆-Cycloalkyl, -N(R'')(R'''), wobei R'' und R''' gleiches oder verschiedenes C₁-C₃-Alkyl darstellt;
- X =: Sauerstoff oder Schwefel; und
- n =: eine ganze Zahl 0, 1 oder 2.

Unter dem Begriff Alkyl selbst oder als Bestandteil eines anderen Substituenten wie Haloalkyl, Alkoxy oder Haloalkoxy, sind je nach Zahl der angegebenen Kohlenstoffatome beispielsweise die folgenden geradkettigen oder verzweigten Gruppen zu verstehen: Methyl, Ethyl, Propyl, Butyl sowie ihre Isomeren, Isopropyl, Isobutyl, sek. Butyl, tert.-Butyl. Halogen und Halo stehen für Fluor, Chlor, Brom oder Jod. Haloalkoxy steht somit für einen einfach bis perhalogenierten Alkoxyrest, wie z.B. OCH₂F, OCHF₂, OCHFCH₃, OCH₂CH₂Br, OCF₂CHFCl usw.
Der Begriff Cycloalkyl steht für Cyclopropyl, Cyclobutyl, Cyclopentyl oder Cyclohexyl.

Unter den angegebenen ungesättigten 5-gliedrigen Heterocyclus sind Pyrrol, Thiophen, Pyrazol, Imidazol, Thiazol und Isothiazol zu verstehen, unter dem entsprechenden 6-gliedrigen Heterocyclus Pyridin, Pyrimidin, Pyrazin und Pyridazin.

Die Verbindungen der Formel I sind bei Raumtemperatur stabile Oele oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende mikrobizide, insbesondere fungizide Wirkung, sondern auch durch besonders gute Pflanzenverträglichkeit aus.

Im Umgang der Formel I sind jene Verbindungen von Wichtigkeit, worin R₁ einen unsubstituierten oder substituierten Pyridin-, Pyrimidin-, Pyrazin- oder Pyridazinring bedeutet [Untergruppe Ib], und unter diesen solche, worin der 6-gliedrige Heterocyclus durch ein bis drei Substituenten ausgewählt aus Halogen, Methyl, Ethyl, Isopropyl, Methoxy, C₁-C₂-Haloalkyl mit F und/oder Cl als Halogen, CF₃O, CHF₂O, Nitro, substituiert ist. [Untergruppe Ic].

Eine der besonders wichtigen Stoffgruppen im Umfang der Untergruppe Ic sind jene, worin der 6-gliedrige Heterocyclus unsubstituiertes oder ein- bis dreifach substituiertes Pyridin ist und R₂ gleich oder verschieden Fluor, Chlor, Brom, Methyl, Methoxy, CF₃, CF₃O, CHF₂O bedeutet, mit n = 0, 1 oder 2 [Untergruppe Id], besonders solche mit n = 0 oder 1 [Untergruppe Ie]. Besonders wichtige Stoffgruppen im Umfang der Untergruppe Id sind jene mit R₂ gleich Halogen oder C₁-C₂-Haloalkyl [Untergruppe Id'], vor allem solche, worin der Pyridinring mit CF₃ substituiert ist Untergruppe Id''].

Eine andere wichtige Stoffgruppe im Umfang der Untergruppe Ic sind jene, worin der 6-gliedrige Heterocyclus unsubstituiertes oder halosubstituiertes oder haloalkylsubstituiertes Pyrimidin ist und R₂ Fluor, Chlor, Brom, Methyl, Methoxy, CF₃ bedeutet, wobei n = 0, 1 oder 2 ist [Untergruppe If]. Im Umfang der Untergruppe If sind besonders wichtig jene Verbindungen, worin R₂ Fluor, Chlor, Brom, Methyl, Methoxy, CF₃ bedeutet, mit n = 0, 1 oder 2 [Untergruppe If'], sowie die Stoffgruppe worin der Pyrimidinring in 4-Stellung verknüpft ist [Untergruppe If''].

Im Umfang der Untergruppe Id, worin R₁ einen 6-gliedrigen Heterocyclus bedeutet, sind solche Verbindungen bevorzugt, worin R₄ Methyl, Dimethylamino, Cyclopropyl, Cyclopentyl oder Cyclohexyl bedeutet [Untergruppe Ig], insbesondere solche, worin R₄ Methyl ist [Untergruppe Ig'] oder solche, worin R₄ Dimethylamino bedeutet [Untergruppe Ig''].

Im Umfang der Formel I sind weiterhin jene Verbindungen von Wichtigkeit, worin X Sauerstoff ist, solche worin X Schwefel ist und R₁ einen unsubstituierten oder substituierten Pyrrol-, Thiophen-, Thiazol-, Isothiazol-, Imidazol- oder Pyrazol-Ring bedeutet [Untergruppe Ii].

Unter diesen Verbindungen sind solche bevorzugt, worin der 5-gliedrige Heterocyclus durch Halogen und/oder Methyl substituiert ist [Untergruppe Ij], insbesondere solche, worin R₂ gleich oder verschieden Halogen, Methyl, Methoxy, CF₃, CF₃O, CHF₂O bedeutet, mit n = 0, 1 oder 2 [Untergruppe Ik]. Wichtig sind z.B. Verbindungen ohne Substituenten R₂ (d.h. n = 0).

Im Umfang der Formel I sind jene Verbindungen von Wichtigkeit, worin R₃ Cyano oder C(S)NH₂ ist [Untergruppe Im] oder solche, worin XR₁ die 5/6-Stellung in der Benzimidazolring einnimmt [Untergruppe In].

Wichtig sind Verbindungen der Formel I, worin X Sauerstoff bedeutet, mit Einschluss der Untergruppen Ib bis In. Bevorzugte Einzelverbindungen im Umfang der Untergruppe Id sind beispielsweise
a) 1(3)-(Dimethylaminosulfonyl)-2-cyano-4-bromo-6-(3,5-dichloropyridin-2-yloxy)-benzimidazol [Verb. 1.23];
b) 1(3)-Methansulfonyl-2-cyano-6-(5-trifluormethylpyridin-2-yloxy)-benzimidazol [Verb. 1.2];
c) 1(3)-Methansulfonyl-2-cyano-6-(3-chloro-5-trifluormethylpyridin-2-yloxy)-benzimidazol [Verb. 1.66];
d) 1(3)-Cyclopropansulfonyl-2-cyano-6-(3-chloro-5-trifluormethylpyridin-2-yloxy)-benzimidazol [Verb. 1.74].

Die Verbindungen der Formel I lassen sich herstellen durch Reaktion einer Verbindung der Formel II 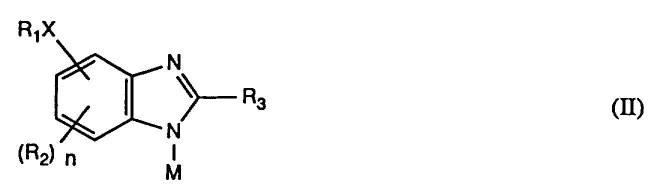 mit einer Verbindung der Formel III

Q-SO₂-R₄ (III)

worin R₁, R₂, R₃, R₄, X und n die unter Formel I angegebenen Bedeutungen besitzen und M Wasserstoff oder ein Alkalimetall, bevorzugt Na, K oder Li, und Q ein Halogenatom, bevorzugt Chlor oder Brom, oder den Rest O-SO₂-R₄ darstellen, in einem inerten Lösungsmittel, gegebenenfalls in Gegenwart einer Base, bei Temperaturen von -30° bis +180°C, bevorzugt -10° bis 80°C, unter Normaldruck, vermindertem oder erhöhtem Druck, bevorzugt Normaldruck.

Als Lösungsmittel kommen vor allem polare Reaktionsmedien in Frage, wie Ketone allein (z.B. Aceton, Methylethylketon, tert. Butylmethylketon) oder in Gemischen mit Ethern (wie Diethylether, Dioxan, Tetrahydrofuran, 1,2-Dimethoxyethan) oder z.B. mit Dimethylformamid oder Dimethylsulfoxid. Zweckmässig wird das Benzimidazol-Derivat II in Gegenwart einer starken anorganischen Base (wie KOH, NaOH) zur Reaktion gebracht.

Die Reaktivität der Thioamidgruppe [R₃ = -CSNH₂] oder der substituierten Isothioamidgruppe [R₃ = -C(-SR')=NH] empfiehlt eine Vorgehensweise, bei der diese Gruppen im letzten Reaktionsschritt nach der Sulfonylierung eingeführt werden. In diesem Falle wird also das sulfonylierte 2-Cyanobenzimidazol der Formel I' 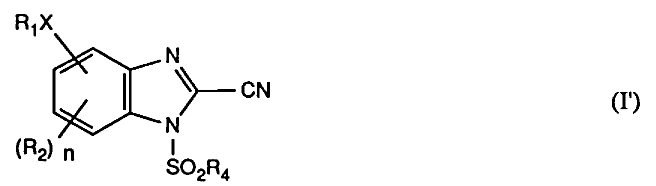 nachträglich wahlweise mit H₂S bzw. HS-R' zur Reaktion gebracht, wobei R' die für Formel I gegebene Bedeutung hat. Die zum Thioamid [R₃ = -CSNH₂] führende Reaktion wird in polaren Lösungsmitteln (z.B. Alkoholen wie Ethanol), bevorzugt aber in Dimethylformamid (DMF) oder in Ethern (z.B. Diethylether, Tetrahydrofuran) bzw. Acetonitril oder in Pyridin unter Verwendung eines tert. Amins wie Trialkylamin durchgeführt. Die Einleitung des H₂S erfolgt bei -20° bis +80°C, besonders -10° bis +40°C.

Zur Herstellung der substituierten Isothioamidgruppe kann zweckmässig so verfahren werden, dass man das sulfonylierte 2-Cyanobenzimidazol der Formel I' in einem polaren aprotischen Lösungsmittel (wie Acetonitril) in Gegenwart schwacher Basen (wie Alkalicarbonat) bei -20° bis +100°C, vorzugsweise -10° bis +40°C, mit dem Thiol HS-R' behandelt.

Die Herstellung von 2-Cyanobenzimidazol-Derivaten der Formel II erfolgt nach an sich bekannten Methoden aus o-Phenylendiamin-Derivaten oder ihren Salzen:
a) 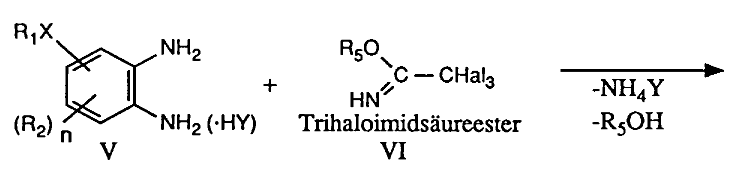 (Y= Anion einer Säure; Hal = Halogen, bevorzugt Cl)
b)

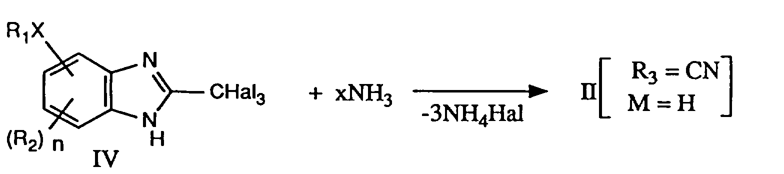

HY bedeutet eine Säure, bevorzugt eine Halogenwasserstoffsäure, oder Schwefelsäure. Das o-Phenylendiamin-Derivat lässt sich jedoch auch als freie Base einsetzen, wenn der Reaktionsschritt a) in Eisessig durchgeführt wird. Bevorzugte Lösungsmittel sind Eisessig; Ether wie Diethylether, Dioxan, 1,2-Dimethoxyethan; Ester wie Ethylacetat oder Alkohole wie Methanol und Ethanol.

Der Trihaloimidsäureester (z.B. Trichlor- oder Chlordifluor-methylimidsäuremethylester) wird zweckmässig bei -20°C bis +100°C zu dem gelösten oder suspendierten o-Phenylendiamin-Derivat zugegeben. Im Reaktionsschritt b) gibt man zweckmässig das gewonnene 2-Trihalomethylbenzimidazol-Derivat zu einer konzentrierten wässrigen Ammoniaklösung (USP. 3,576,818).

Die Herstellung der 2-Cyanobenzimidazol-Derivate der Formel II kann auch mit dem entsprechenden o-Nitroanilin-Derivat an Stelle des o-Phenylendiamin-Derivats durch Reaktion mit Formaldehyd und KCN in Eisessig unter Zusatz von Zinkchlorid (oder einer anderen Lewis-Säure) als Katalysator durchgeführt werden, wobei Verbindungen der Formel VII 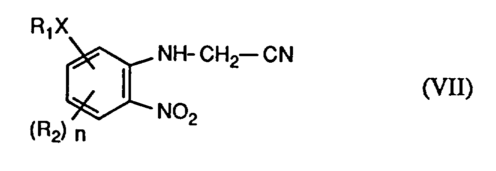 erhalten werden [K. Dimroth et al., Ber. 98, 3902 (1965)], die mit K₂CO₃ zu l-Hydroxy-2-cyanobenzimidazol-Derivaten cyclisiert [B. Serafinowa et al. Rocz. Chem. 51, 1783 (1977)] und mit PCl₃ zu Verbindungen der Formel II (M = Wasserstoff; R₃ = CN) umgesetzt werden können.

Die Zwischenprodukte der Formel II mit R₃ = CN und M = Wasserstoff sind neu und bilden einen weiteren Gegenstand vorliegender Erfindung. (Verbindungsgruppe II'). 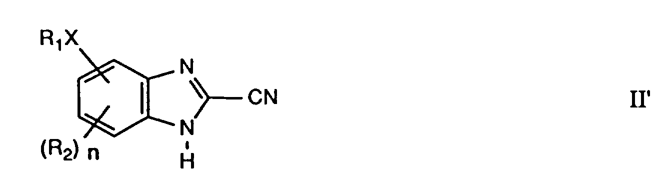 Verbindungen der Formel V lassen sich durch Reduktion der nitrogruppenhaltigen Verbindungen der Formel VIII herstellen. Als Reduktionsmittel können die klassischen Reduktionsmittel wie Eisen (Bechamps-Reduktion), Zinn(II)chlorid oder aber Wasserstoff mit einem Katalysator wie beispielsweise Raney-Nickel oder Palladium/Kohle verwendet werden. Die Reaktionsbedingungen entsprechen denen, die in der Literatur (z.B. Houben Weyl "Methoden der organischen Chemie") aufgeführt sind.

Verbindungen der Formel VIII lassen sich falls R₁ ein 6-Ring Heterocyclus ist, aus Verbindungen der Formel IX durch Umsetzung mit entsprechend substituierten 2-Halopyridinen, 2-Halopyrazinen, 3-Halopyridazinen, 2-Halopyrimidinen oder 4-Halopyrimidinen (Formelgruppe X) erhalten. Die Reaktion findet in inerten organischen Lösungsmitteln, bevorzugt in polaren wie DMF, DMSO, DMA oder ketonen wie Aceton oder Ethylmethylketon, Alkoholen wie Ethanol, Propanol, Butanol oder Ethern wie Diethylether oder Tetrahydrofuran in Gegenwart einer Base, bevorzugt Alkali(hydrogen)carbonate oder -hydroxyde wie Na₂CO₃, K₂CO₃, NaOH oder KOH oder Amine wie Triethylamin oder Pyridin statt. Die Reaktionstemperatur liegt zwischen O°C und +150°C bevorzugt bei 80-120°C.

Ein alternativer Zugang zu Verbindungen der Formel VIII ist die Umsetzung von Verbindungen der Formel XI mit entsprechend substituierten eine Hydroxy- oder Mercapto-Gruppe tragenden Pyridinen, Pyrimidinen, Pyridazinen oder Pyrazinen (Formelgruppe XII). Die Reaktion verläuft vorzugsweise unter denselben Bedingungen wie oben beschrieben.

Die Verbindungen der Formel IX, X, XI und XII sind entweder in der Literatur bekannt, oder können nach bekannten Methoden hergestellt werden.

Verbindungen der Formel II' lassen sich auch aus Verbindungen der Formel XIII durch Umsetzung mit 2-Halopyridinen, 2-Halopyrazinen, 3-Halopyridazinen oder 2-, bzw. 4-Halopyrimidinen (Formelgruppe X) synthetisieren. Die Reaktion wird in inerten organischen Lösungsmitteln, bevorzugt in polaren Lösungsmitteln wie beispielsweise DMF, DMSO, DMA oder Ketonen wie Aceto oder Ethylmethylketon, Alkoholen wie Ethanol, Propanol, Butanol oder Ethern wie Diethylether oder Tetrahydrofuran in Gegenwart einer Base durchgeführt. Bevorzugte Basen sind beispielsweise Carbonate wie Natriumcarbonat oder Kaliumcarbonat aber auch Hydroxyde wie Kaliumhydroxyd oder Natriumhydroxyd oder Amine wie Triethylamin oder Pyridin. Die Reaktionstemperatur liegt zwischen 0°C und 150°C bevorzugt bei 80°C bis 120°C.

Verbindungen der Formel XIII lassen sich aus Verbindungen der Formel XIV wie oben beschrieben herstellen.

Verbindungen der Formel XIV lassen sich durch Reduktion der Nitrogruppe in Verbindungen der Formel IX herstellen. Als Reduktionsmittel können beispielsweise Eisen (Bechamps-Reduktion), Zinn(II)chlorid oder aber Wasserstoff in Gegenwart eines Katalysators wie z.B. Raney-Nickel oder Palladium auf Kohle verwendet werden. Die Reaktionsbedingungen entsprechen denen, die in der Literatur (z.B. Houben Weyl) angegeben sind.

### Allgemeines Reaktionsschema 1

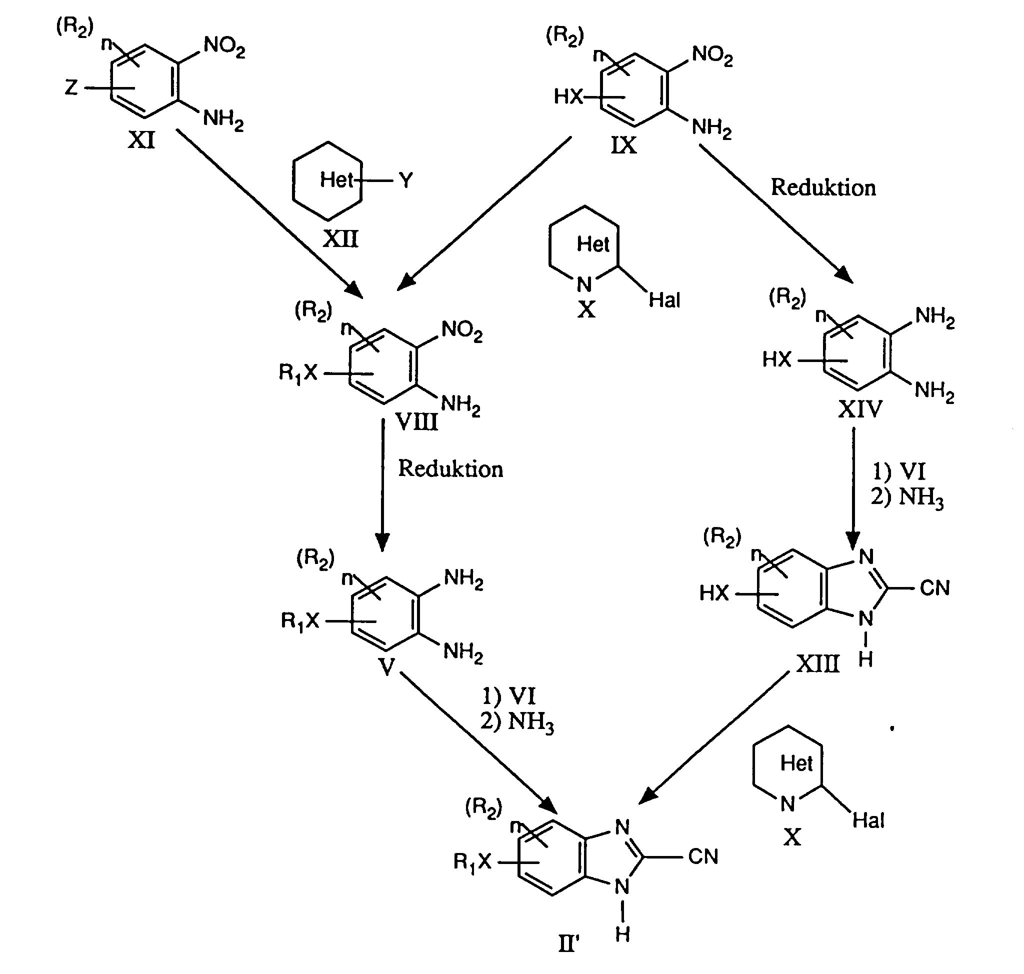

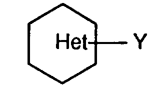

= gegebenenfalls substituiertes Hydroxypyridin, Mercaptopyridin, Hydroxypyrimidin, Mercaptopyrimidin, Hydroxypyrazin, Mercaptopyrazin, Hydroxypyridazin oder Mercaptopyridazin
Z = Abgangsgruppe (wie z.B. Halogen), die sich o- oder p-ständig zu der NO₂-Gruppe befindet. 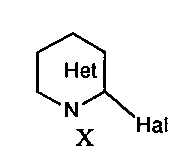 = gegebenenfalls substituiertes 2-Halopyridin, -2-Halopyrazin, 2- oder 4-Halopyrimidin, 3-Halopyridazin

Falls R₂ Fluor, Chlor oder Brom ist, so können die entsprechenden Verbindungen auch durch Halogenierung von Verbindungen, in denen R₂ Wasserstoff bedeutet, erhalten werden. Dies gilt für Verbindungen der Formel II', V, VII, VIII, IX, XI, XIII und XIV.

Verbindungen in denen R₁ ein 5-Ring Heterocyclus ist, lassen sich je nach Reaktivität analog herstellen.

Falls R₁ einen Thiazolring bildet, ist folgende Reaktionssequenz möglich:

### Allgemeines Reaktionsschema 2

Herstellung der Thaizolverbindungen: 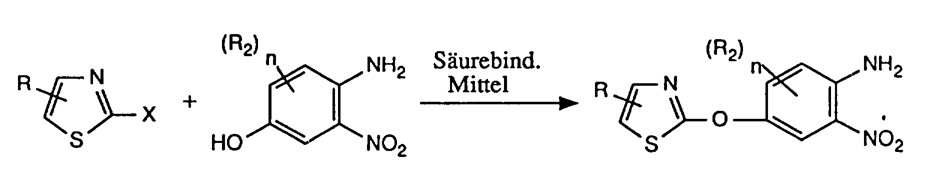 oder durch Aufbau des Thiazolringes: z.B. 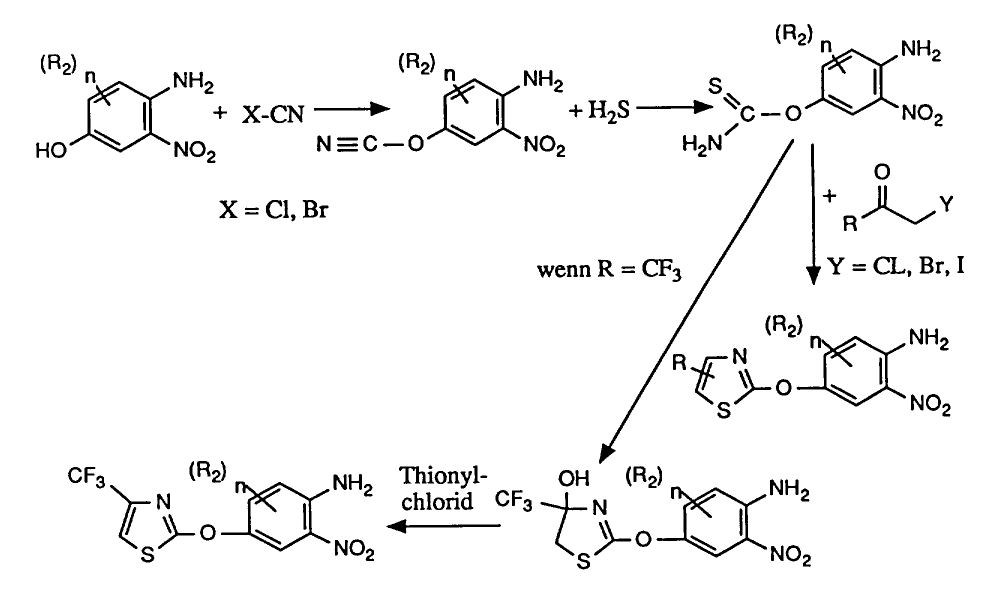 Im Falle von X = S können Verbindungen der Formel IX auch als Rhodanid maskiert eingesetzt werden (US-Patentschrift 4,076,828). 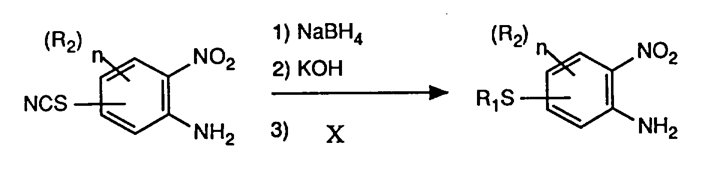 Verbindungen des Typs 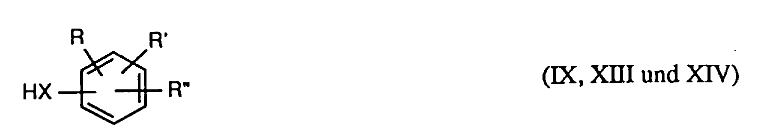 können, falls X = S, ist auch in der dimeren Form als Disulfid vorliegen. Die Mercaptoverbindungen können daraus durch Reduktion erhalten werden. Methoden dafür sind aus der Literatur bekannt.

In der EP-A-152360 sind 2-cyano-1-sulfonamide-benzimidazol-deriva mit fungiziden Eigenschaften beschrieben. Ihre Wirkung ist zum Teil unbefriedigend.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative und präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Pilzen verschont bleiben.

Die neuen Wirkstoffe der Formel I erweisen sich als bevorzugt wirksam gegen spezielle Gattungen der Pilzklasse Fungi imperfecti (z.B. Cercospora), Basidiomyceten (z.B. Puccinia) sowie Ascomyceten (z.B. Erysiphe und Venturia) und insbesondere gegen Oomyceten (z.B. Plasmopara, Peronospora, Pythium und Phytophthora). Sie stellen damit im Pflanzenschutz eine wertvolle Ergänzung der Mittel für die Bekämpfung von phytopathogenen Pilzen dar. Für ihre Anwendung in der Praxis besitzen sie vorteilhafterweise sowohl kurative wie auch präventive Eigenschaften und können zum Schutz von zahlreichen Kulturpflanzen eingesetzt werden. Mit diesen Wirkstoffen werden an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet, wobei auch später zuwachsende Pflanzenteile z.B. vor phytopathogenen Pilzen verschont bleiben. Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel ausgezeichnet ist.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberfiächenaktiven Verbindungen (Tensiden).

Als Lösungsmittel kommen in Frage: Aromatische Kohlenwasserstoffe, bevorzugt die Fraktionen C₈ bis C₁₂, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulattträger kommen poröse Typen, wie Bimsstein, Ziegelbruch, Sepiolit oder Bentonit, als nicht-sorptive Trägermaterialien z. B. Calcit oder Sand in Frage. Darüber hinaus kann eine Vielzahl von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypolyethylenethanol, Polyethylenglycol und Octylphenoxypolyethoxyethanol erwähnt.

Ferner kommen auch Fettsäureester von Polyoxyethylensorbitan, wie das Polyoxyethylensorbitan-trioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

Die nachfolgenden Beispiele illustrieren die vorstehend beschriebene Erfindung, ohne dieselbe in ihrem Umfang in irgendeiner Weise einzuschränken. Temperaturen sind in Celsiusgraden angegeben.

### Herstellungsbeispiele

### H-1. Herstellung von

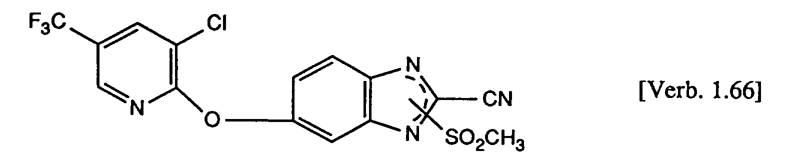

### 1(3)-Methansulfonyl-2-cyano-6-(3-chlor-5-trifluormethylpyridin-2-yloxy)-benzimidazol

### a) Herstellung des Zwischenprodukts 2-Cyano-5-(3-chlor-5-trifluormethylpyridin-2-yloxy)-benzimidazol.

4,55 g (15 mMol) 1,2-Diamino-4-(3-chlor-5-trifluormethylpyridin-2-yloxy)-benzol werden in 35 ml Eisessig gelöst und unter Rühren bei Raumtemperatur 3,95 g (22 mMol) 2,2,2-Trichloracetimidsäuremethylester zugetropft. Es wird 14 Stunden weitergerührt, auf 150 ml Wasser gegossen, dreimal mit je 40 ml Ethylether extrahiert, die vereinigten Extrakte werden über Natriumsulfat getrocknet und das Lösungsmittel verdampft. Das gebildete 2-Trichlormethyl-5-(3-chlor-5-trifluorrnethylpyridin-2-yloxy)-benzimidazol in Form eines dunkelbraunen Oeles wird ohne weitere Reinigung in 30 ml Tetrahydrofuran gelöst und unter Rühren bei Raumtemperatur innerhalb einer Stunde mit 70 ml einer ' 25%igen wässrigen Ammoniaklösung versetzt und noch 1 Stunde weitergerührt. Nach dem Abdestillieren des Tetrahydrofurans im Vakuum wird mit konz. Chlorwasserstoffsäure ein pH-Wert von 5 eingestellt, der ausgefallene Niederschlag absaugt und über eine Kieselgelsäule mittels Ethylacetat/Hexan (1:1) chromatographiert. Nach dem Verdampfen des Laufmittels wird der kristalline Niederschlag mit 15 ml Hexan digeriert, filtriert und getrocknet. Die beigefarbenen Kristalle schmelzen >270°C.

### b) Herstellung des Endprodukts

Zu einer Lösung von 4,14 g (12 mMol) 2-Cyano-5-(3-chlor-5-trifluormethylpyridin-2-yloxy)-benzimidazol in 65 ml Aceton werden bei Raumtemperatur 1,02 g (15,5 mMol) 85%iges Kaliumhydroxyd auf einmal zugegeben und eine Stunde gerührt. Zu diesem Gemisch werden 3,2 g (28 mMol) Methansulfochlorid innerhalb einer Stunde bei Raumtemperatur zugeropft und 16 Stunden bei 20°C weitergerührt. Zur Vervollständigung der Reaktion werden nochmals 0,5 g (7,6 mMol) 85%iges Kaliumhydroxyd und nach einer halben Stunde 1,6 g (14 mMol) Methansulfochlorid zugegeben und weitere vier Stunden bei Raumtemperatur gerührt. Anschliessend wird das Reaktionsgemisch auf 200 ml Wasser gegossen, dreimal mit je 30 mi Ethylacetat extrahiert, die vereinigten organischen Phasen werden zweimal mit je 20 ml gesättigter wässriger Sodalösung und einmal mit 20 ml Wasser gewaschen, über Natriumsulfat getrocknet, und filtriert. Das Lösungsmittel wird dann verdampft. Die Kristallmasse wird mit 20 ml Diethylether digeriert, filtriert und getrocknet. Die blass grau gefärbten Kristalle schmelzen bei 196-206°C.

### H-2. Herstellung von

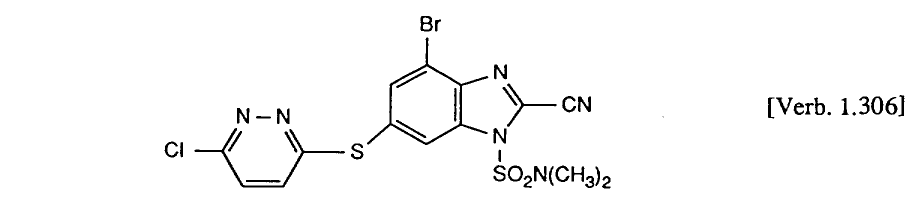

### 1-N,N-Dimethlysulfamoyl-2-cyano-4-bromo-6-(6-chloro-pyridazin-3-ylthio)-benzimidazol

### a) Herstellung des Zwischenprodukts 2-Nitro-4-(6-chloro-pyridazin-3-ylthio)-anilin.

78,1 g 2-Nitro-4-rhodano-anilin werden in 900 ml Diglyme bei 30°C gelöst und sukzessive mit 15,2 g Natriumborhydrid versetzt. Nach 1 Stunde Rühren bei 60°C wird auf Raumtemperatur abgekühlt und 26,4 g pulverisiertes Kaliumhydroxyd sowie 61,4 g 3,6-Dichlorpyridazin zugegeben. Nach 1 Stunde Rühren bei 60°C wird auf Eis gegossen und die Suspension abfiltriert. Umkristallisation aus Toluol und Digerieren mit Diethylether liefert 39,4 g des Produkts a), Smp.>230°C.

### b) Herstellung des Zwischenprodukts

2-Brom-4-(6-chloro-pyridazin-3-ylthio)-6-nitro-anilin.
10,0 g der Verbindung a) werden mit 2,9 g Natriumacetat in 130 ml Eisessig gelöst. 7,5 g Brom werden langsam zugetropft und die Suspension 30 Minuten bei 55°C ausgerührt. Das Gemisch wird auf Eis gegossen, filtriert, mit Wasser gewaschen und getrocknet. Chromatographie an Kieselgel mit Essigsäureethylester:Hexan=1:1 als Laufmittel liefert 6,4 g der Verbindung b), Smp.>250°C.

### c) Herstellung des Zwischenprodukts

1,2-Diamino-3-bromo-5-(6-chloro-pyridazin-3-ylthio)-benzol.
6,4 g der Verbindung b) werden in 200 ml Tetrahydrofuran gelöst, mit 1,2 g Raney-Nickel versetzt und 51 Stunden bei Raumtemperatur unter Wasserstoffatmosphäre gerührt. Dabei wird zweimal insgesamt 3,6 g Raney-Nickel zugegeben. Anschliessend wird der Katalysator abfiltriert und das Lösungsmittel verdampft. Man erhält 4,3 g der Verbindung c), Smp. 160-161°C.

### d) Herstellung des Zwischenprodukts

2-Cyan-4-bromo-6-(6-chloro-pyridazin-3-ylthio)-benzimidazol.
4,3 g der Verbindung c) werden in 30 mi Eisessig suspendiert. 3,55 g 2,2,2-Trichloracetimidsäuremethylester werden zugetropft und 1 Stunde bei Raumtemperatur gerührt. Das Gemisch wird auf Eiswasser gegossen und dreimal mit je 20 ml Diethylether extrahiert. Die organische Phase wird über Natriumsulfat getrocknet, filtriert und eingeengt. Der Rückstand wird in 30 ml Tetrahydrofuran gelöst und bei Raumtemperatur zu 60 ml konzentrierter Ammoniaklösung getropft. Nach 1 Stunde wird das Reaktionsgemisch eingeengt, mit Wasser verdünnt und mit konzentrierter HCl-Lösung angesäuert. Die entstandene Suspension wird filtriert, mit Wasser gewaschen und getrocknet. Man erhält 4,6 g der Verbindung d), Smp.>250°C.

### e) Herstellung des Endprodukts

2,3 g der Verbindung d) werden in 10 ml N-Methyl-2-pyrrolidon gelöst und mit 0,28 g 60%igen Natriumhydrid versetzt. Nach beendeter Gasentwicklung wird noch 10 Minuten bei 40°C gerührt und auf 15°C abgekühlt. 0,876 ml N,N-Dimethylsulfaminsäurechlorid werden zugegeben. Die dunkelbraune Suspension wird 60 Minuten bei Raumtemperatur und 30 Minuten bei 35°C gerührt. Das Gemisch wird auf Eiswasser gegossen und dreimal mit je 20 ml Essigsäureethylester extrahiert. Die organischen Phasen werden mit Wasser und Sole gewaschen, über Natriumsulfat getrocknet, abfiltriert und eingeengt. Der Rückstand wird an Kieselgel mit Essigsäureethylester:Hexan=1:1 chromatographiert. Man erhält 1,17 g der Titelverbindung, Smp. 169-170°C.

### H-3. Herstellung von

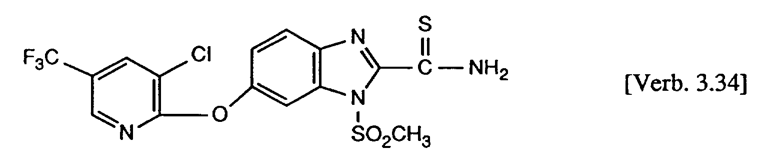

### 1(3)-Methansulfonyl-2-thioamido-6-(3-chlor-5-trifluormethyl-pyridin-2-yloxy)-benzimidazol

1,12 g der Verbindung Nr. 1.66 werden in 12 ml Dioxan und 4 ml Tetrahydrofuran gelöst. Man gibt 0,43 ml Triethylamin zu und leitet bei Raumtemperatur während 30 Minuten Schwefelwasserstoff ein. Die Lösung wird auf das halbe Volumen eingeengt und auf Eiswasser gegossen. Man extrahiert mit Essigsäureethylester. Die organische Phase wird mit Sole gewaschen, über Natriumsulfat getrocknet, filtriert und eingeengt. Die entstehenden Kristalle werden mit Ether digeriert Es resultieren 0,47 g der Verbindung Nr. 3.34, Smp. 181-182°C.

### H-4. Herstellung von

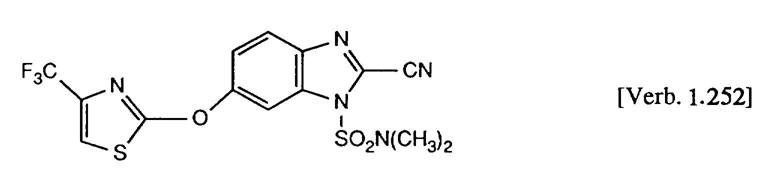

### 1(3)-N,N-Dimethylsulfamoyl-2-cyano-6-(4-trifluormethyl-2-oxo-thiazolyl)-benzimidazol

Dieses Produkt wurde nach den schon unter H-2 beschriebenen Methoden aus dem folgenden Zwischenprodukt hergestellt:

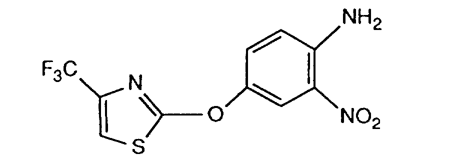

### Herstellung des Zwischenprodukts

50,0 g 4-Amino-3-nitrophenol werden in 40 ml Aceton gelöst und anschliessend 34,4 g Bromcyan zugegeben. Bei 0-5°C wird innert 45 Minuten 32,9 g Triethylamin zugetropft. Gegen Ende der Zugabe des Triethylamins wird nochmals 100 ml Aceton zugegeben. Anschliessend wird 15 Minuten nachgerührt. Das Reaktionsgemisch wird über Hyflo filtriert, mit Aceton gewaschen und eingeengt. Es werden 64,7 g eines orange-gelben Pulvers erhalten, das direkt für die nächste Stufe eingesetzt wird.

Es wird in 500 ml Tetrahydrofuran gelöst und nochmals filtriert, um Spuren von Triethylaminsalzen zu entfernen. Anschliessend werden bei 25-30°C 12,0 g Schwefelwasserstoff innert 3 Stunden eingeleitet und eine weitere Stunde nachgerührt. Zur Aufarbeitung wird das Reaktionsgemisch unter Rühren auf Eis/Wasser gegossen, filtriert und mit Wasser gewaschen. Der feuchte Feststoff wird in Essigsäureethylester gelöst, mit Magnesiumsulfat entwässert, filtriert und eingeengt. Man erhält 56,0 g 4-Amino-3-nitro-phenylthiocarbamat als orange farbenes Pulver.

1,0 g dieses Amino-nitro-phenylthiocarbamats werden in 10 ml Dioxan/Toluol (1:1) gelöst und 1,2 g -Bromotrifluoraceton zugetropft. Nach 1 Stunde Rühren bei 35°C wird das Reaktionsgemisch filtriert, mit Hexan und Ether gewaschen und getrocknet. Zur Abspaltung von Wasser wird dieses Pulver in Dichlorethan suspendiert und mit 2,5 g Thionylchlorid während einer Stunde auf Rückfluss erhitzt. Die Reaktionsmischung wird eingeengt, der Rückstand in Essigester aufgenommen, mit 10%iger Kaliumcarbonatlösung und Sole gewaschen, die organische Phase über Magnesiumsulfat getrocknet und eingeengt. Es werden 0,35 g eines orangen Oels von 1-Amino-2-nitro-4-(4-trifluormethyl-2-oxo-thiazolyl)-benzol erhalten, das gemäss ¹H-NMR-Spektrum rein ist und direkt zum Endprodukt weiter verarbeitet wird.

Reaktionsschema für die Herstellung des Zwischenprodukts:

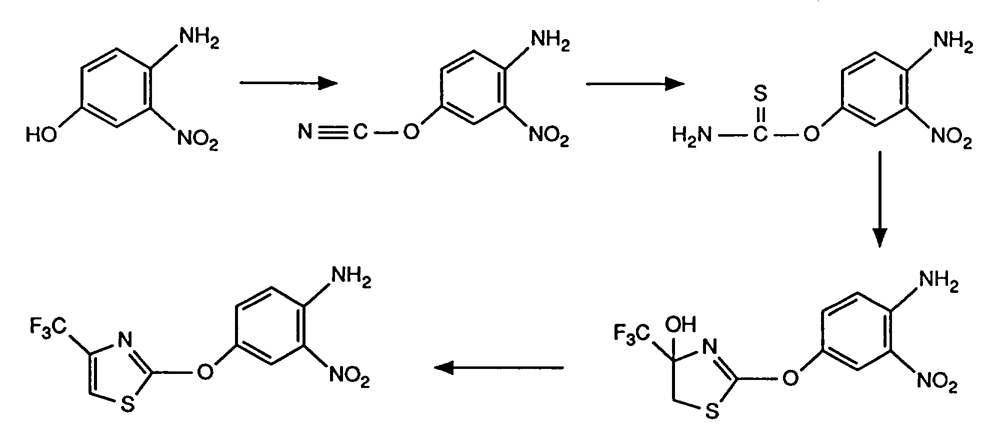

Auf diese Weise oder nach Art einer der weiter oben angegebenen Methoden lassen sich beispielsweise folgende Verbindungen herstellen.

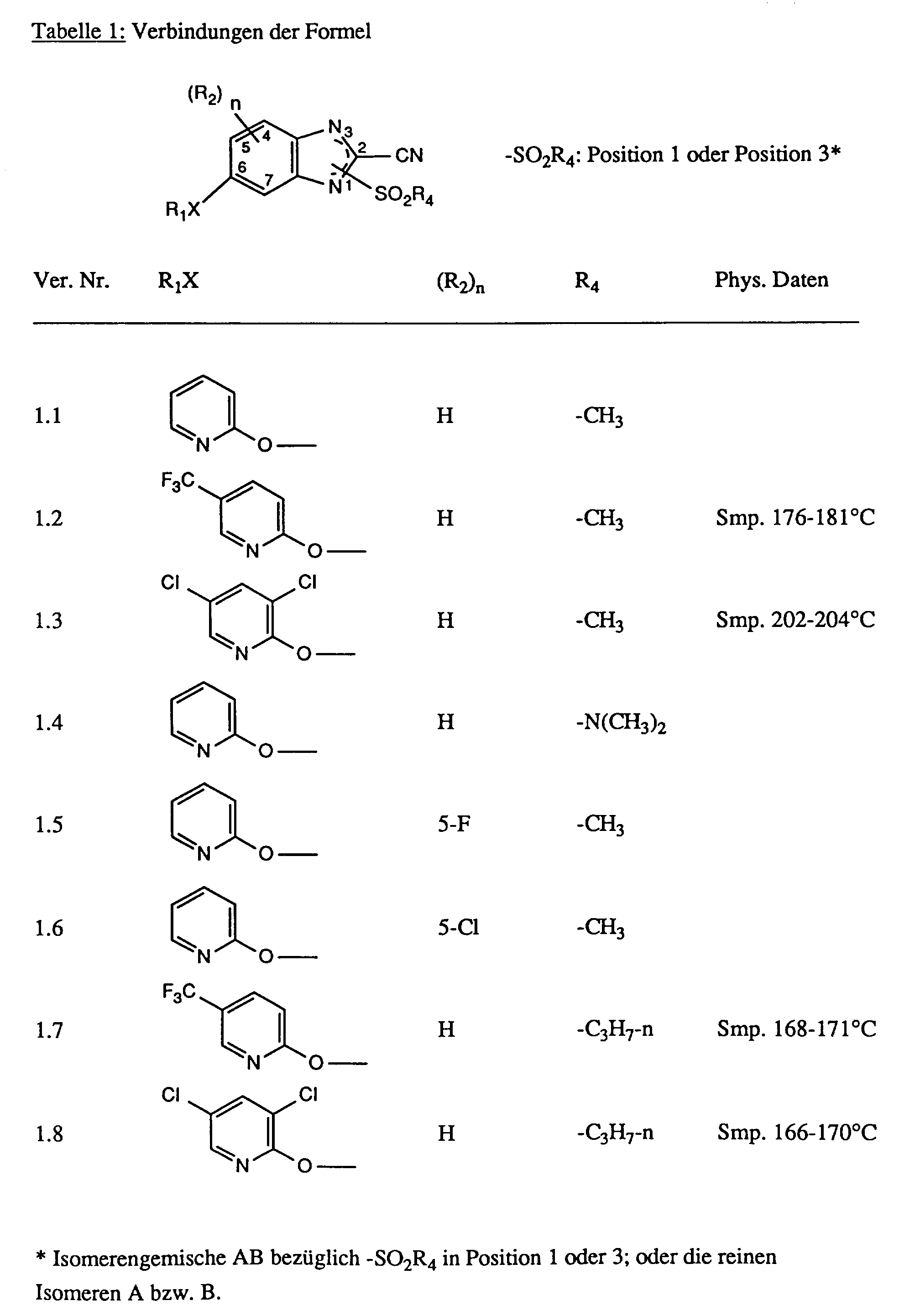

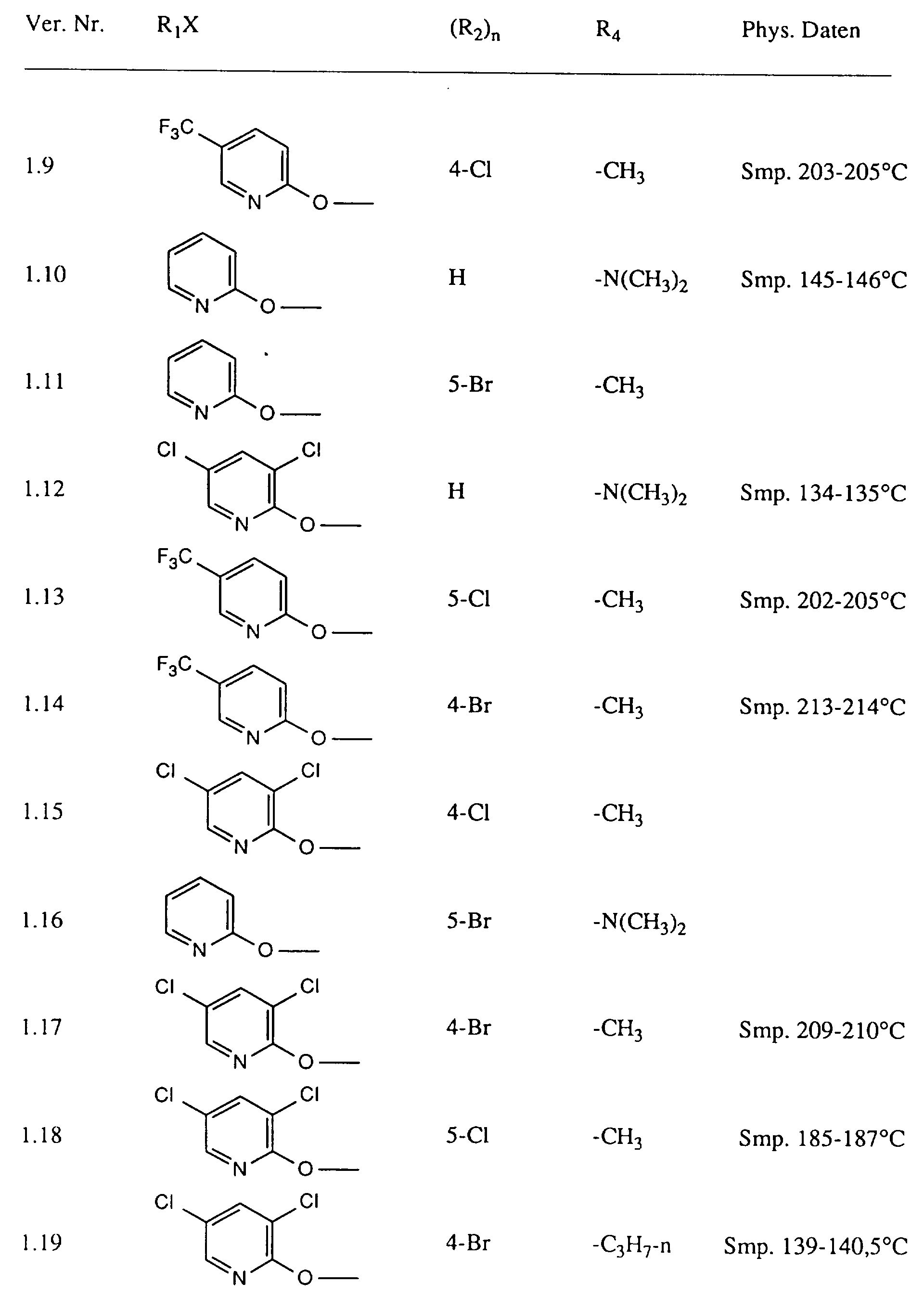

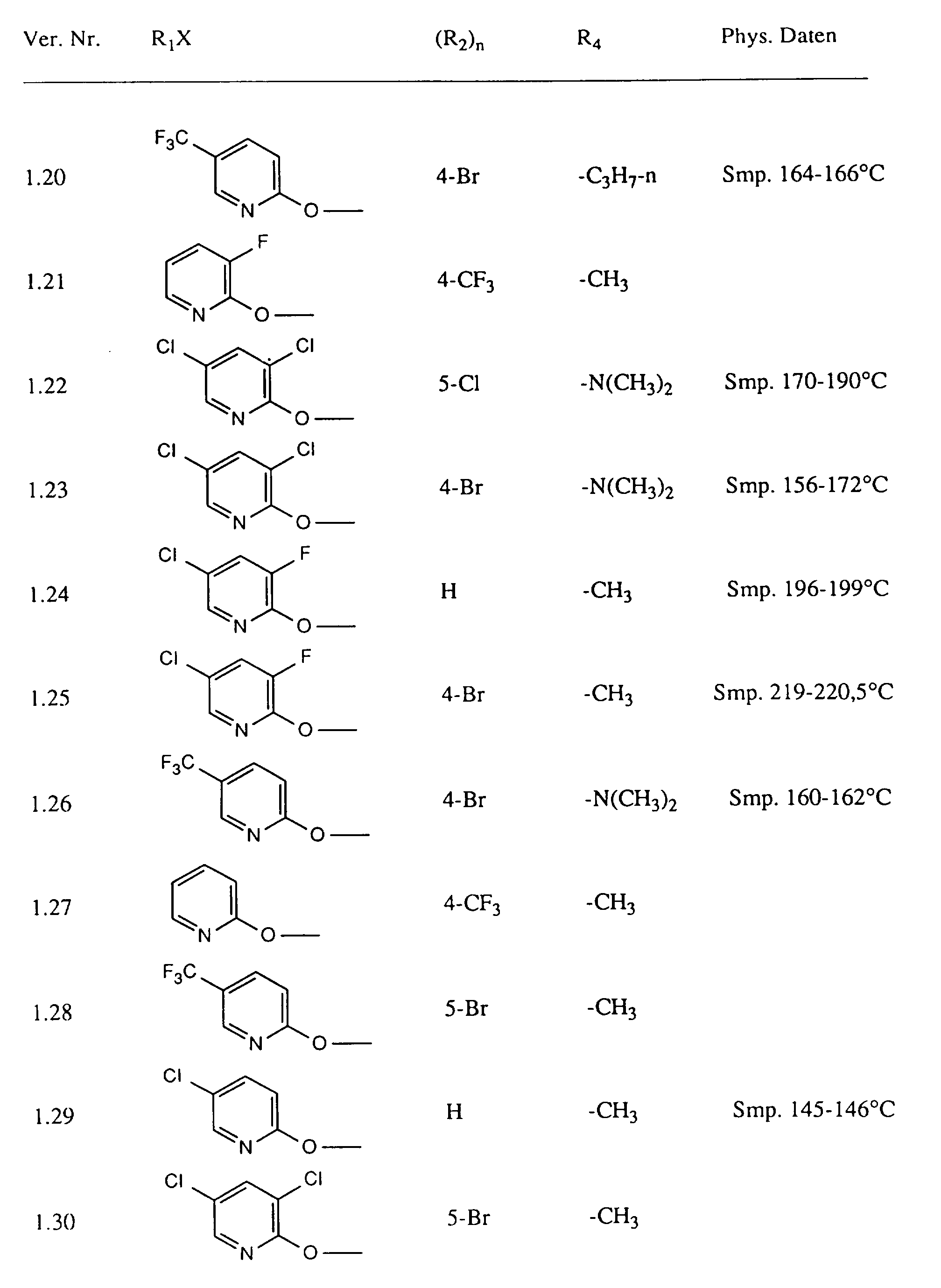

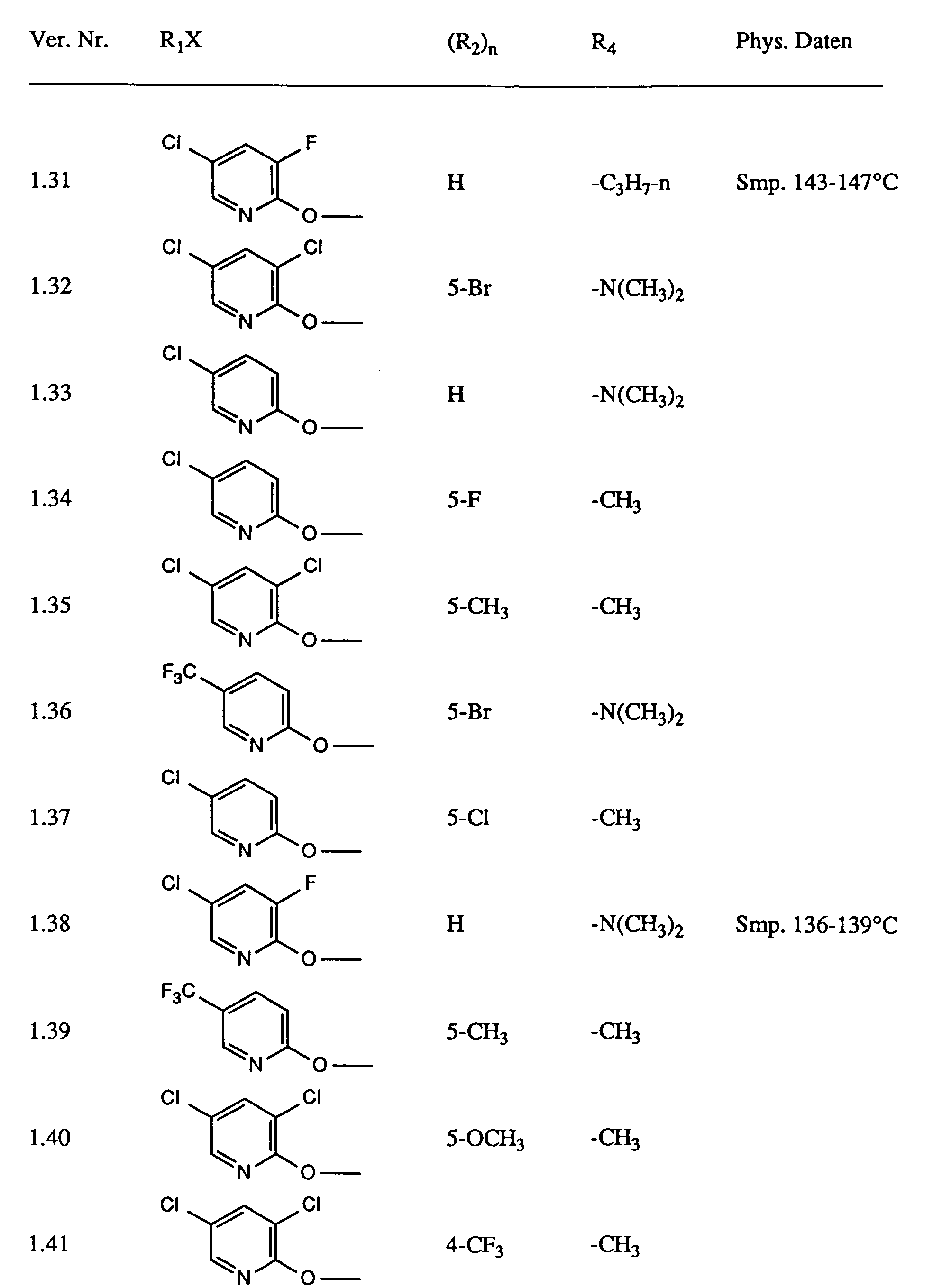

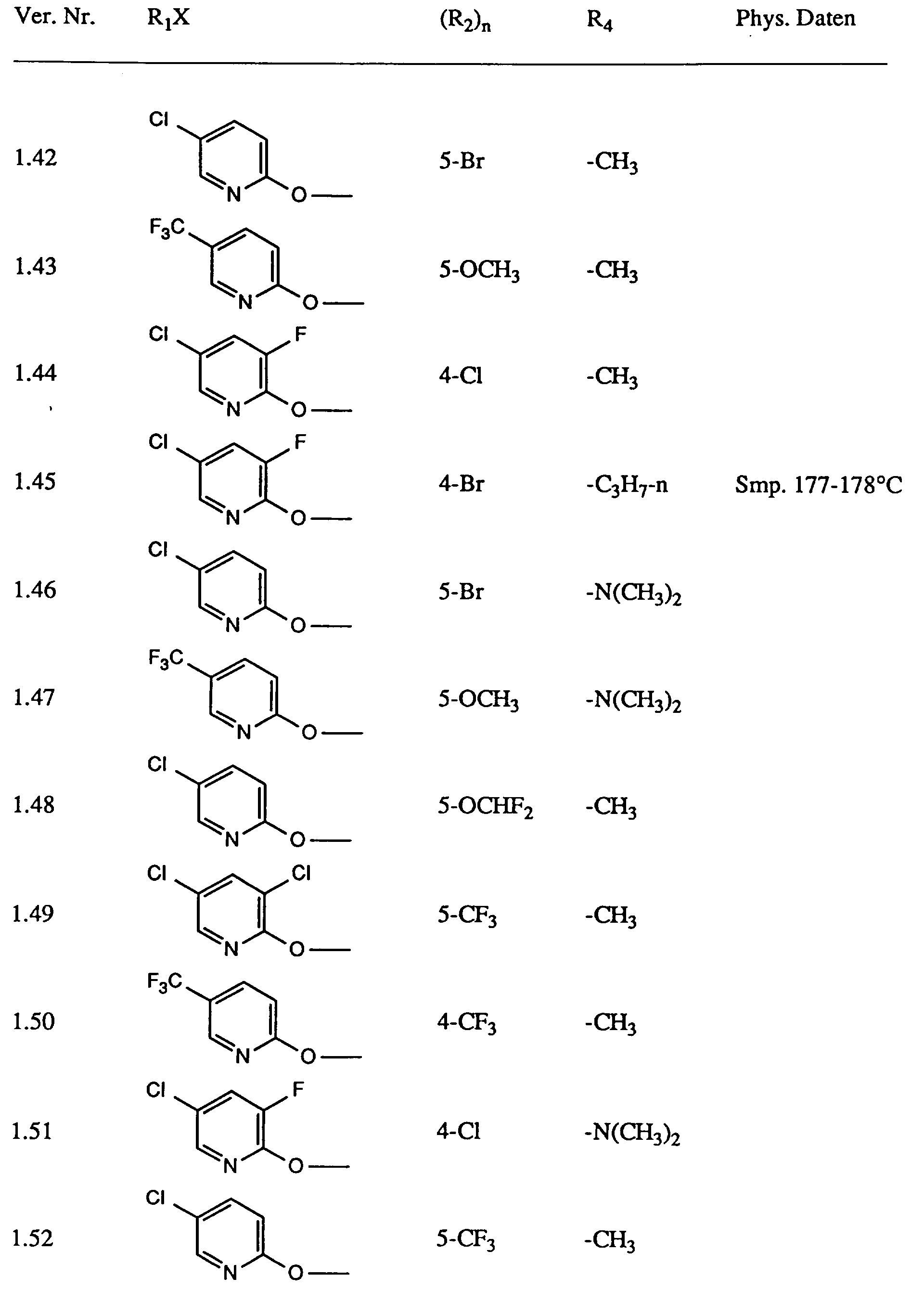

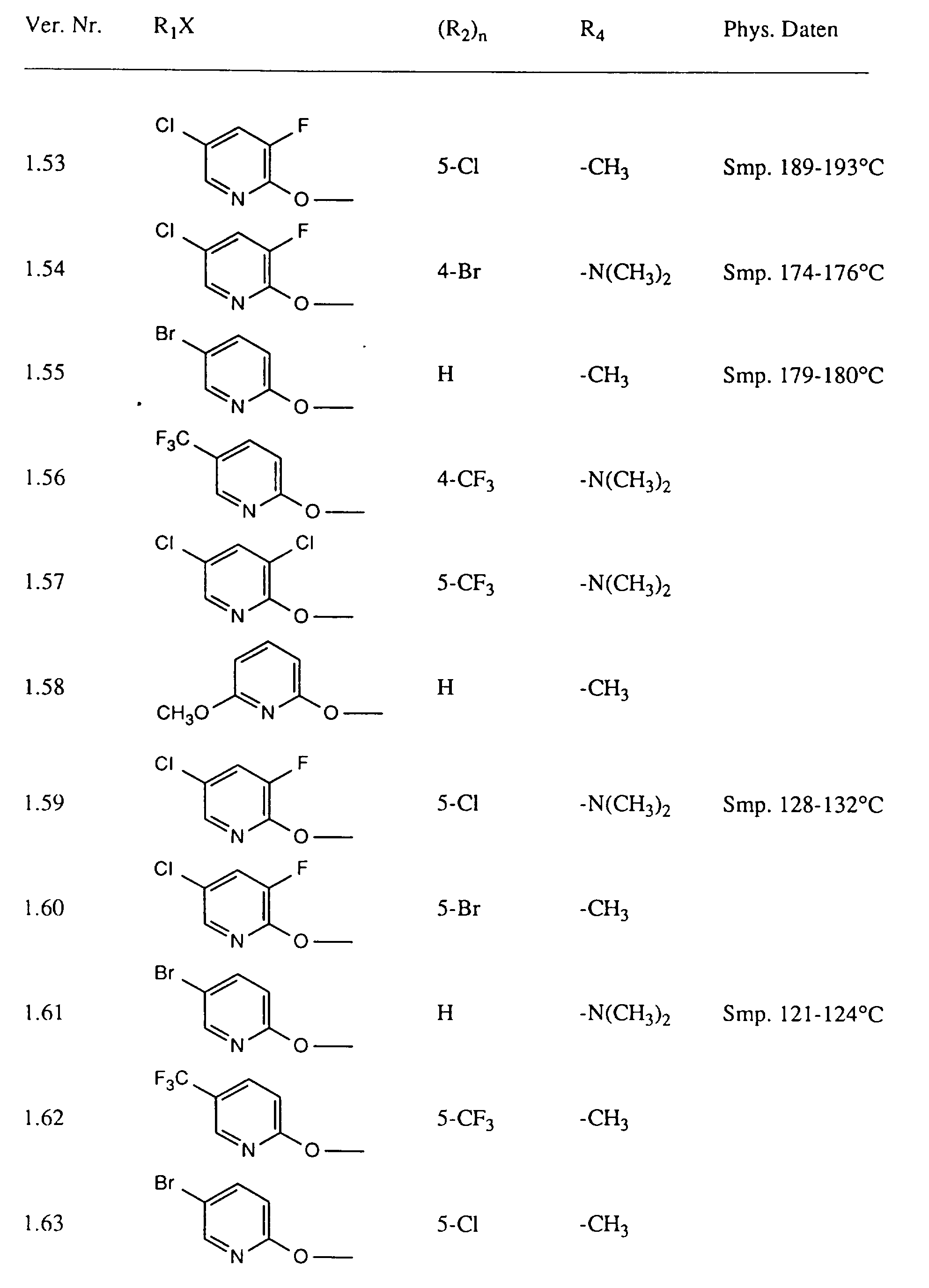

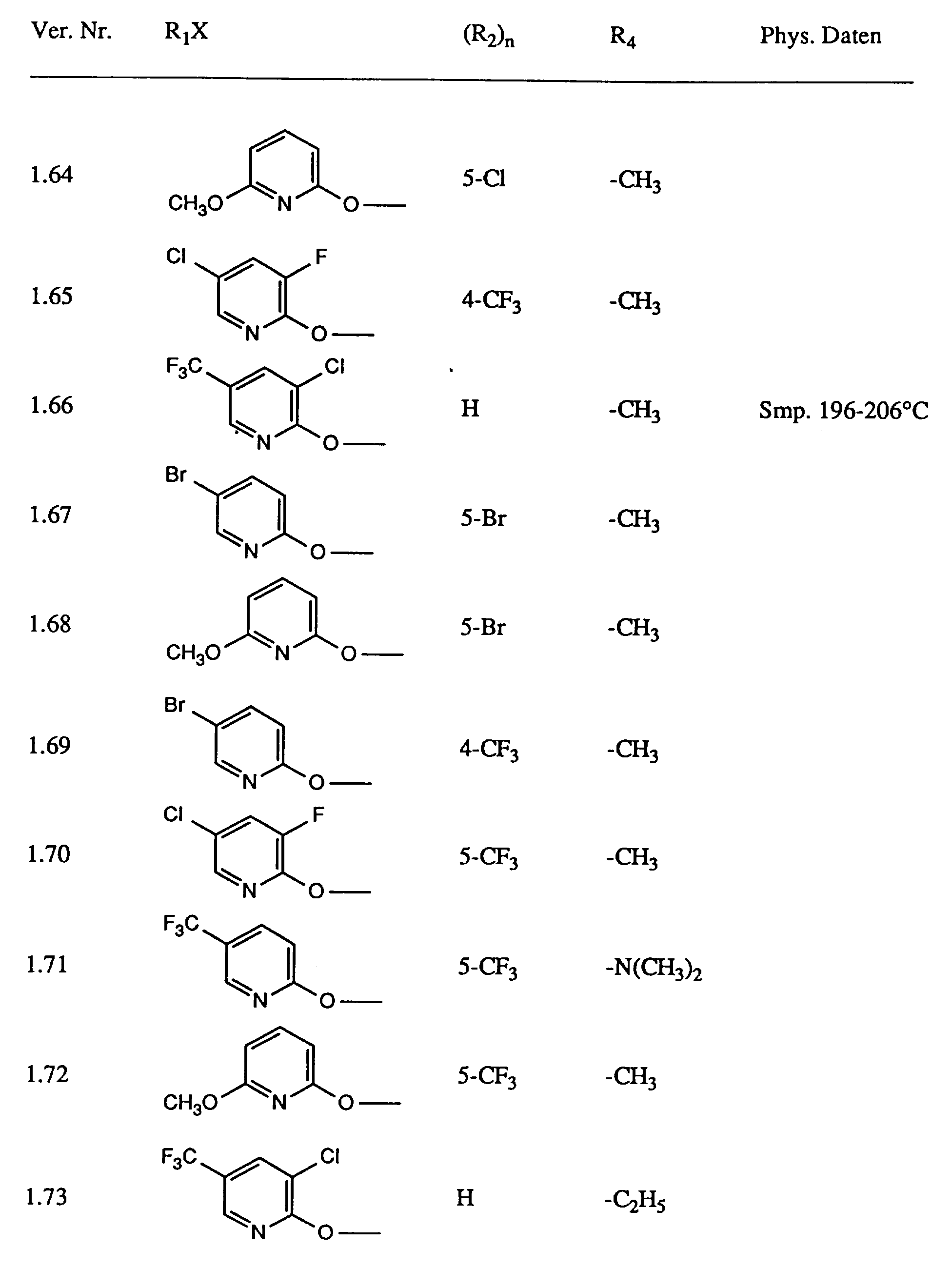

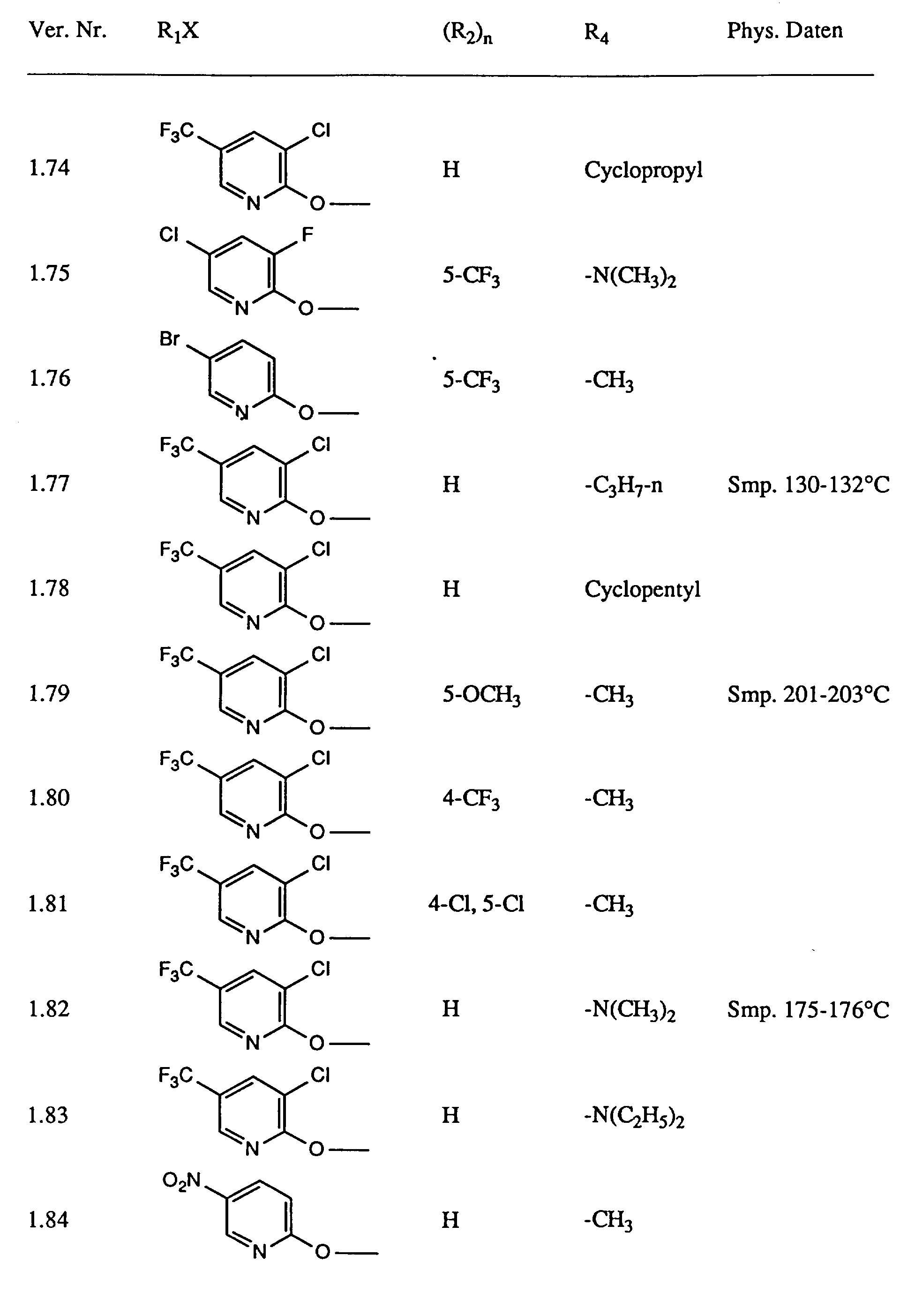

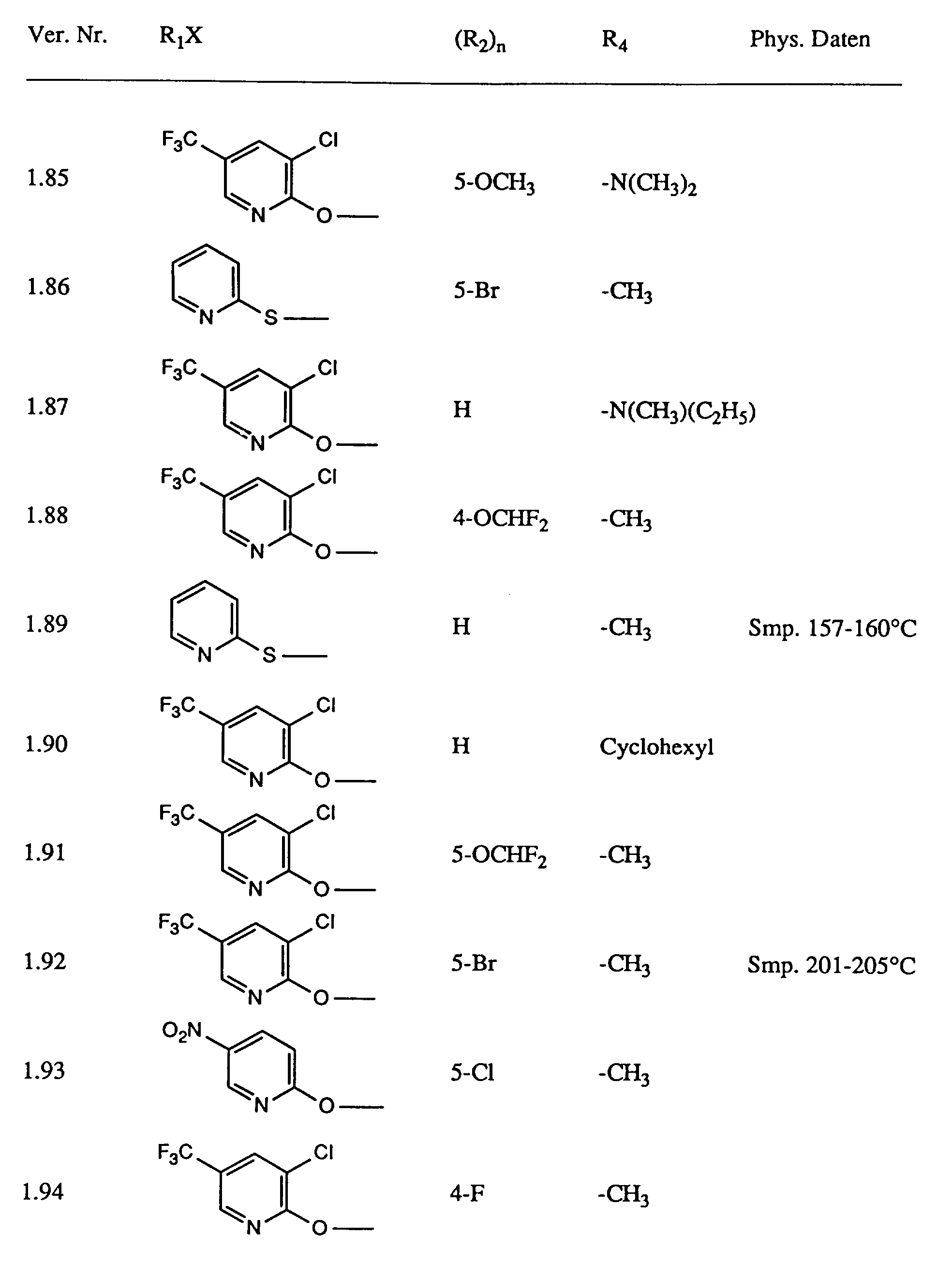

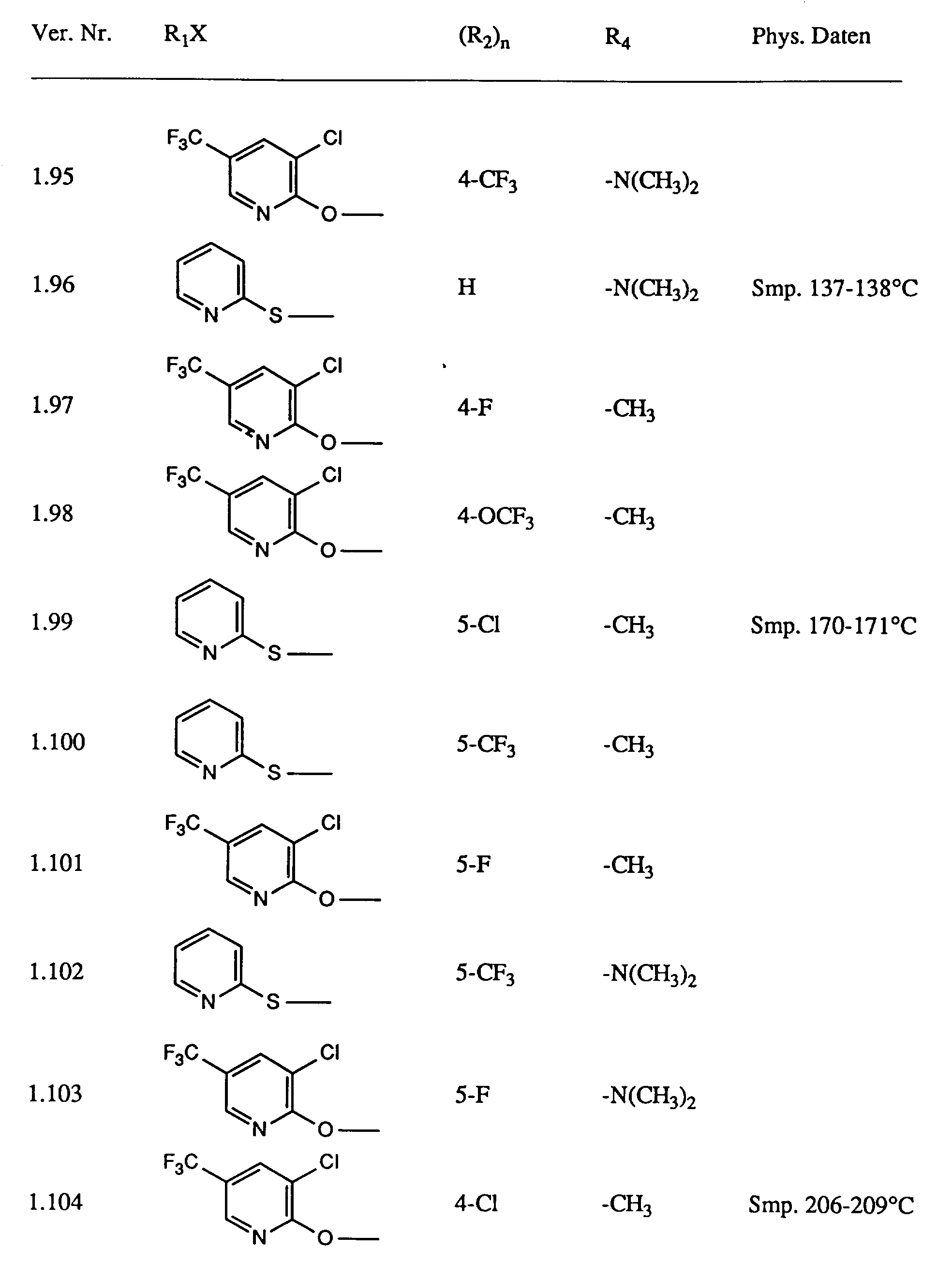

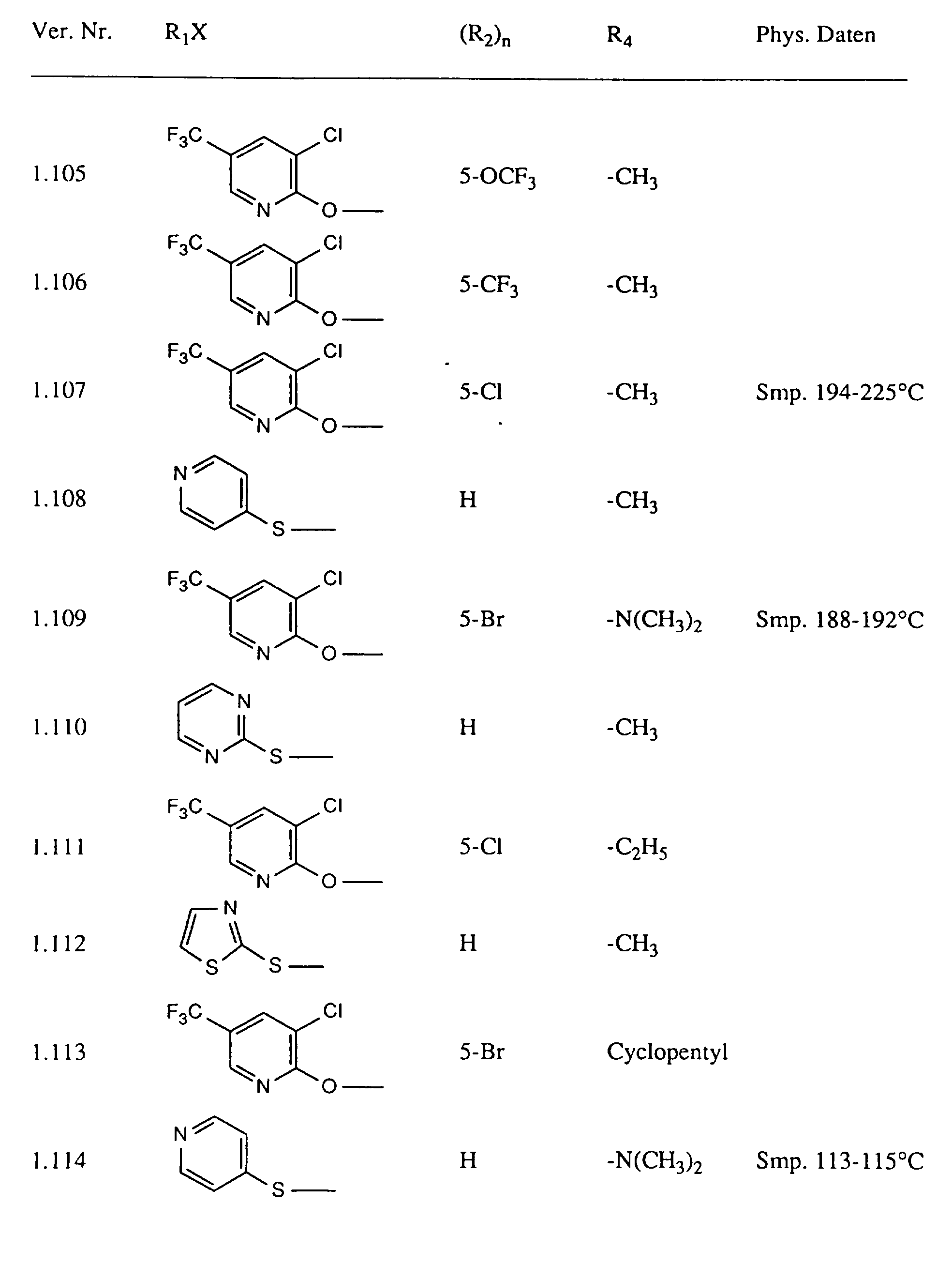

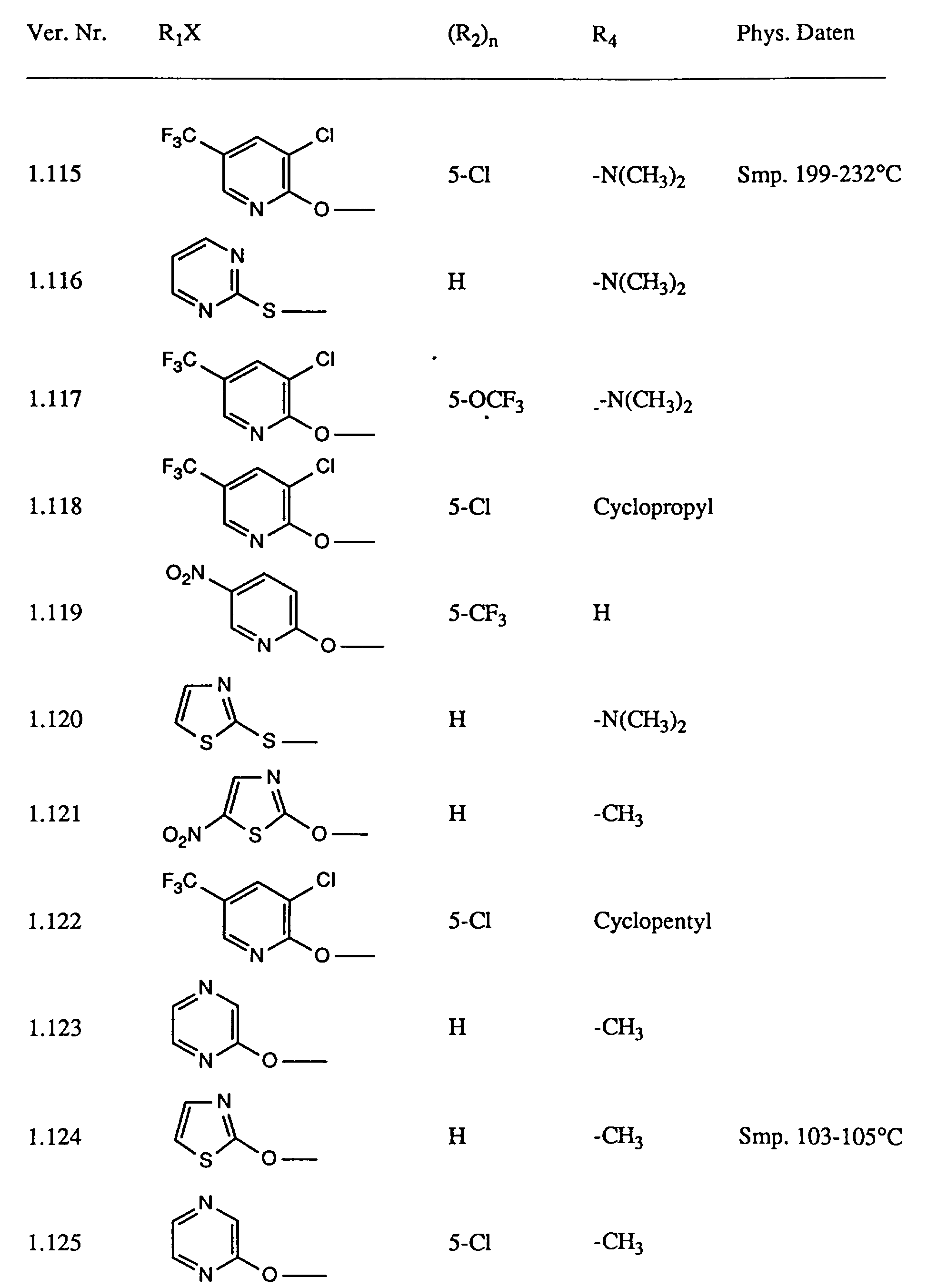

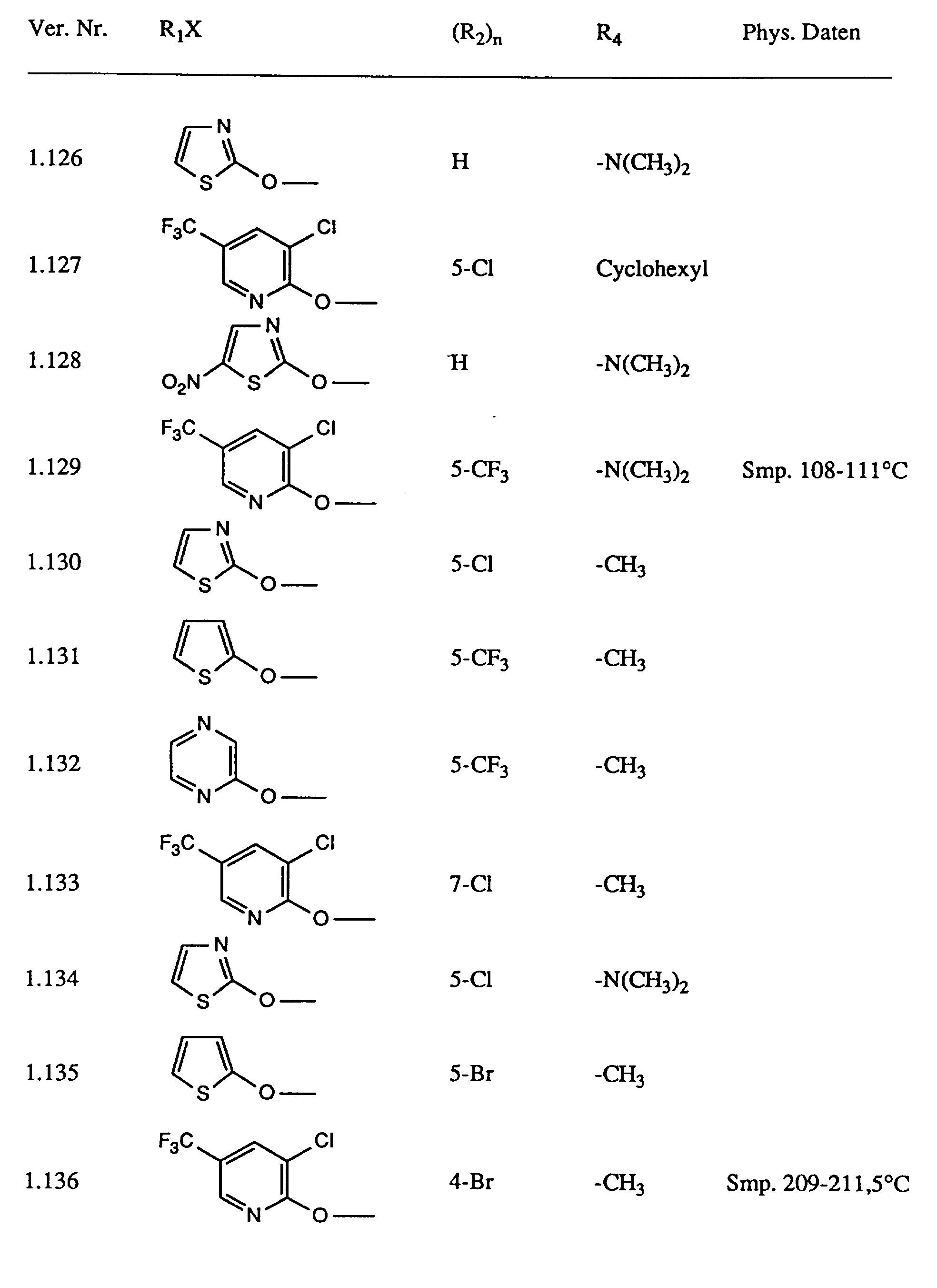

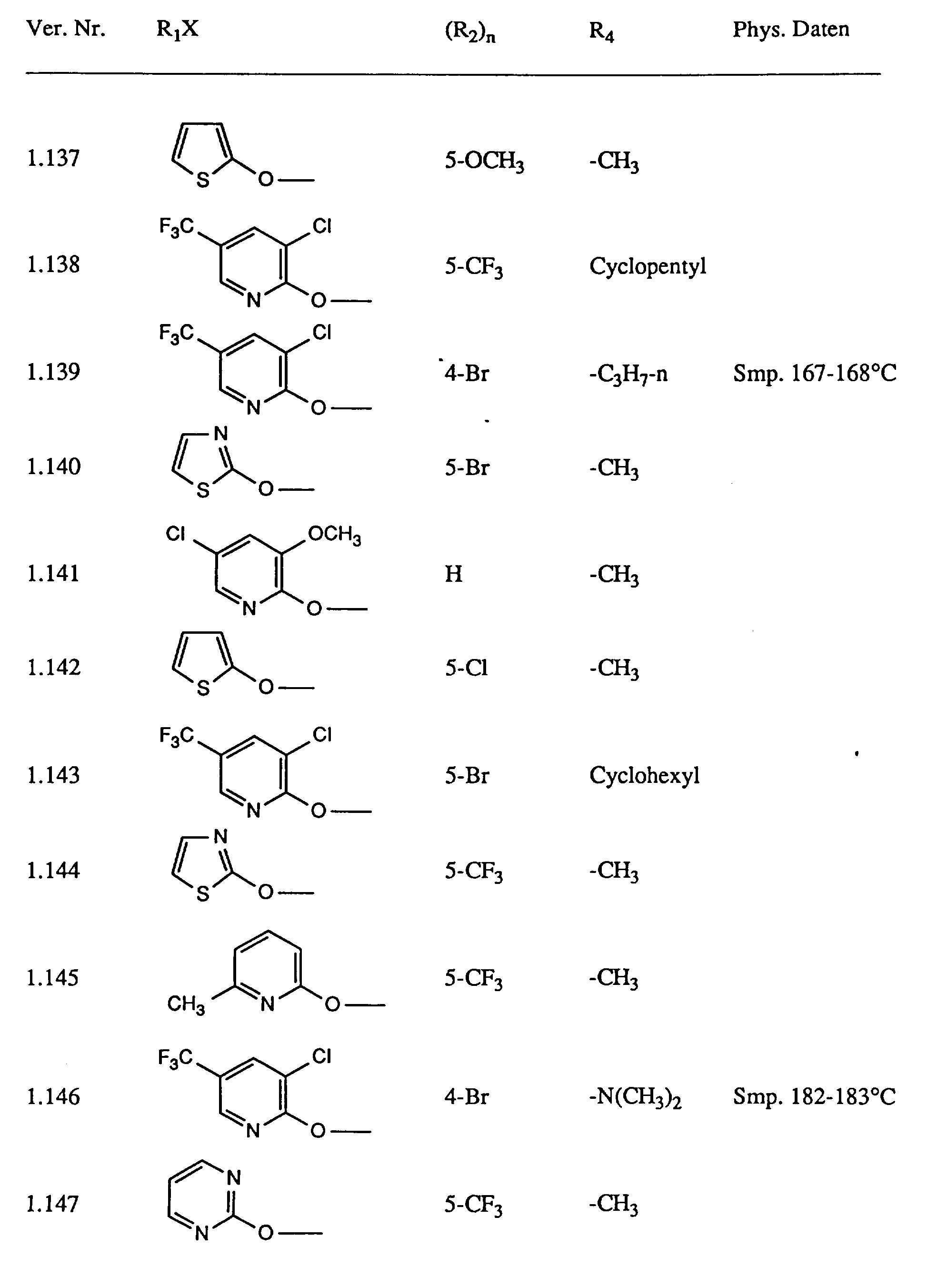

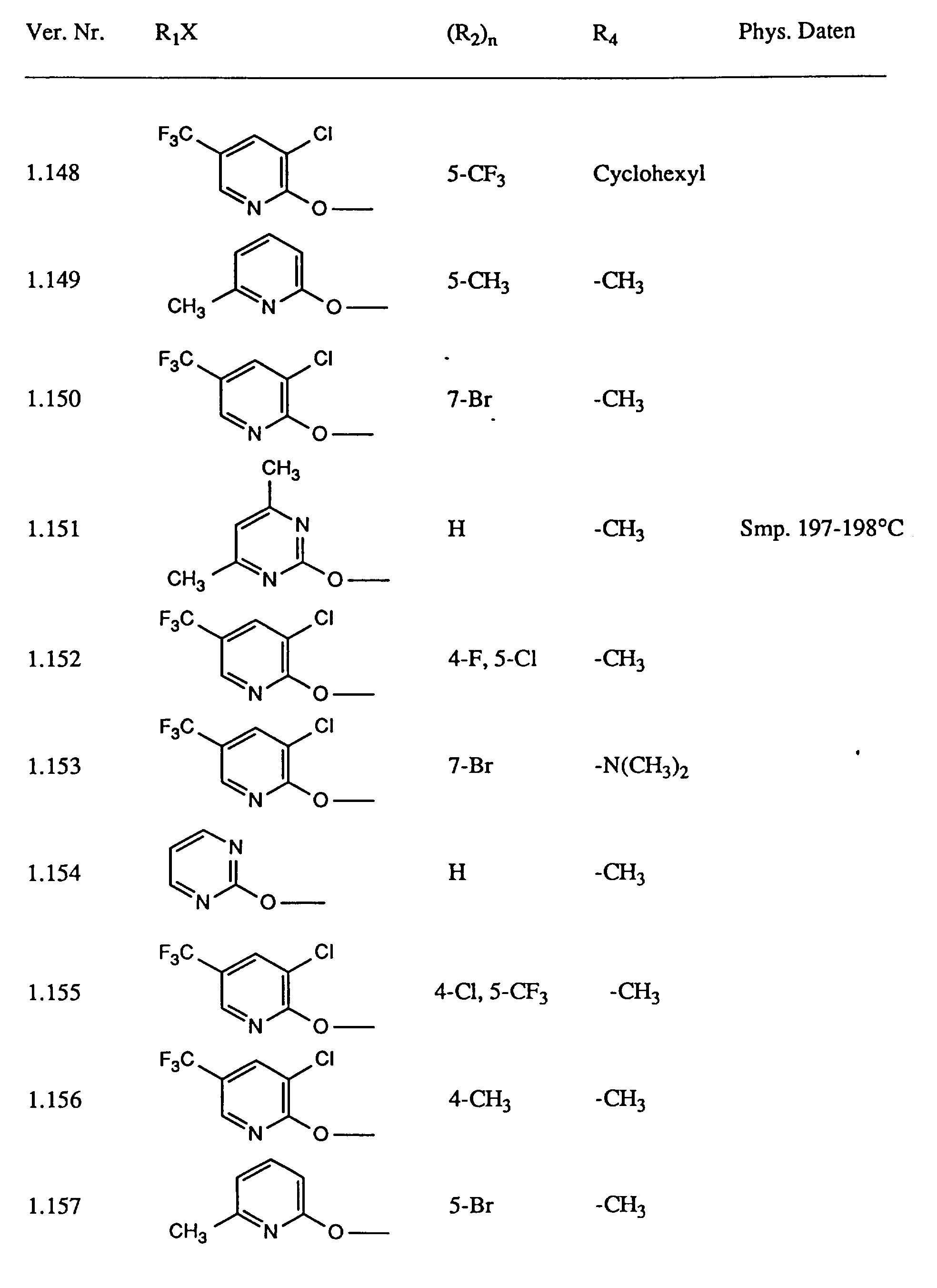

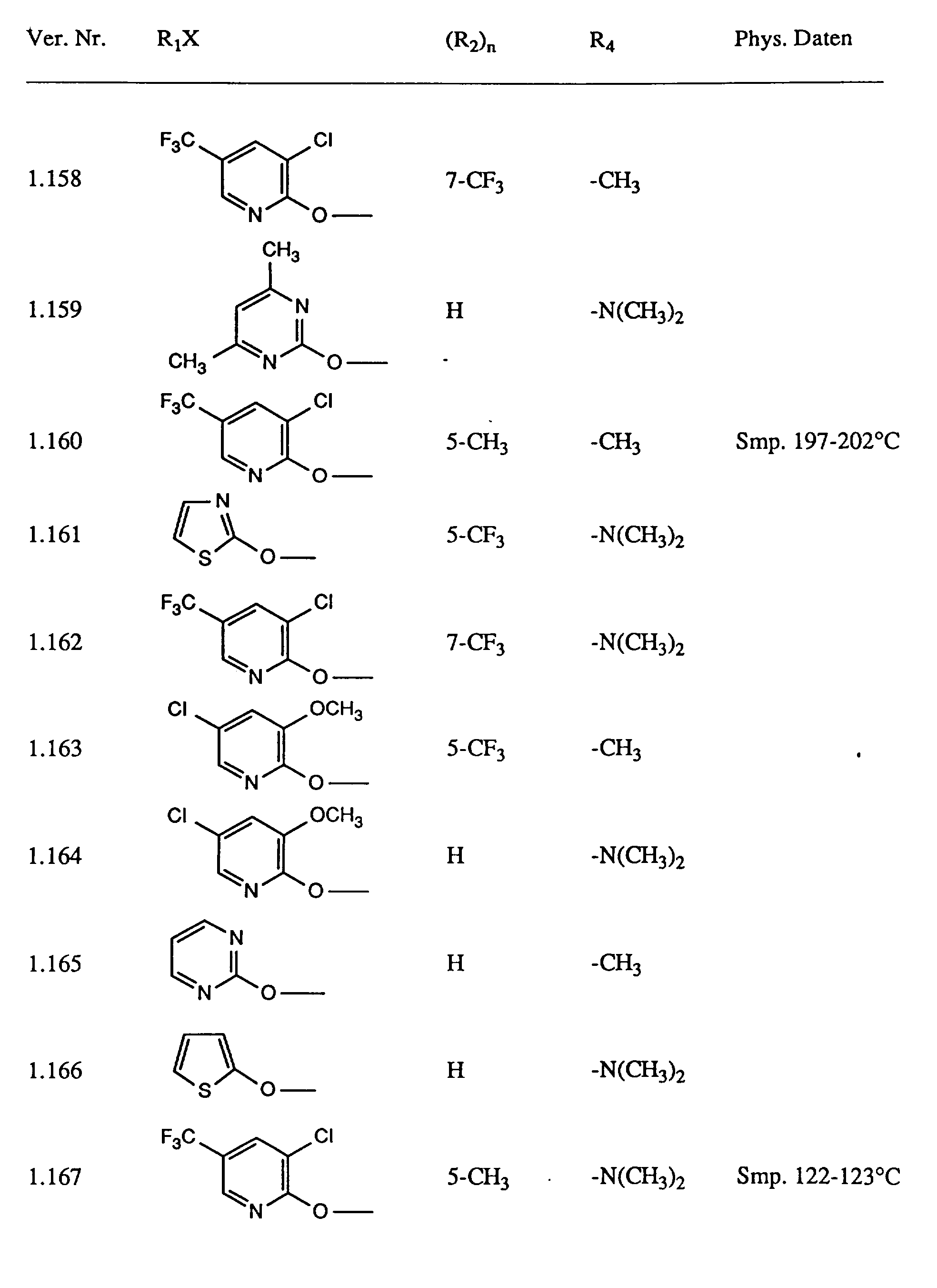

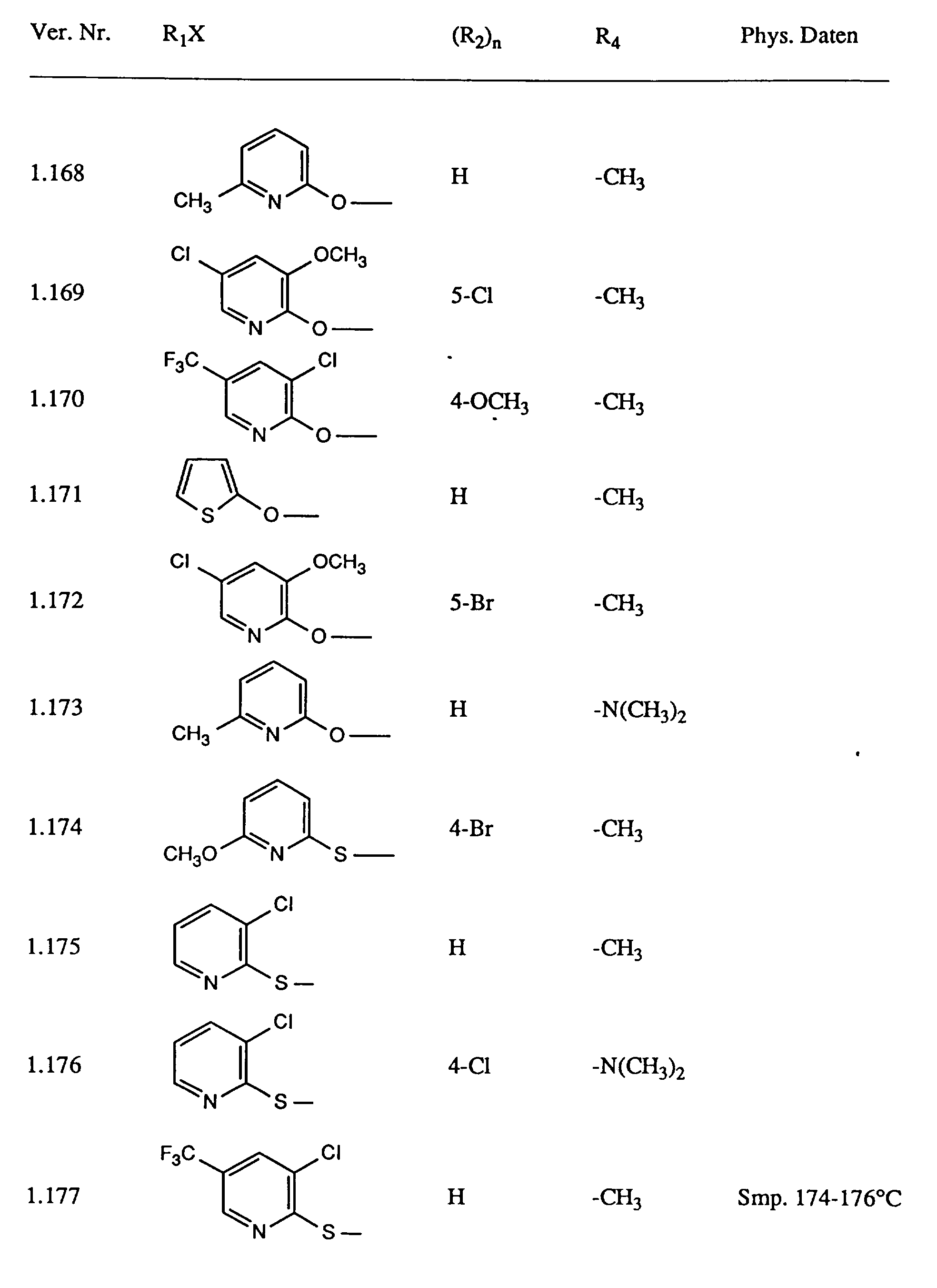

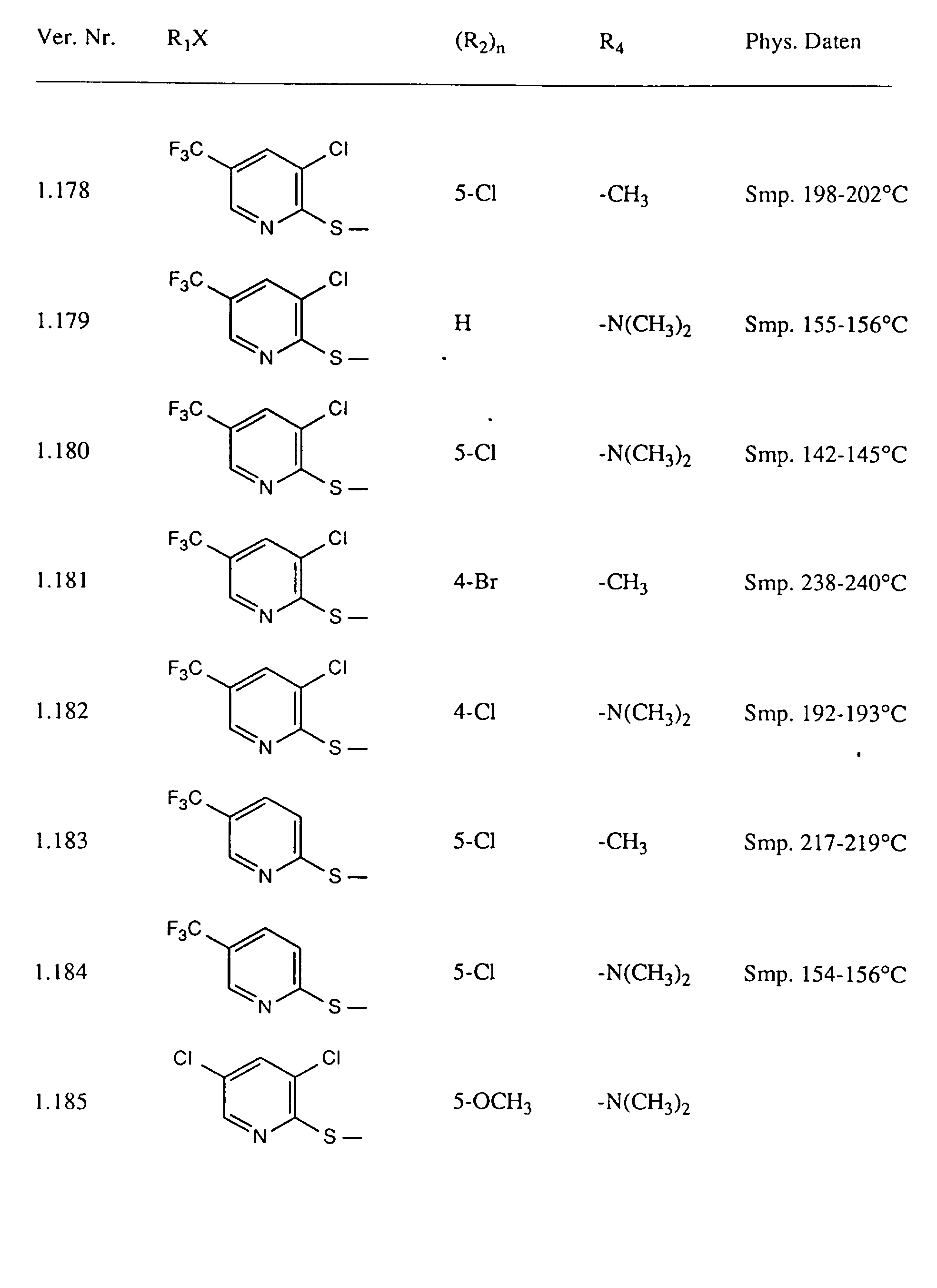

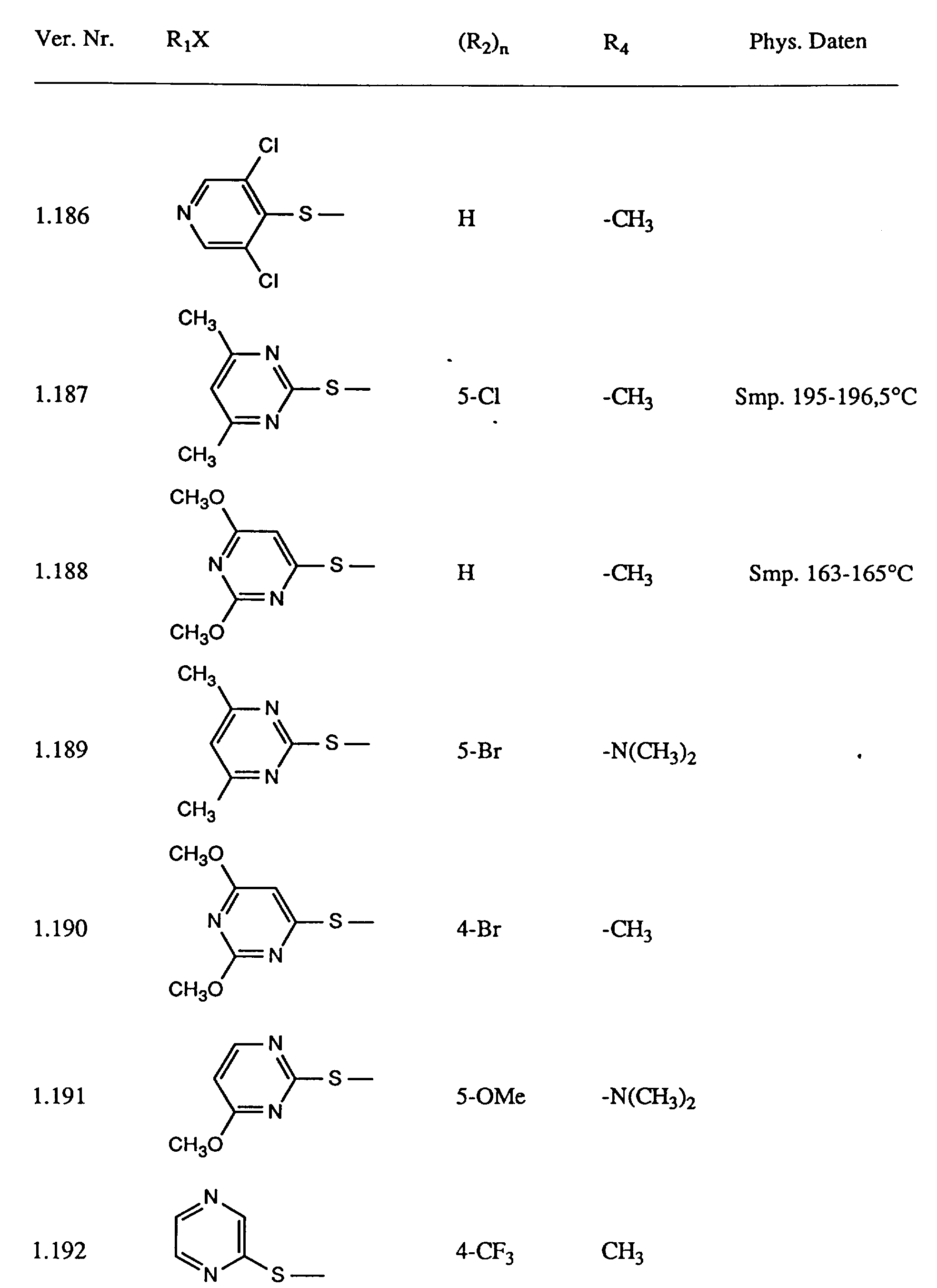

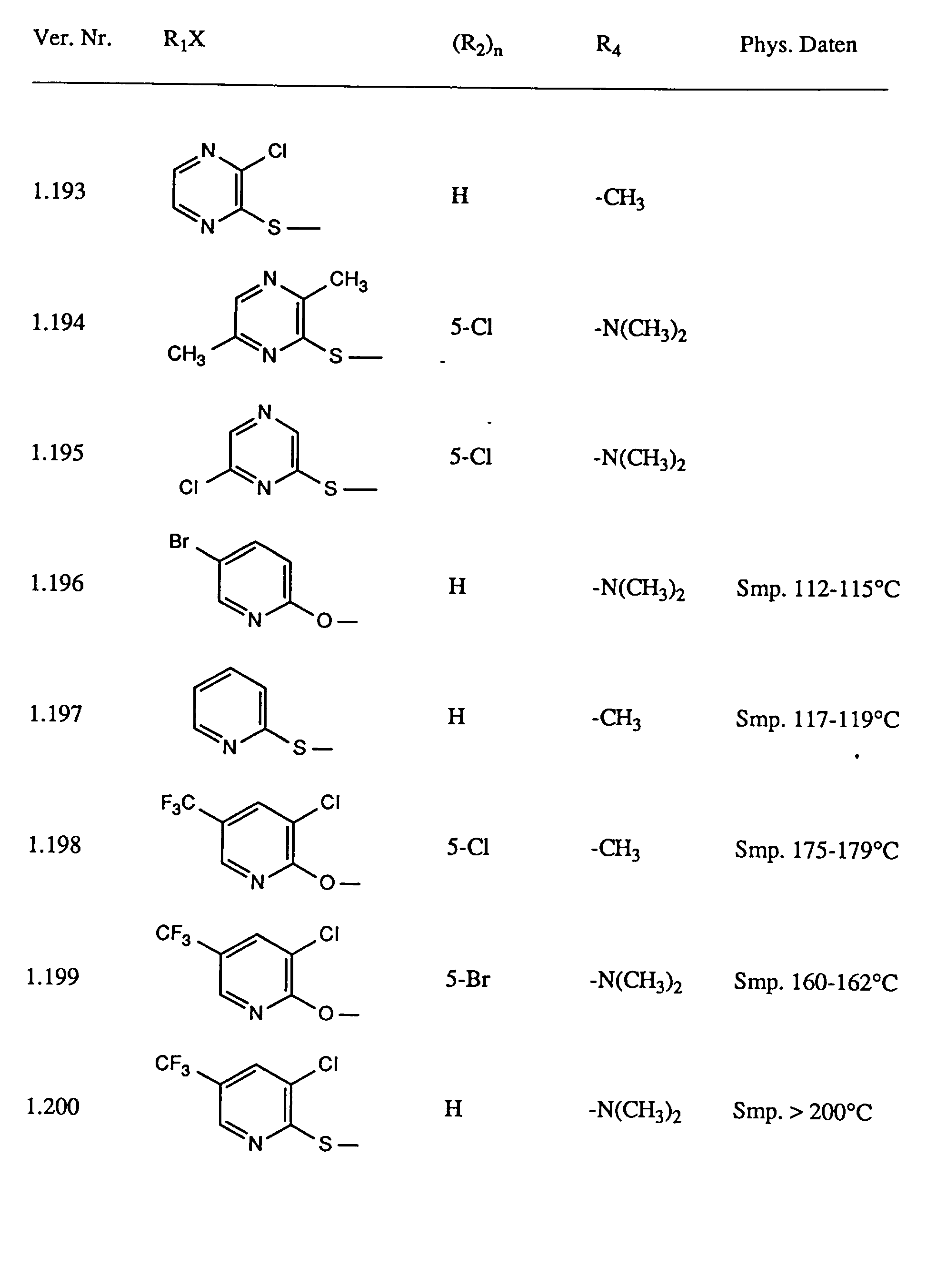

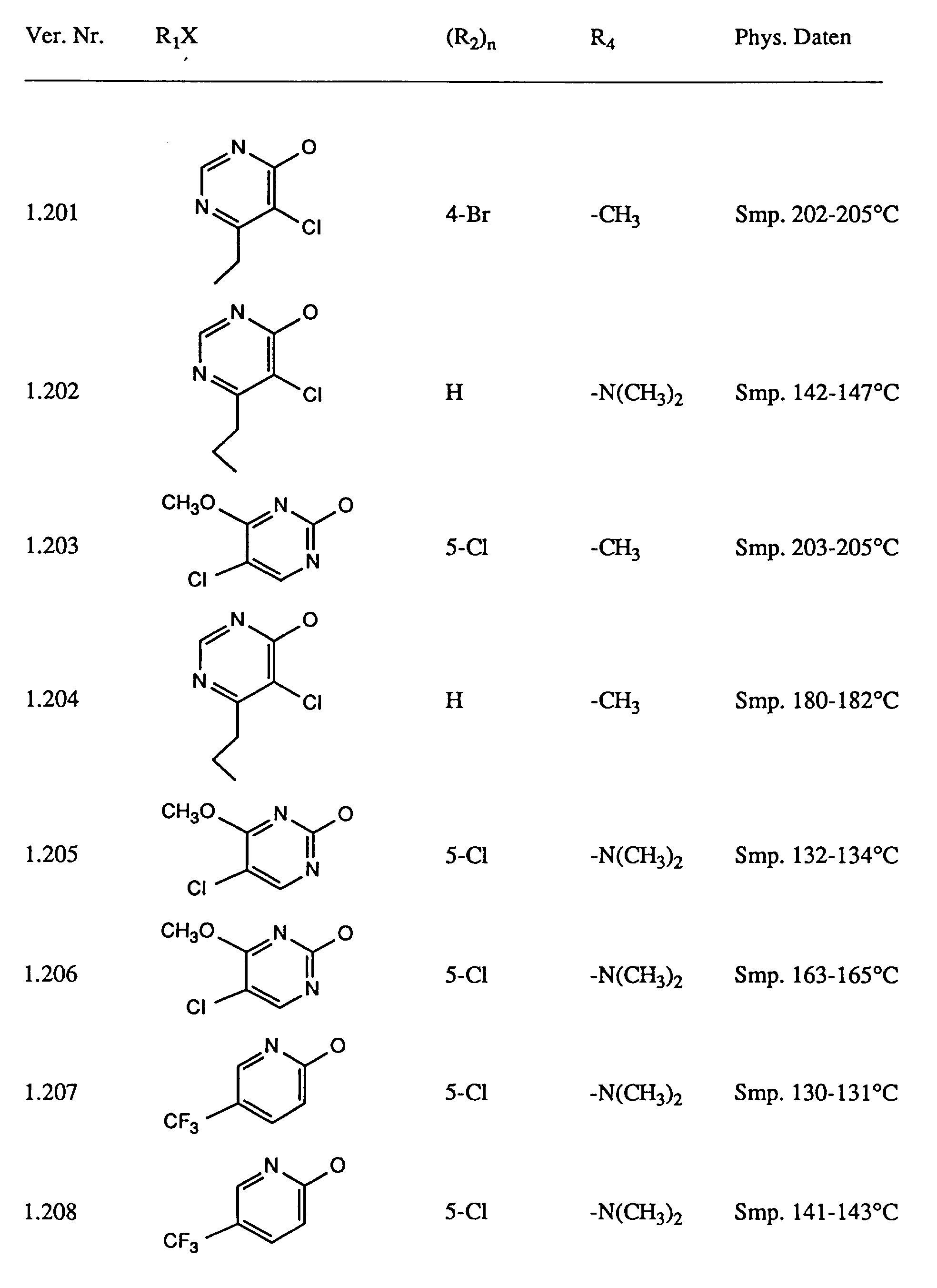

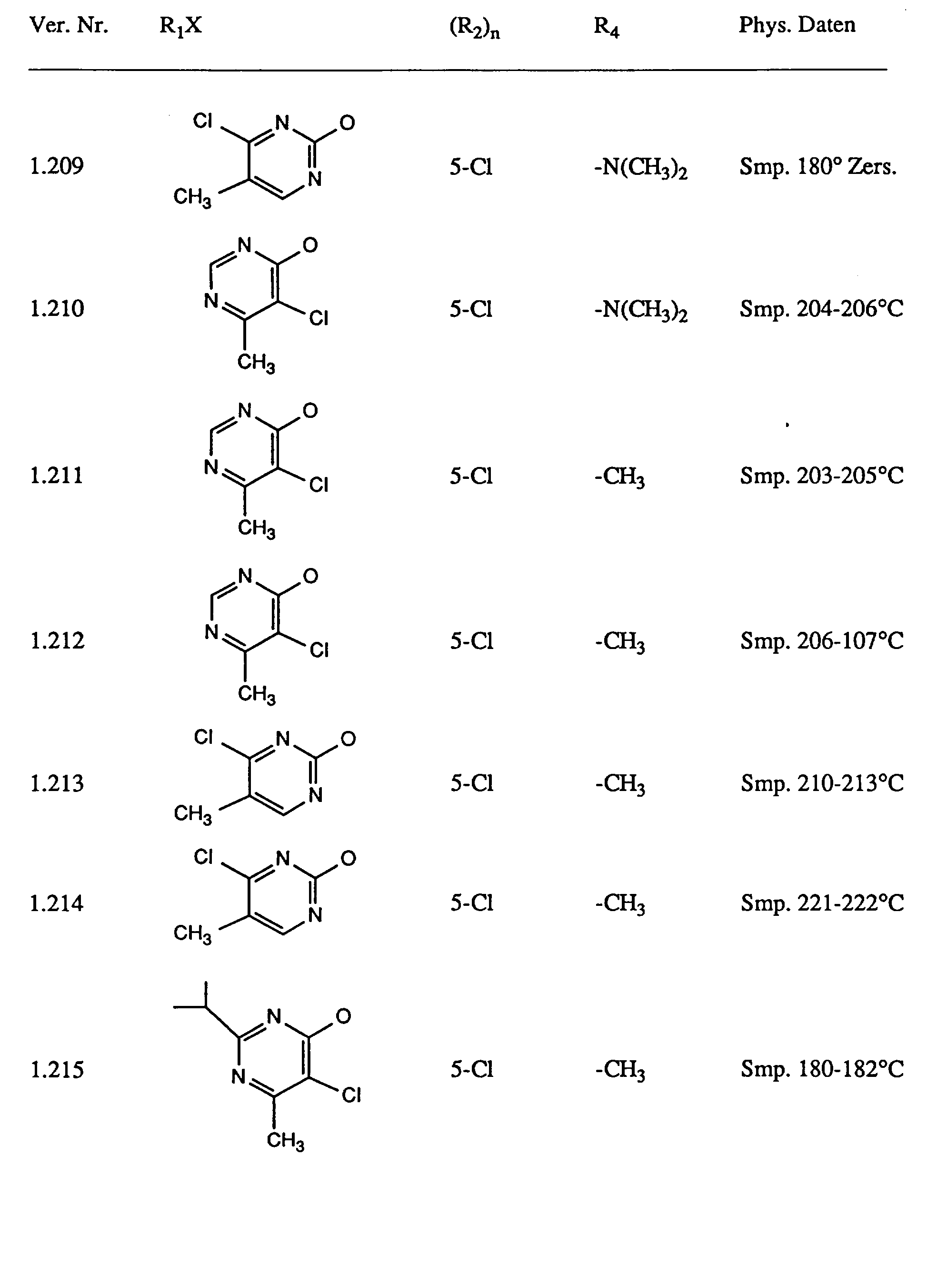

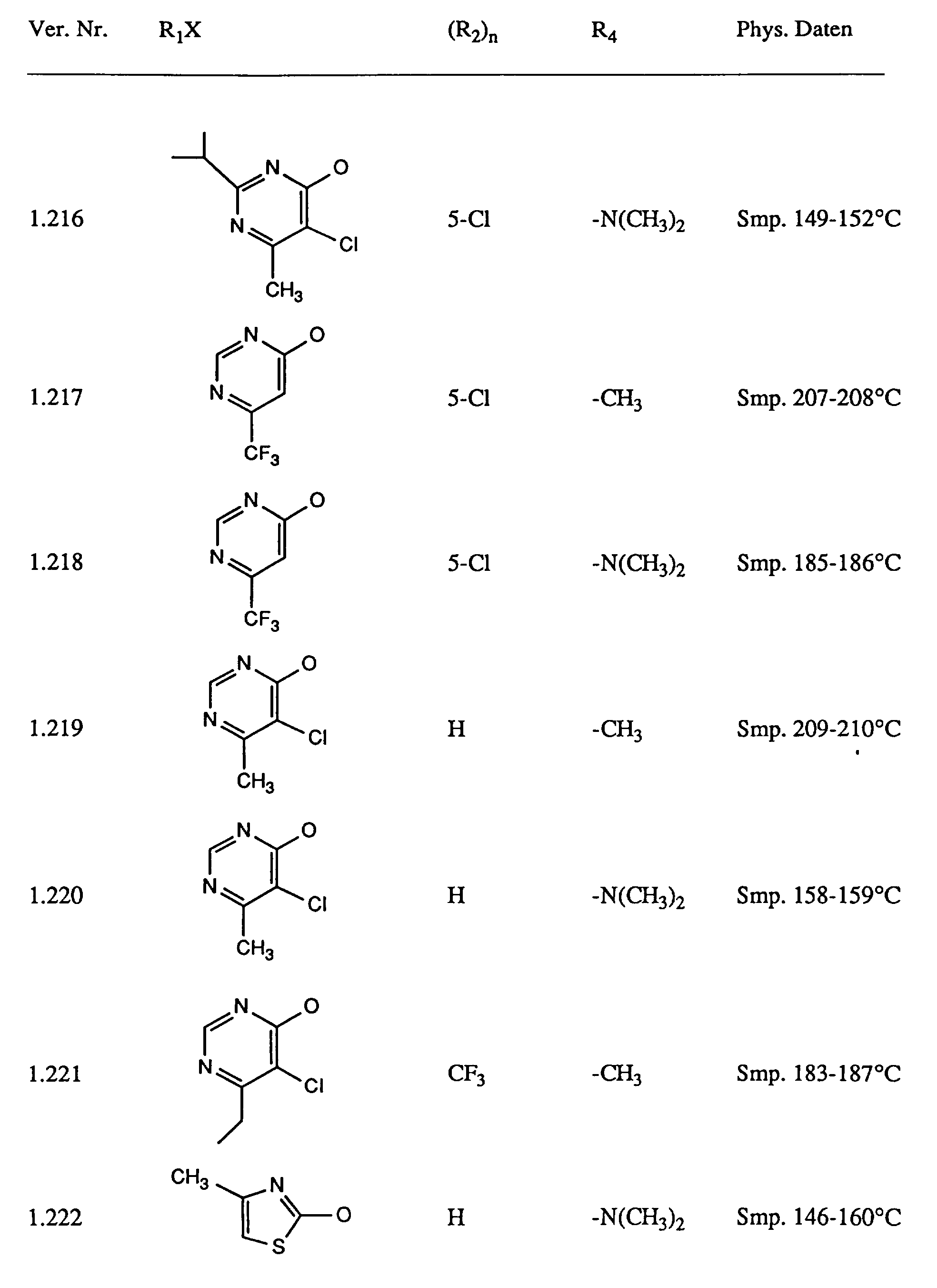

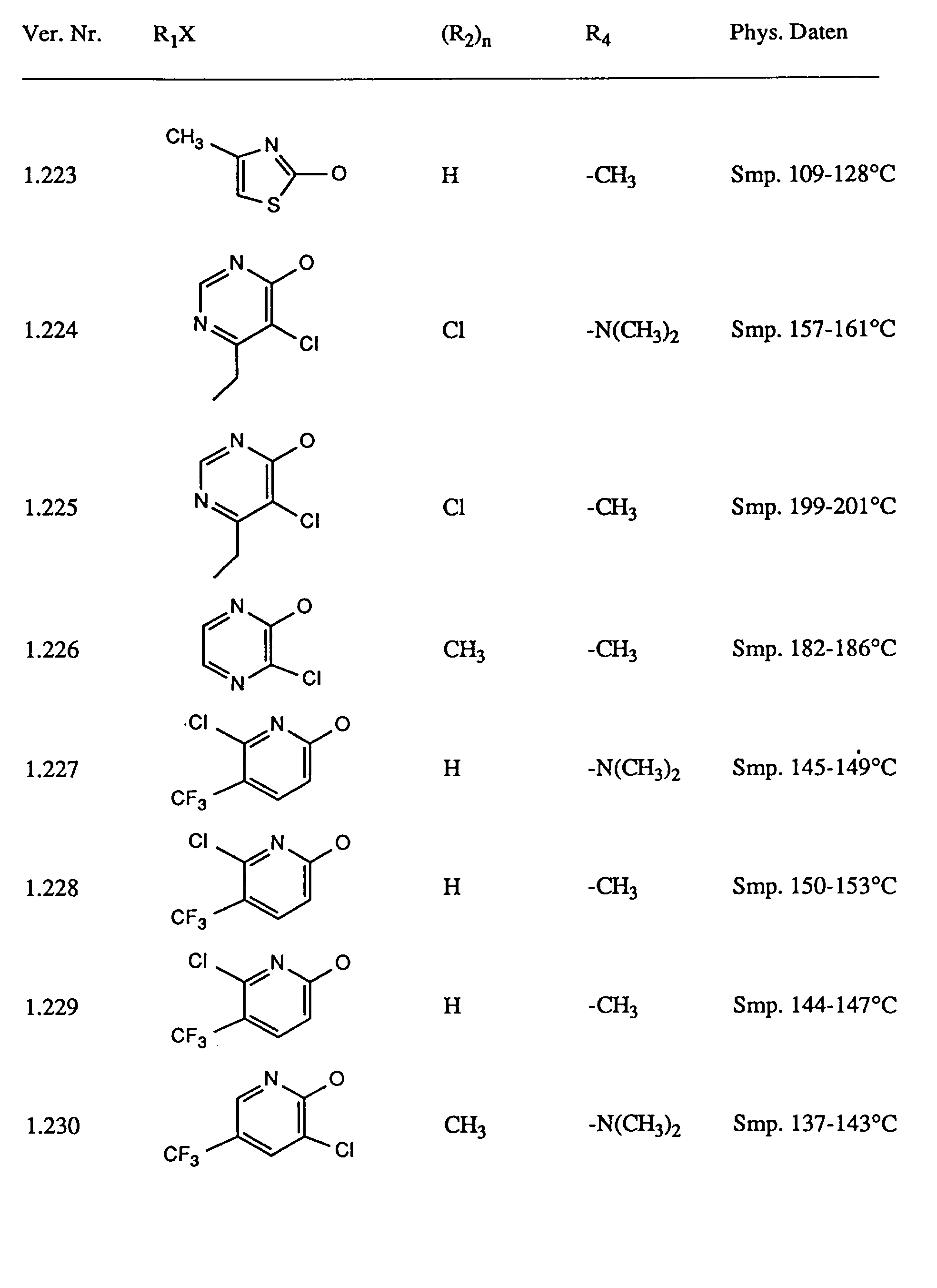

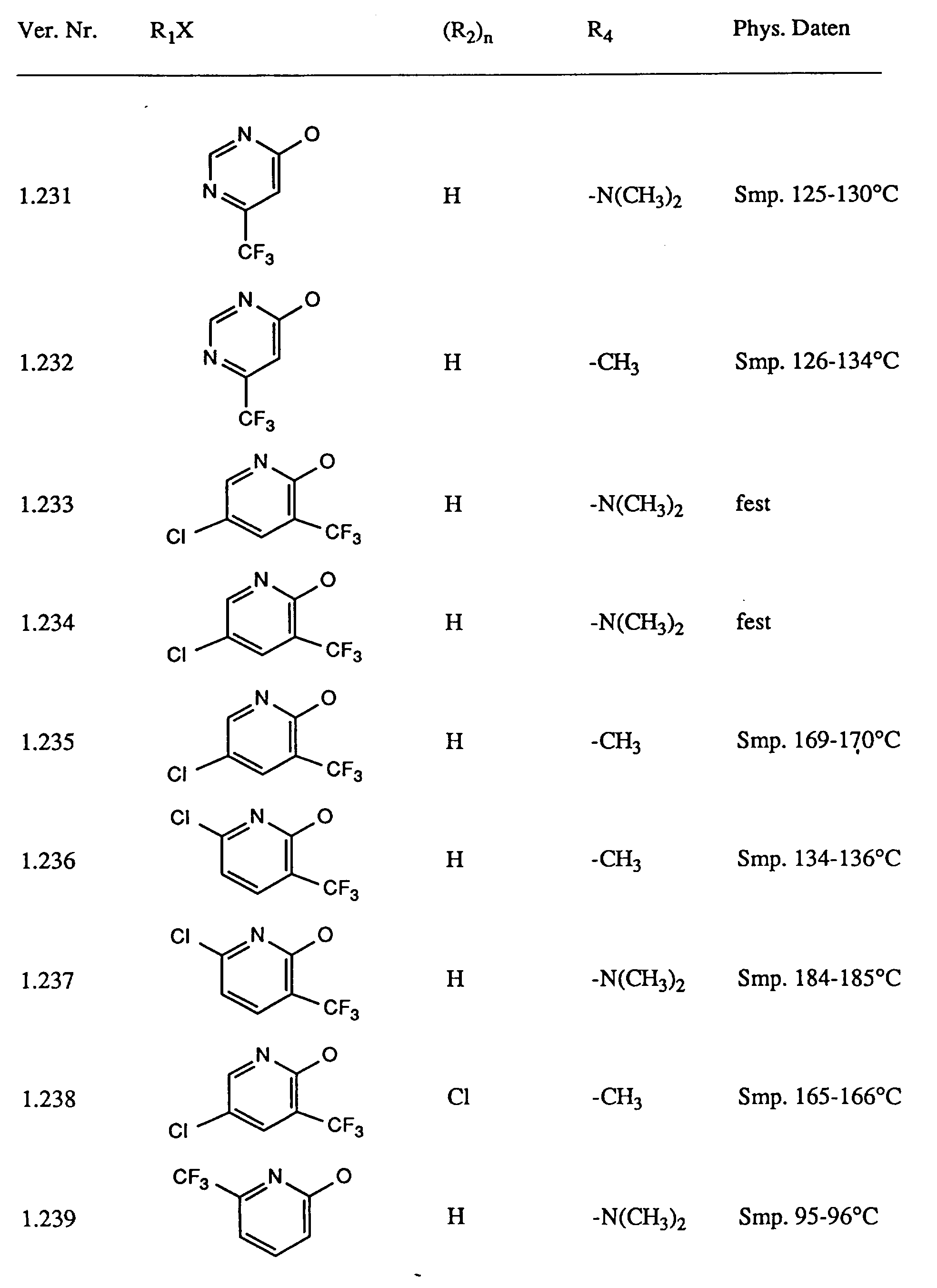

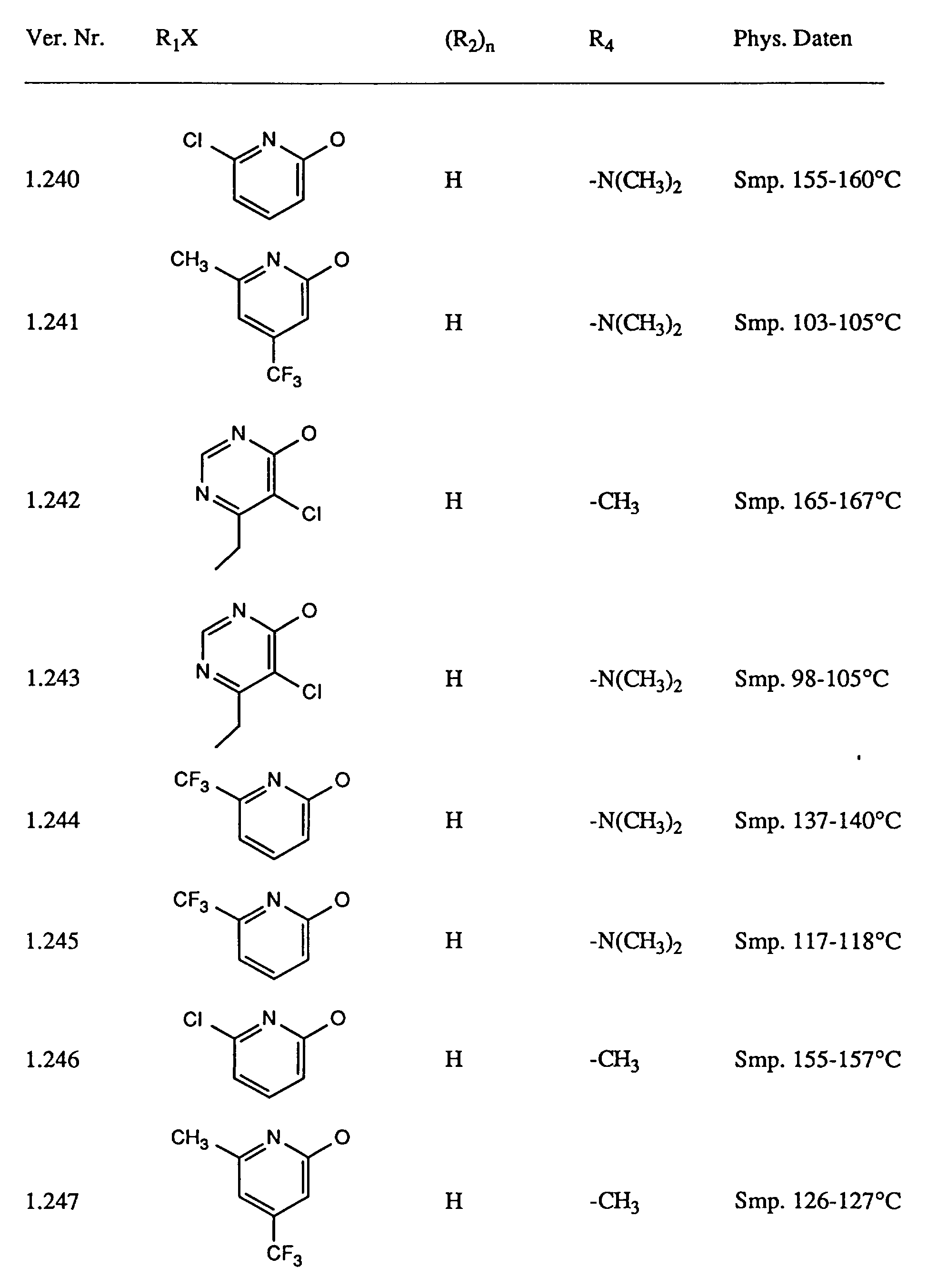

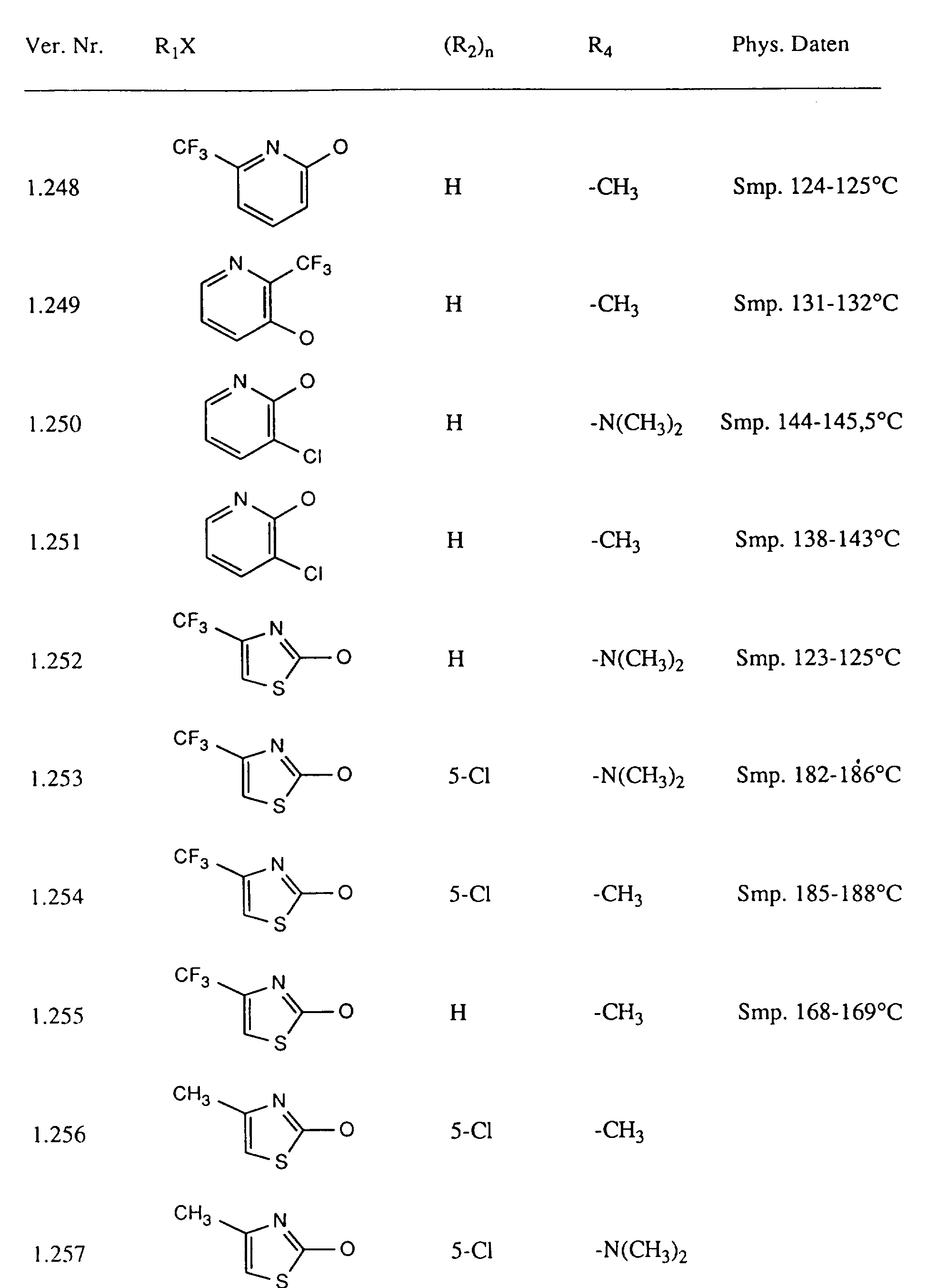

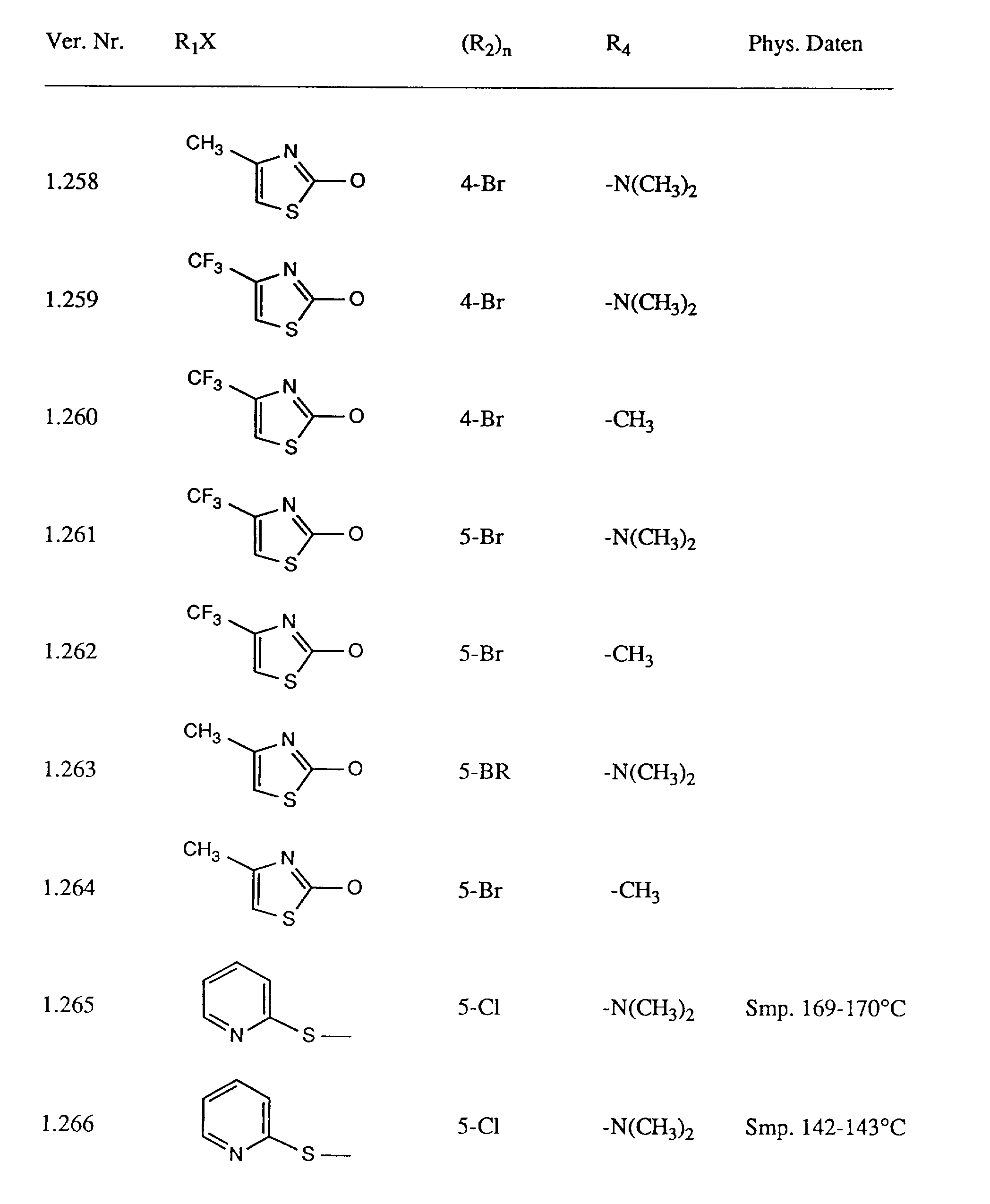

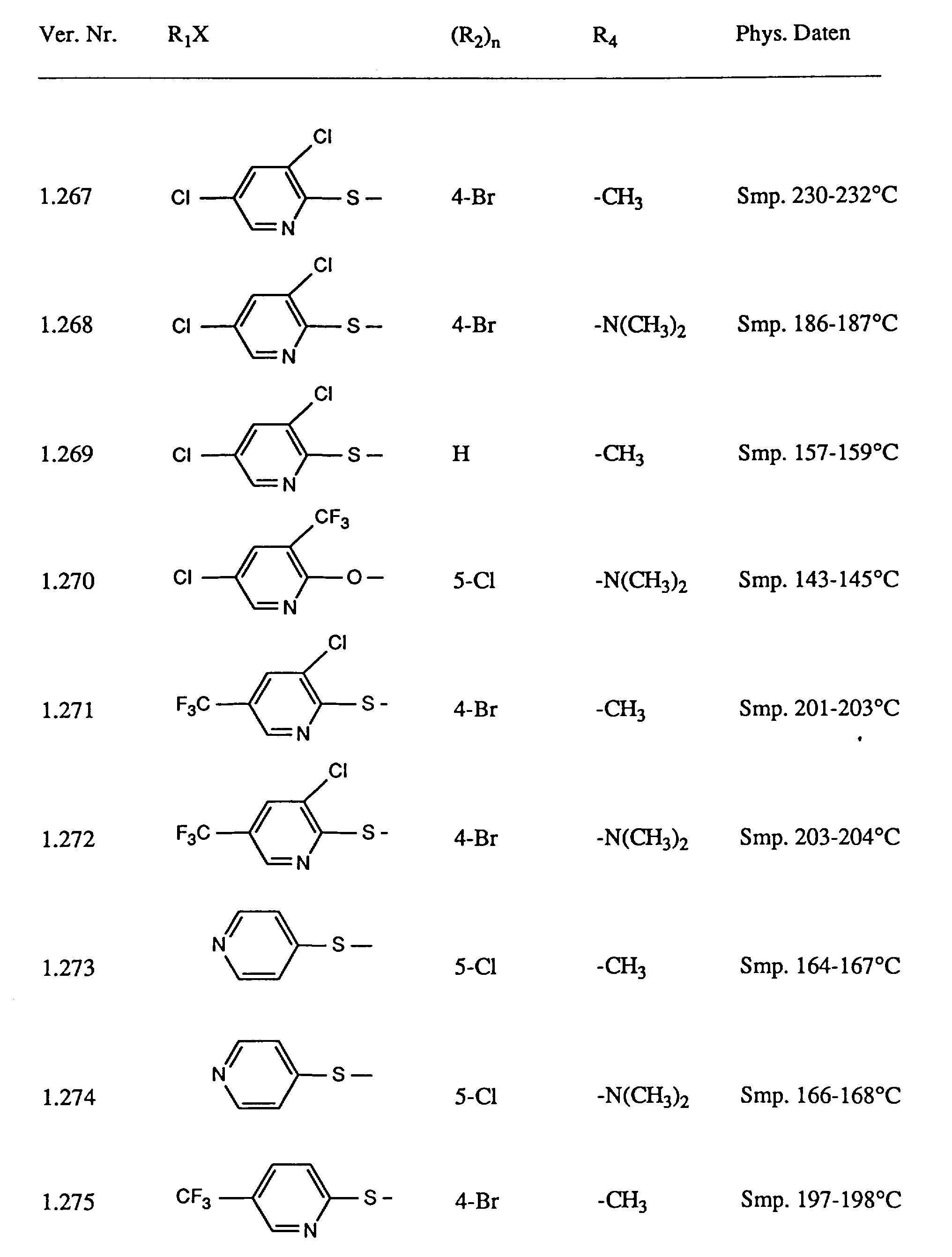

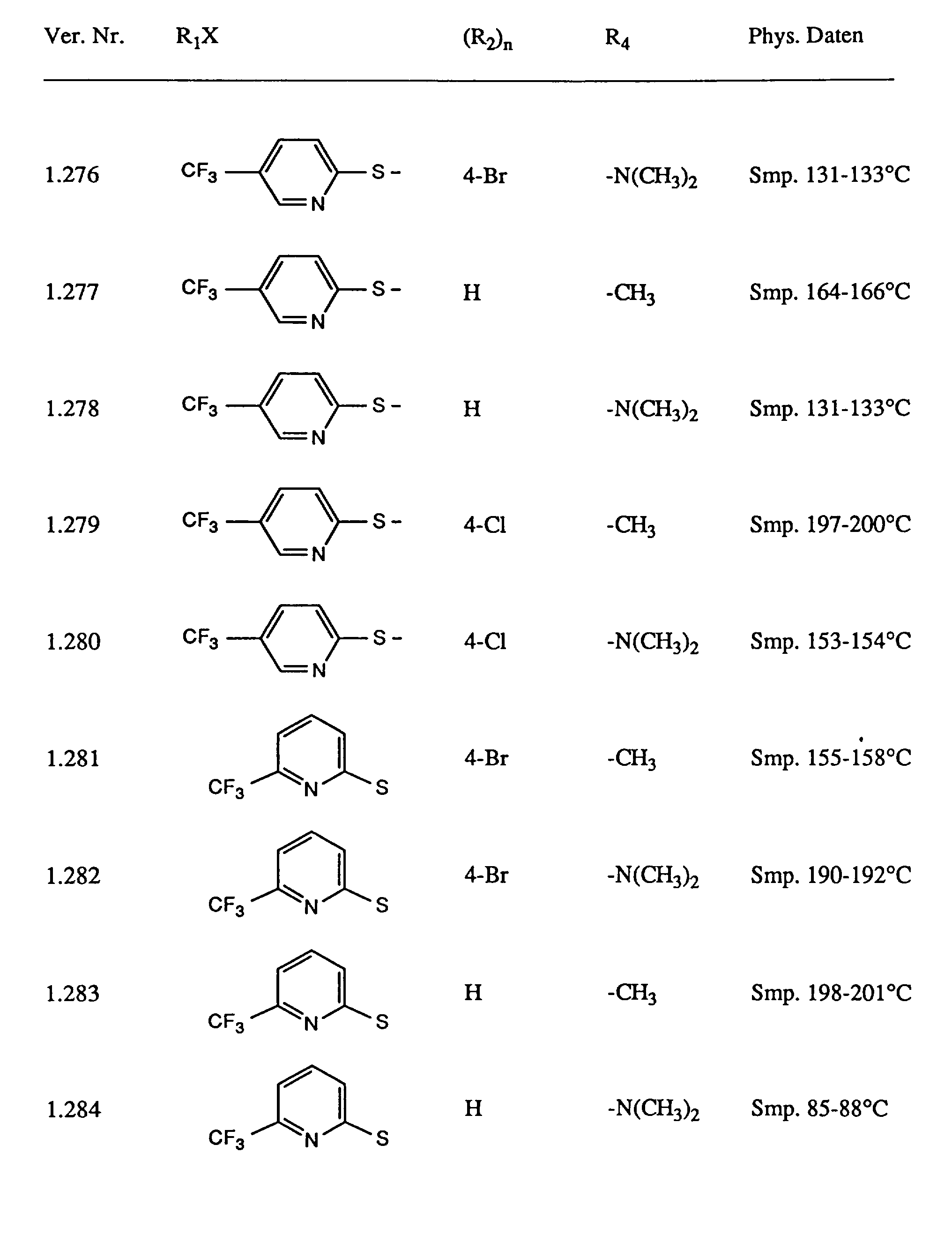

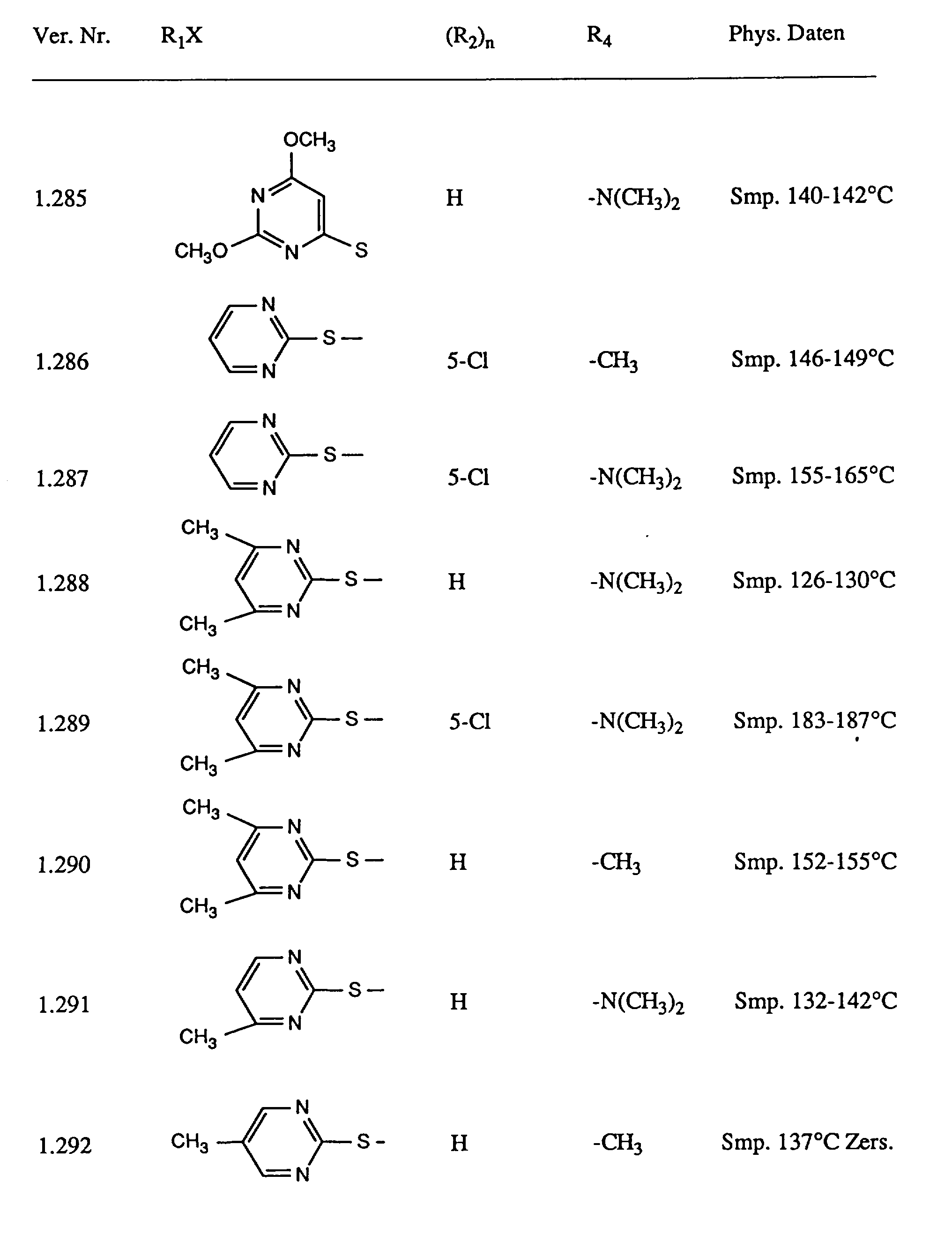

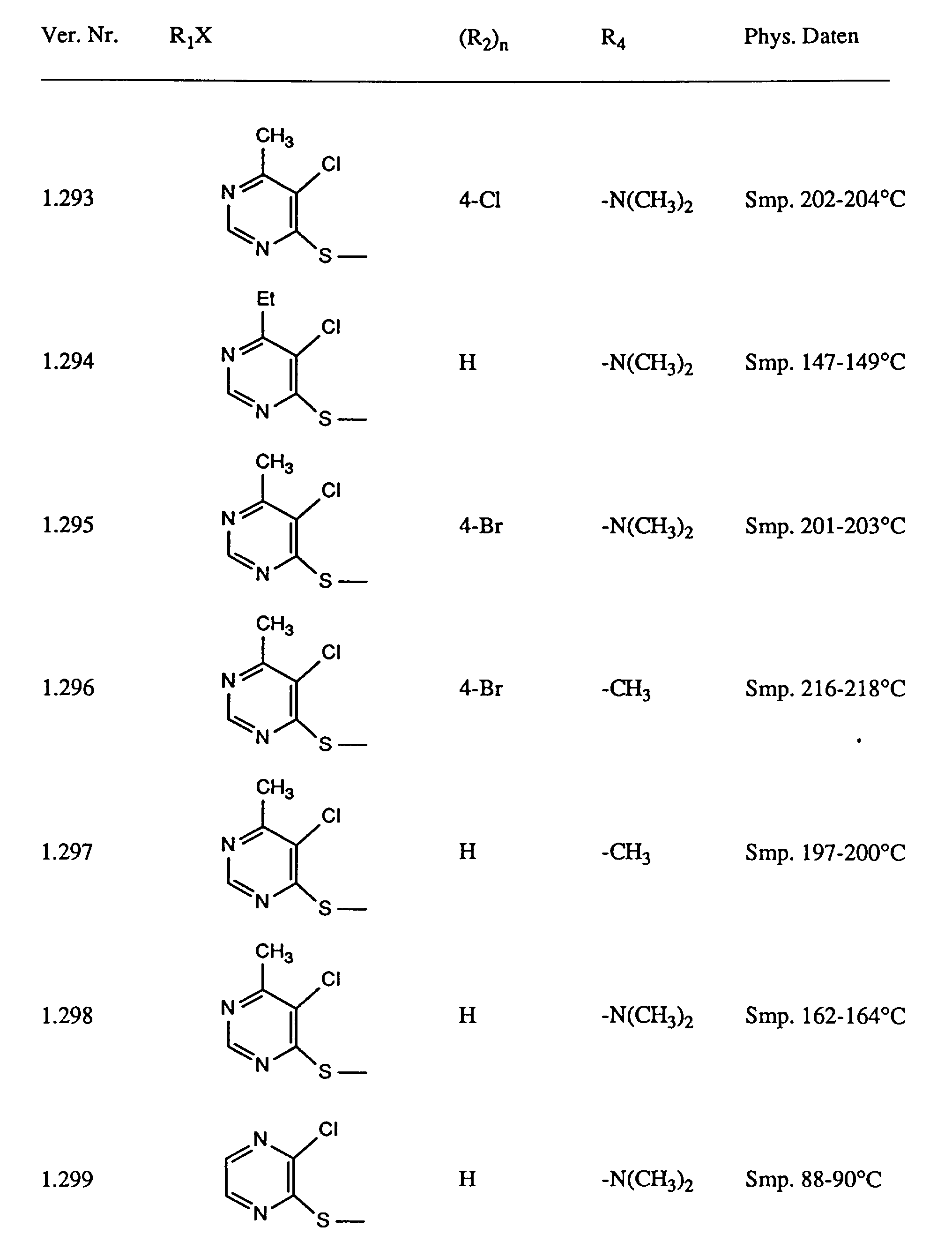

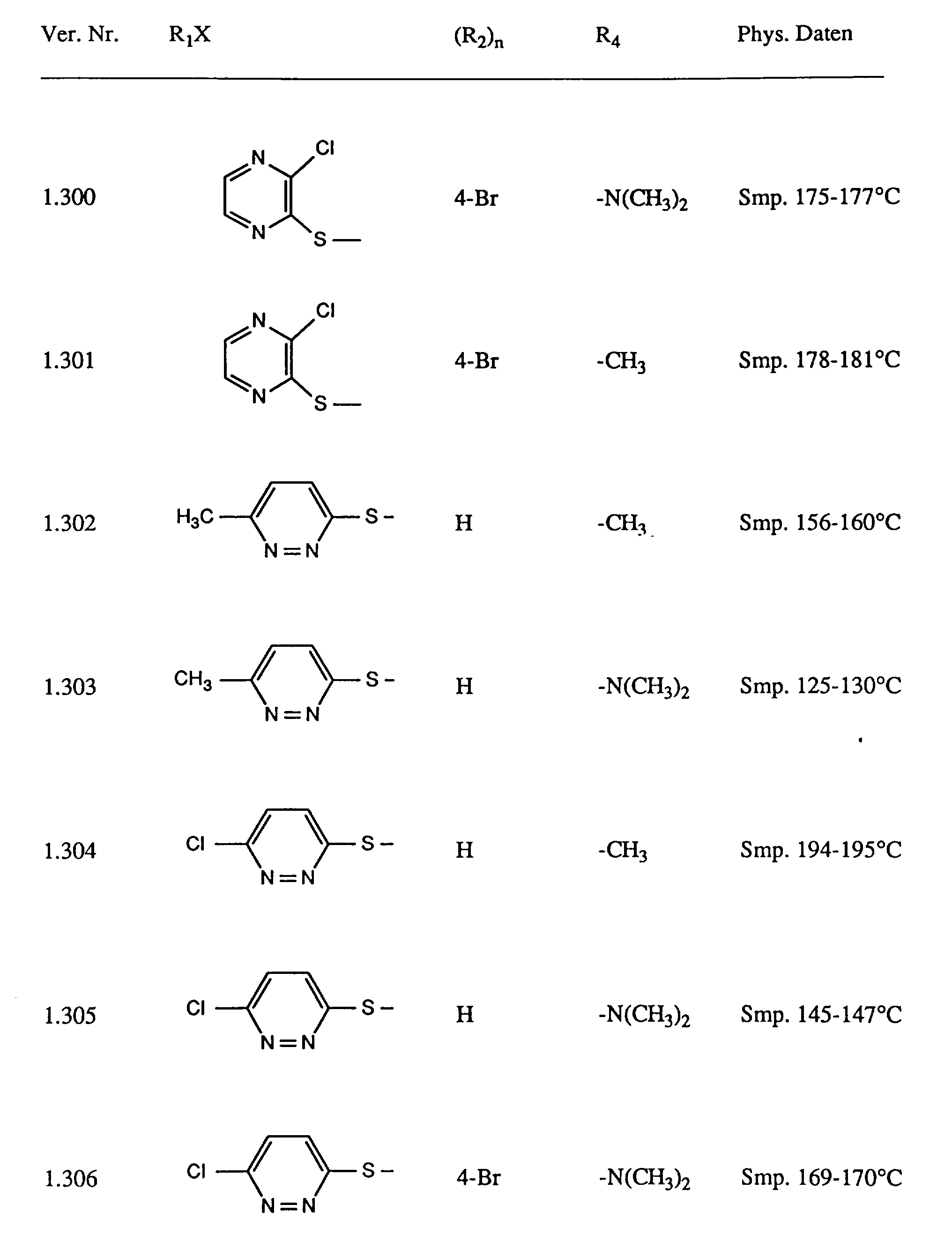

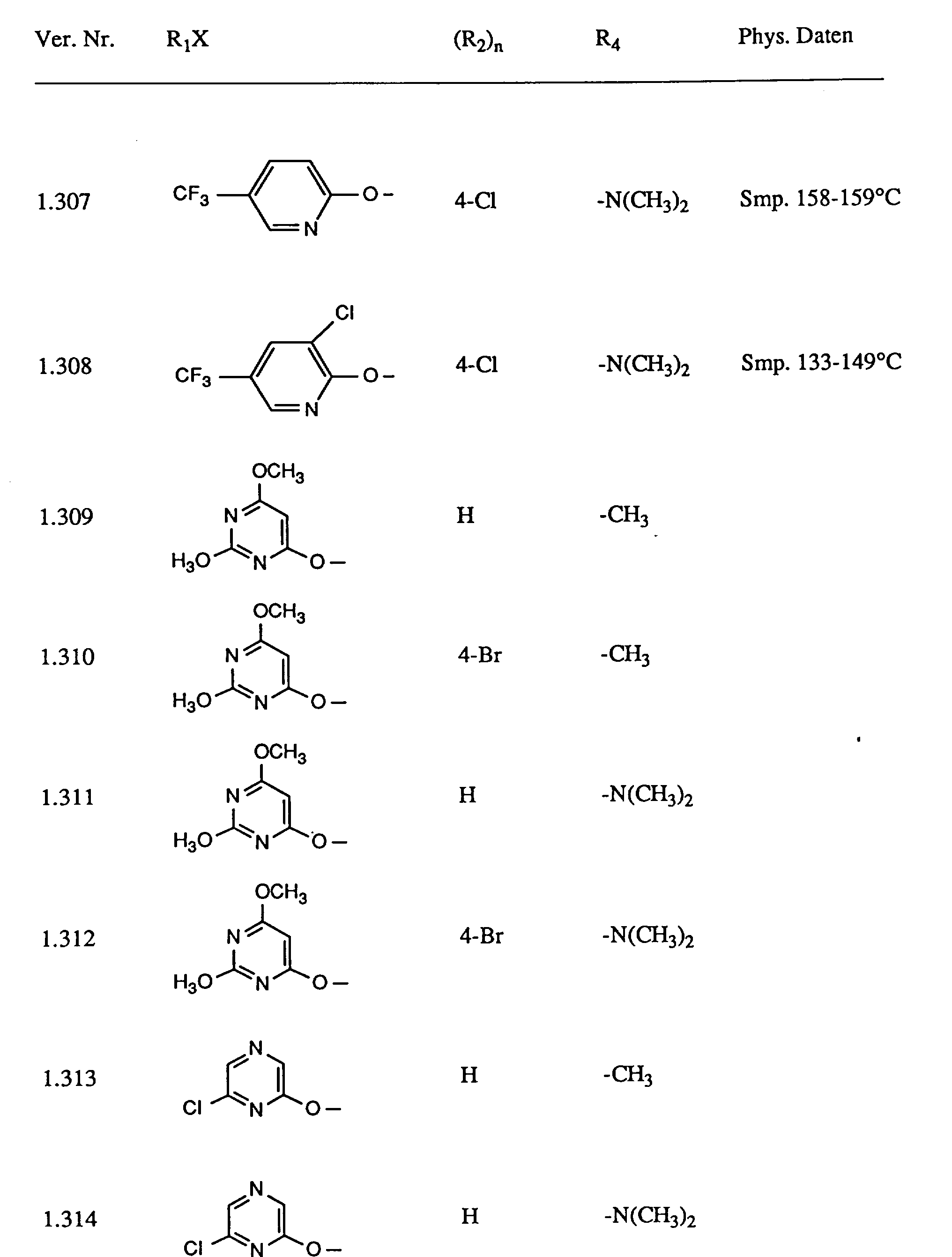

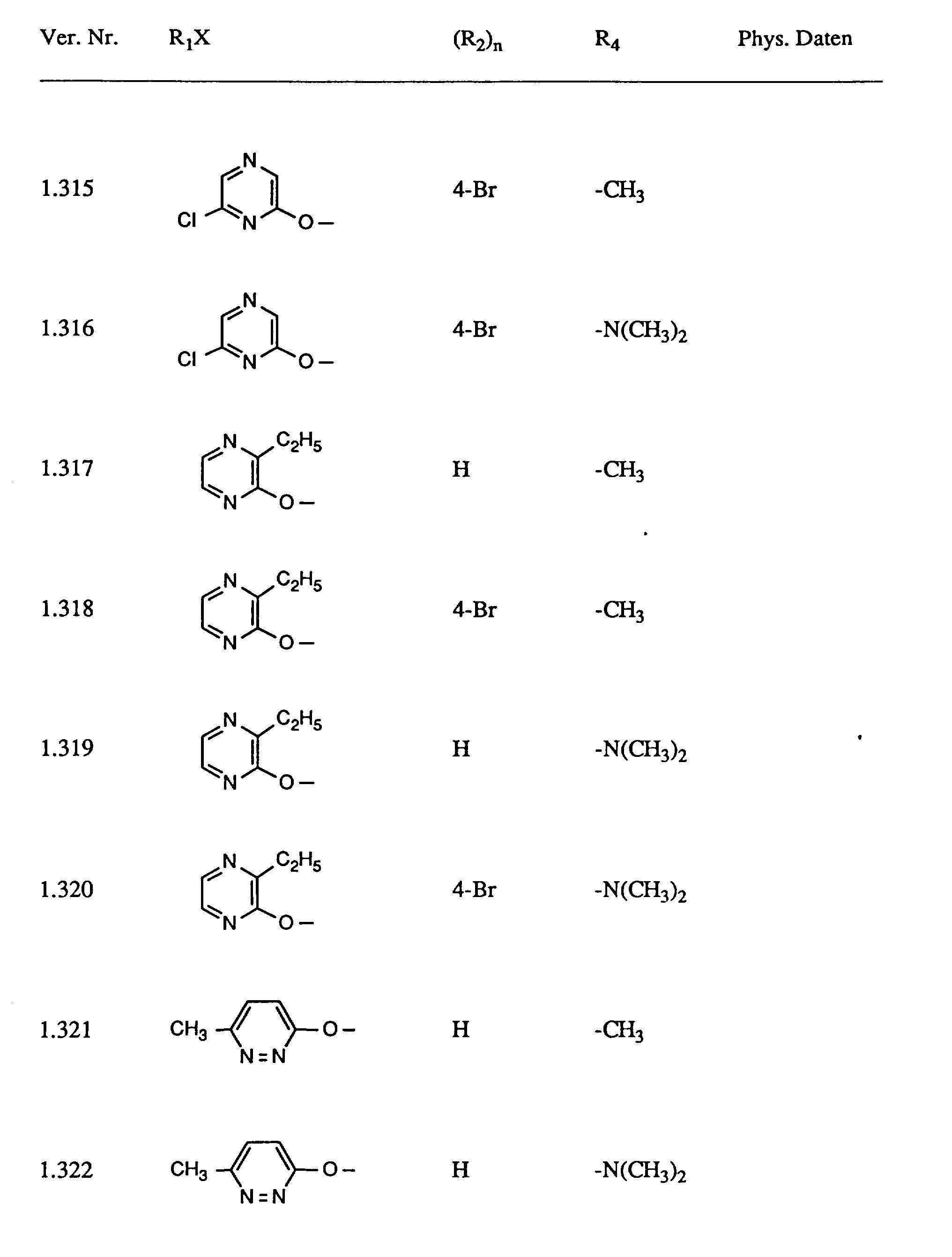

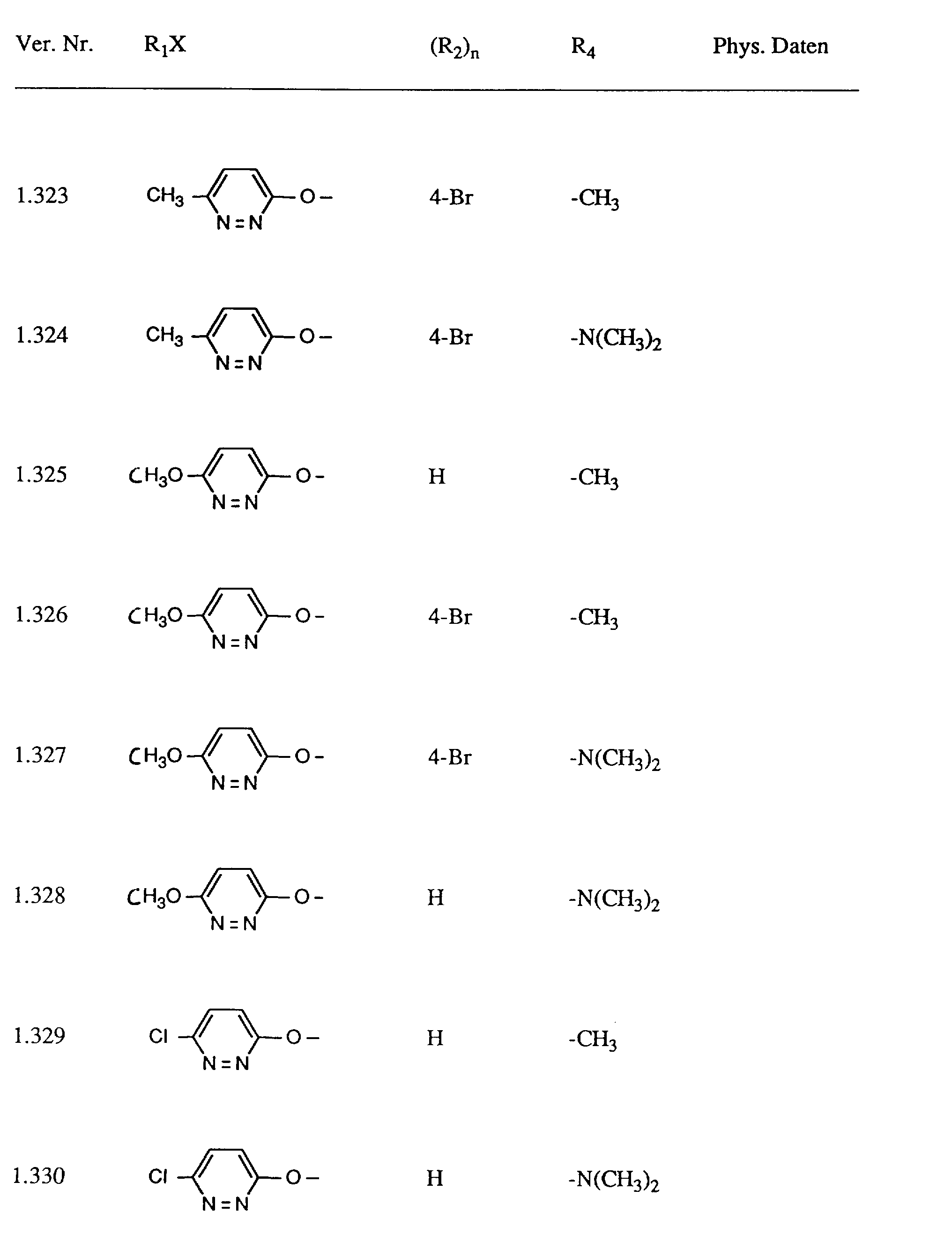

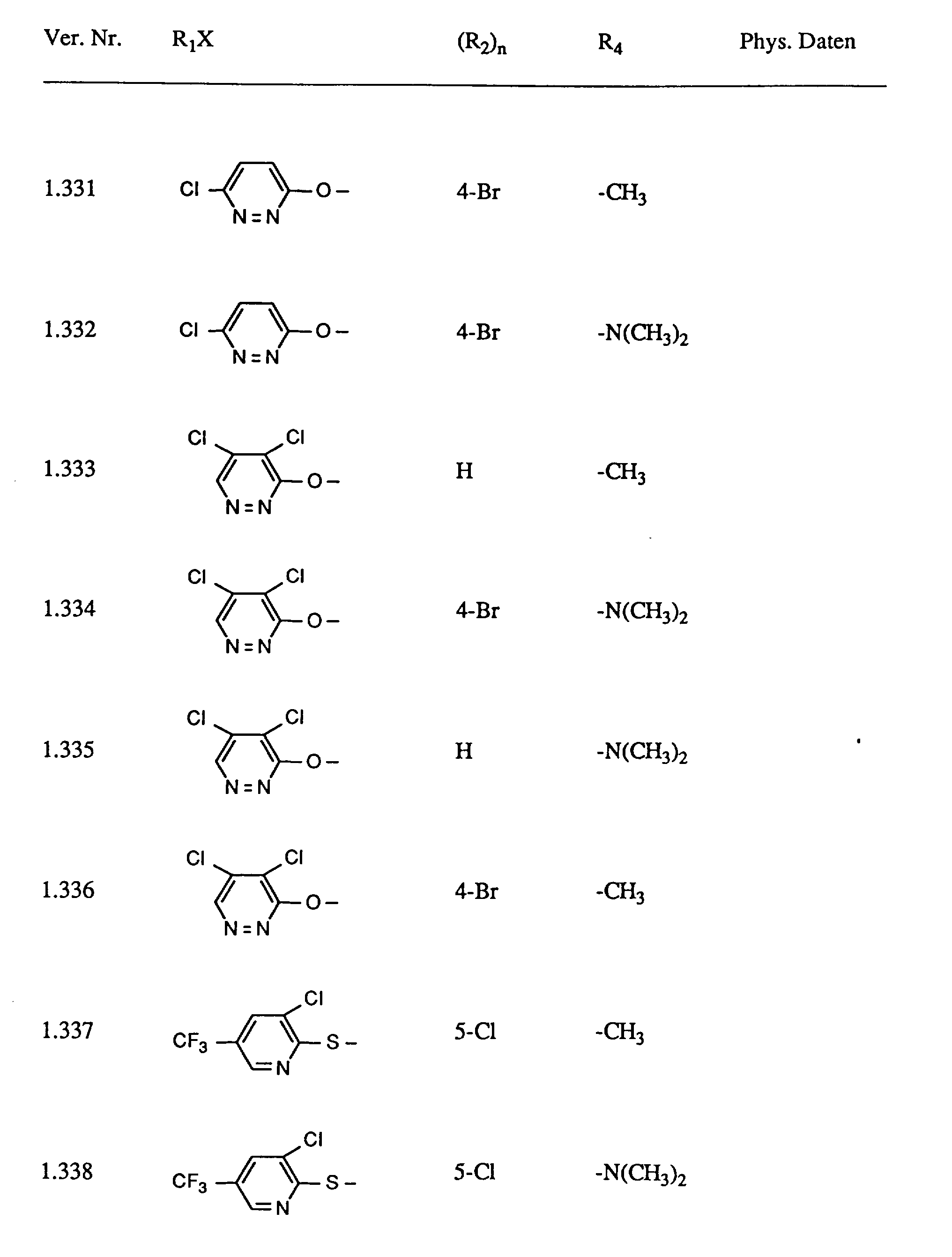

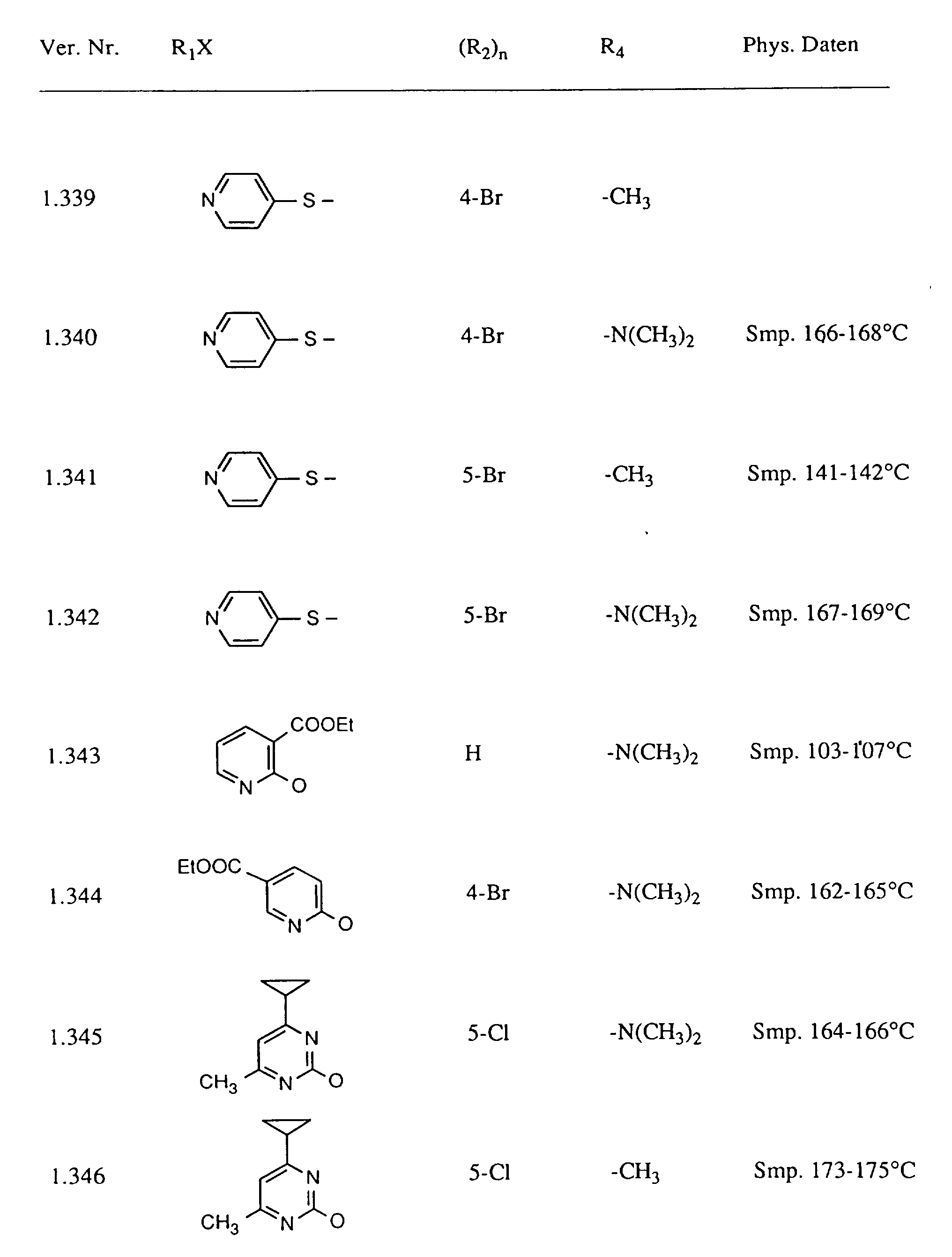

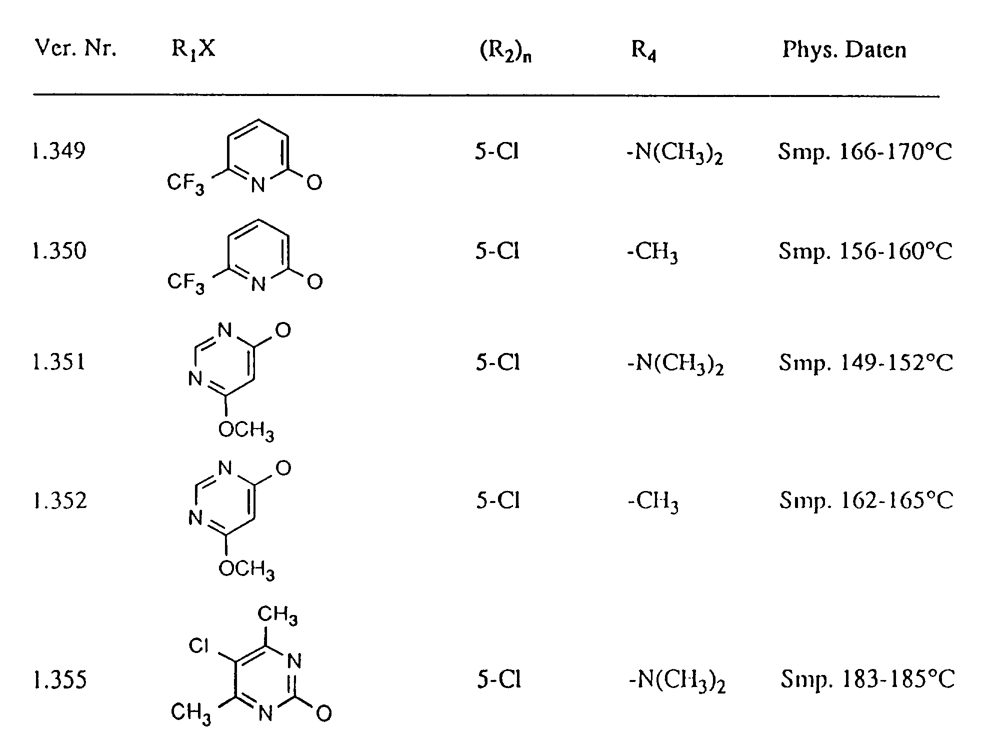

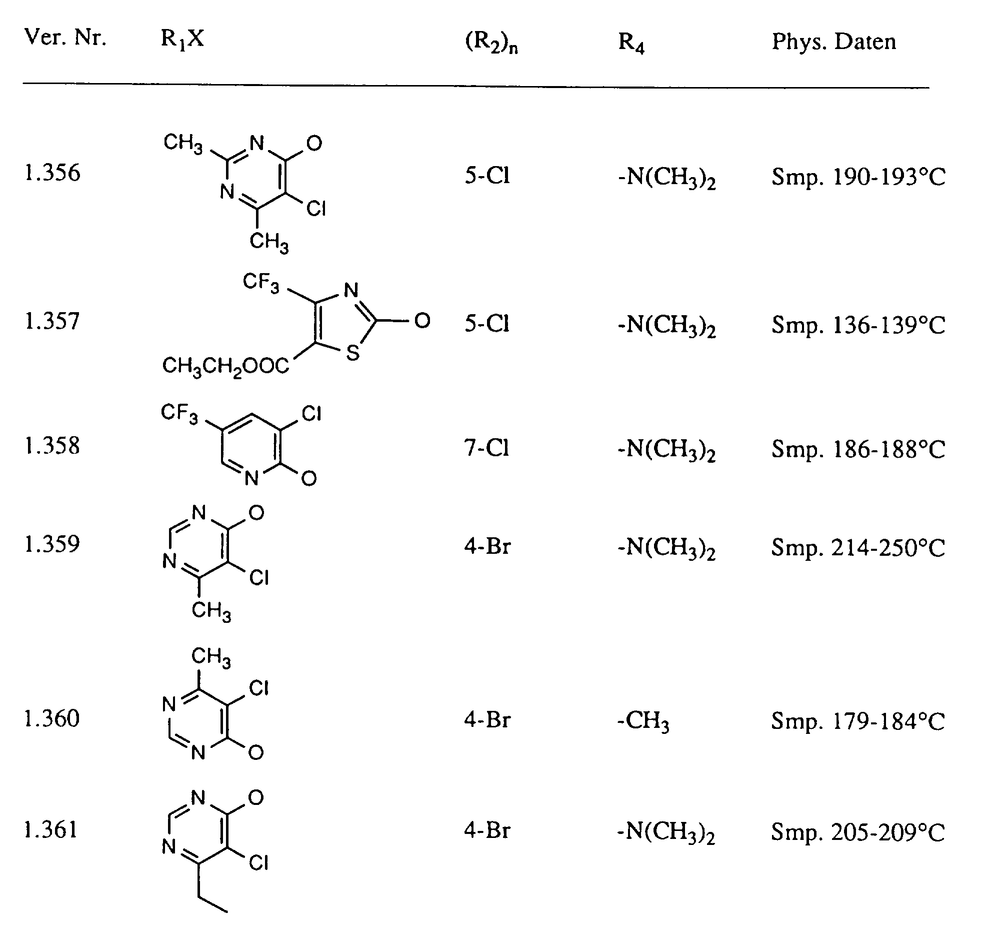

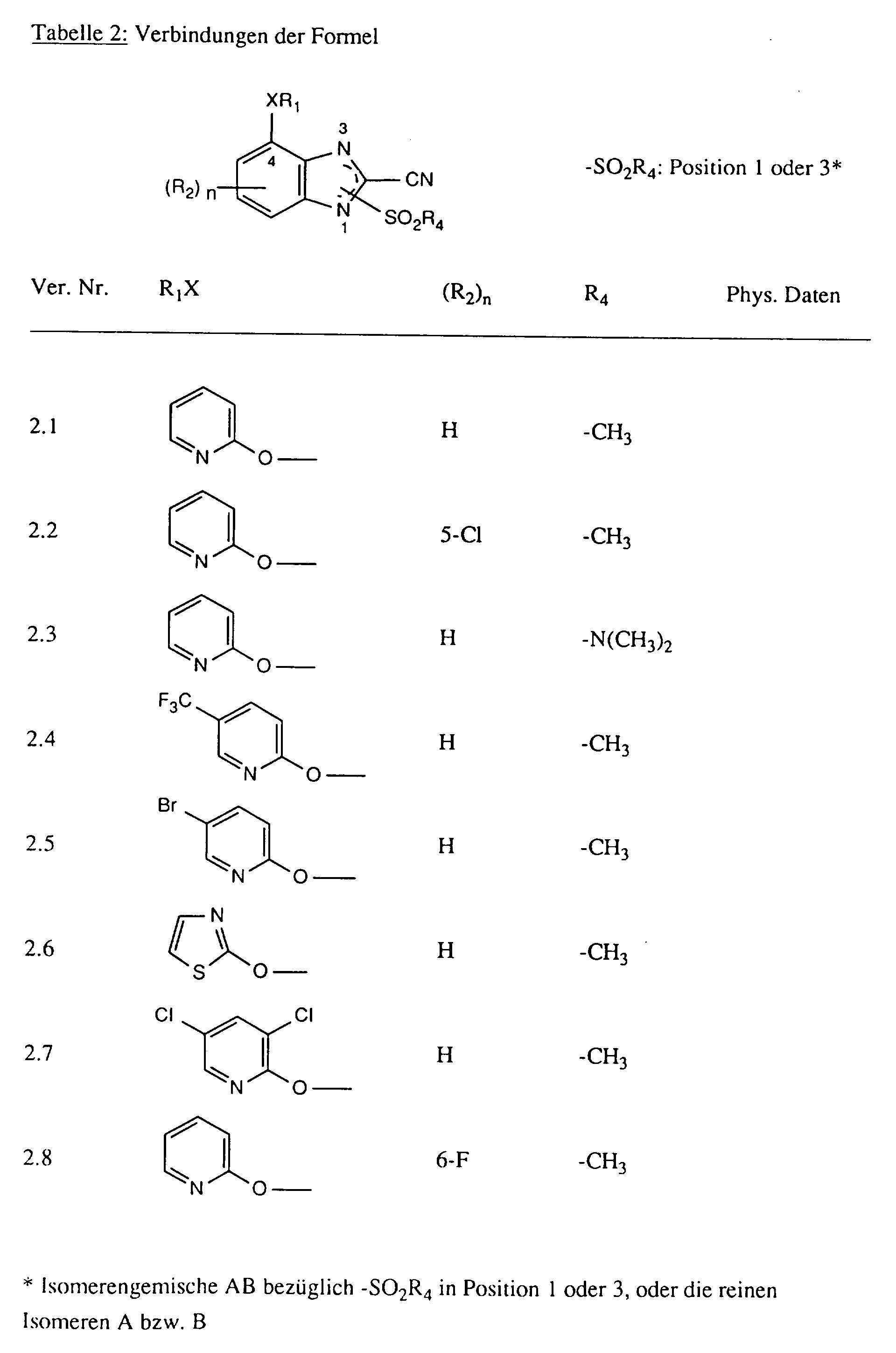

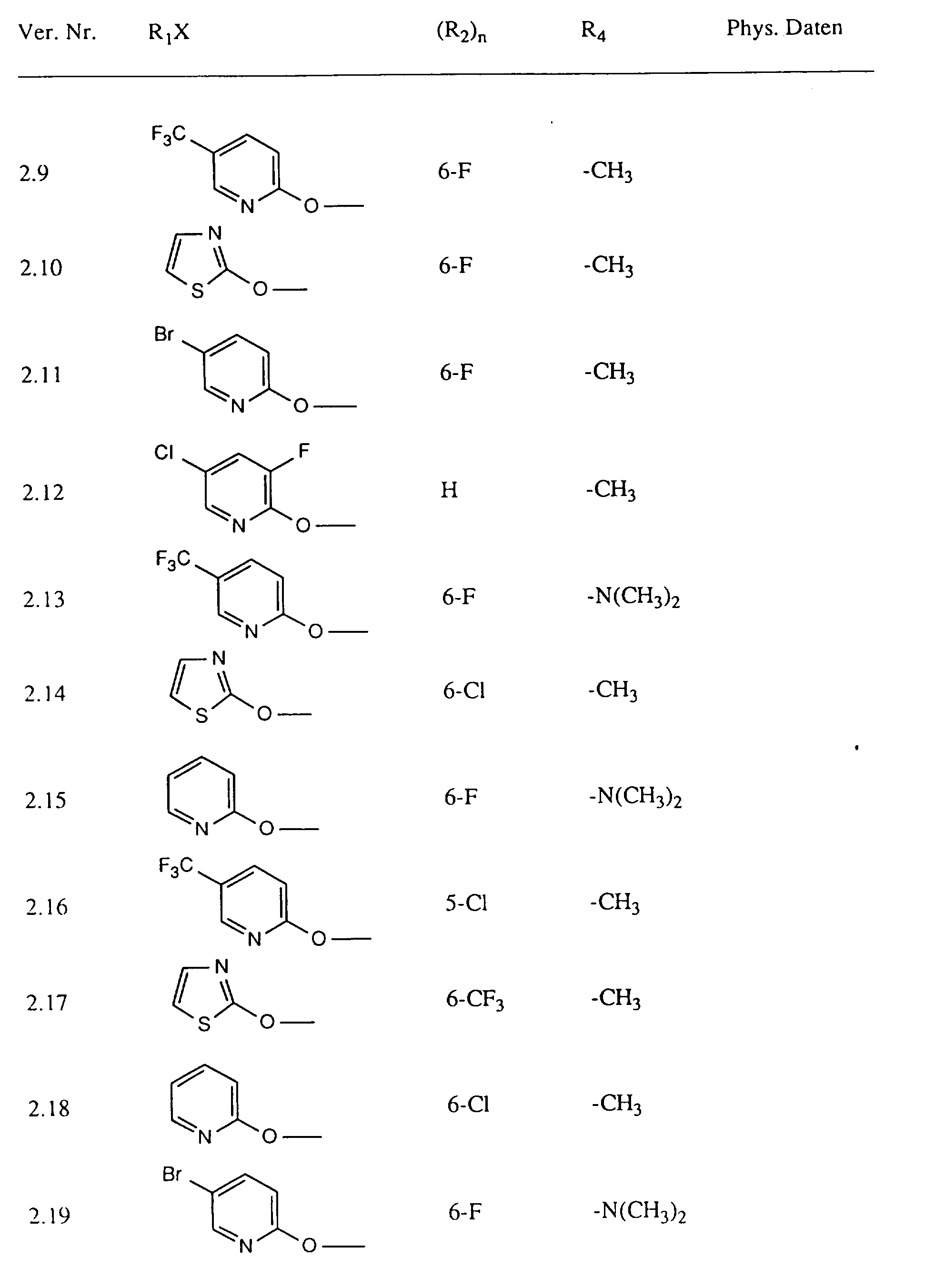

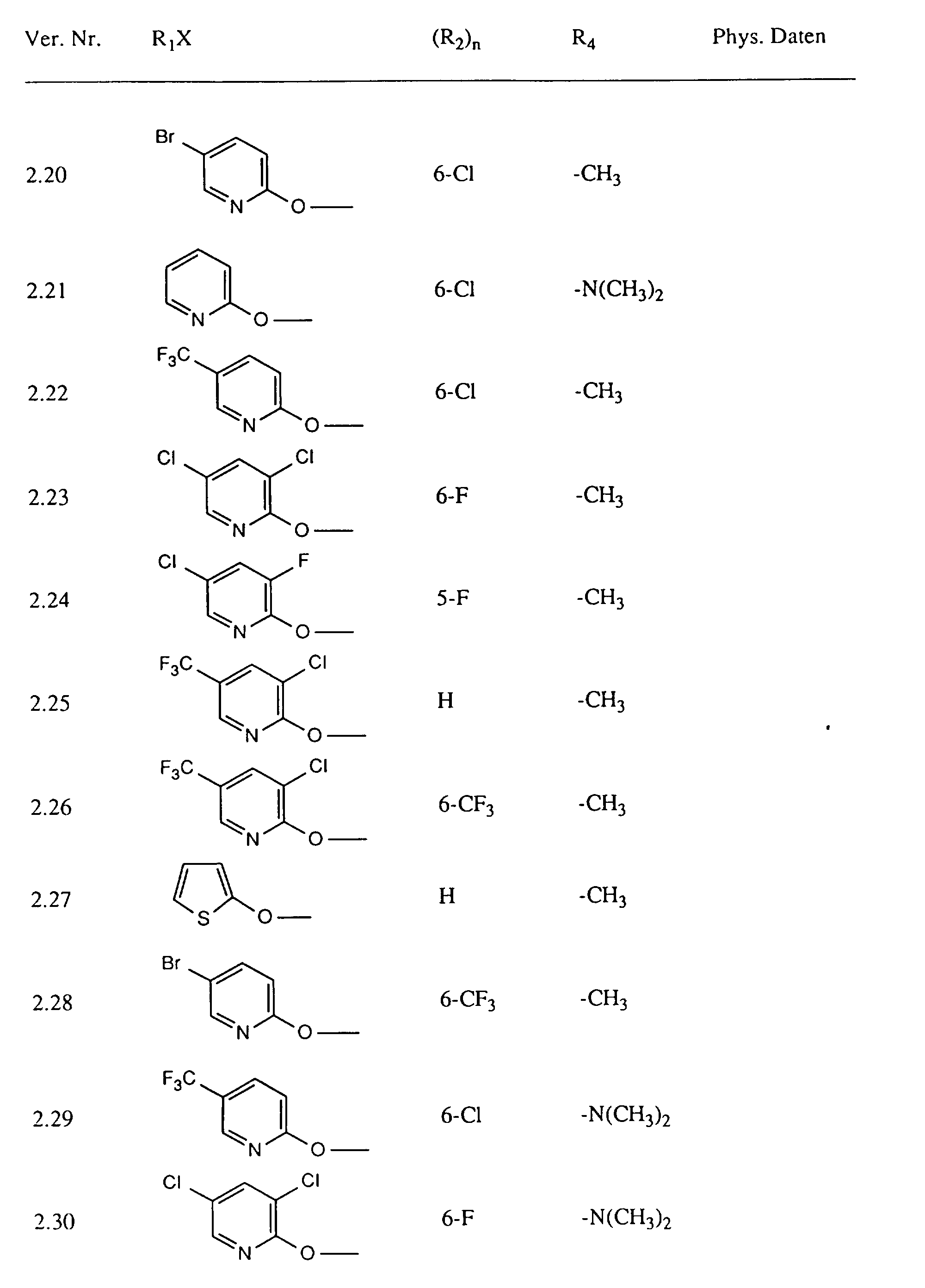

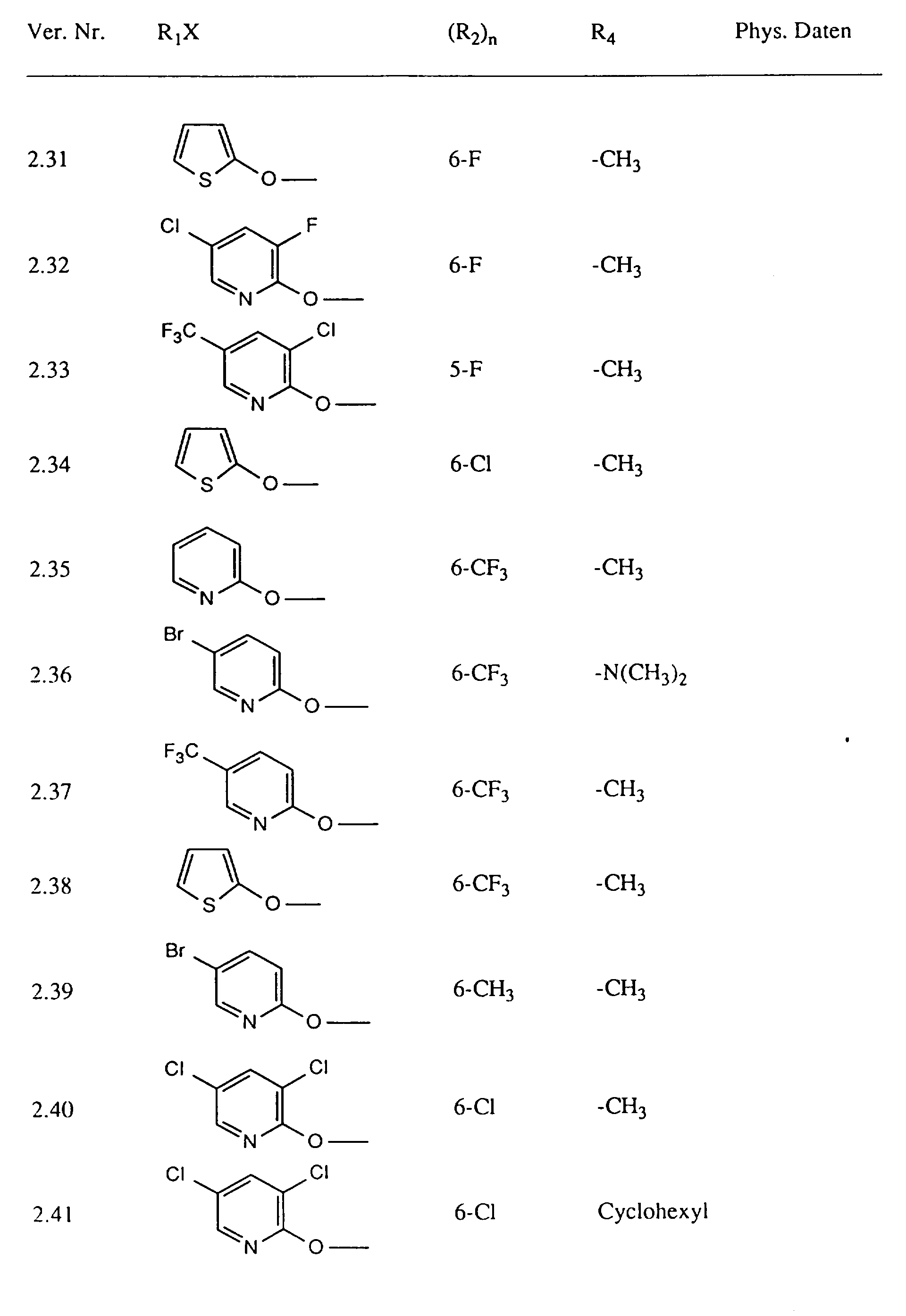

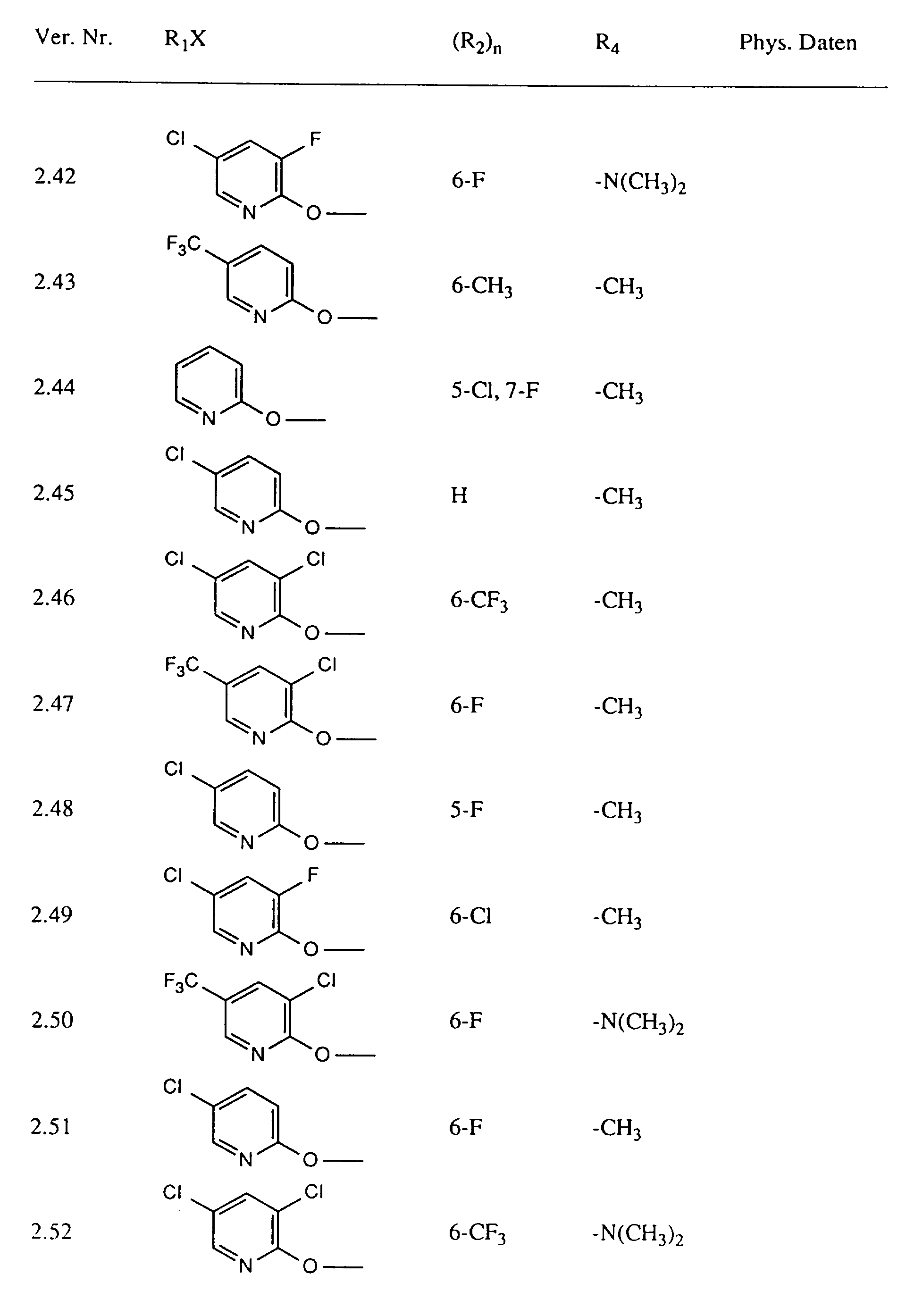

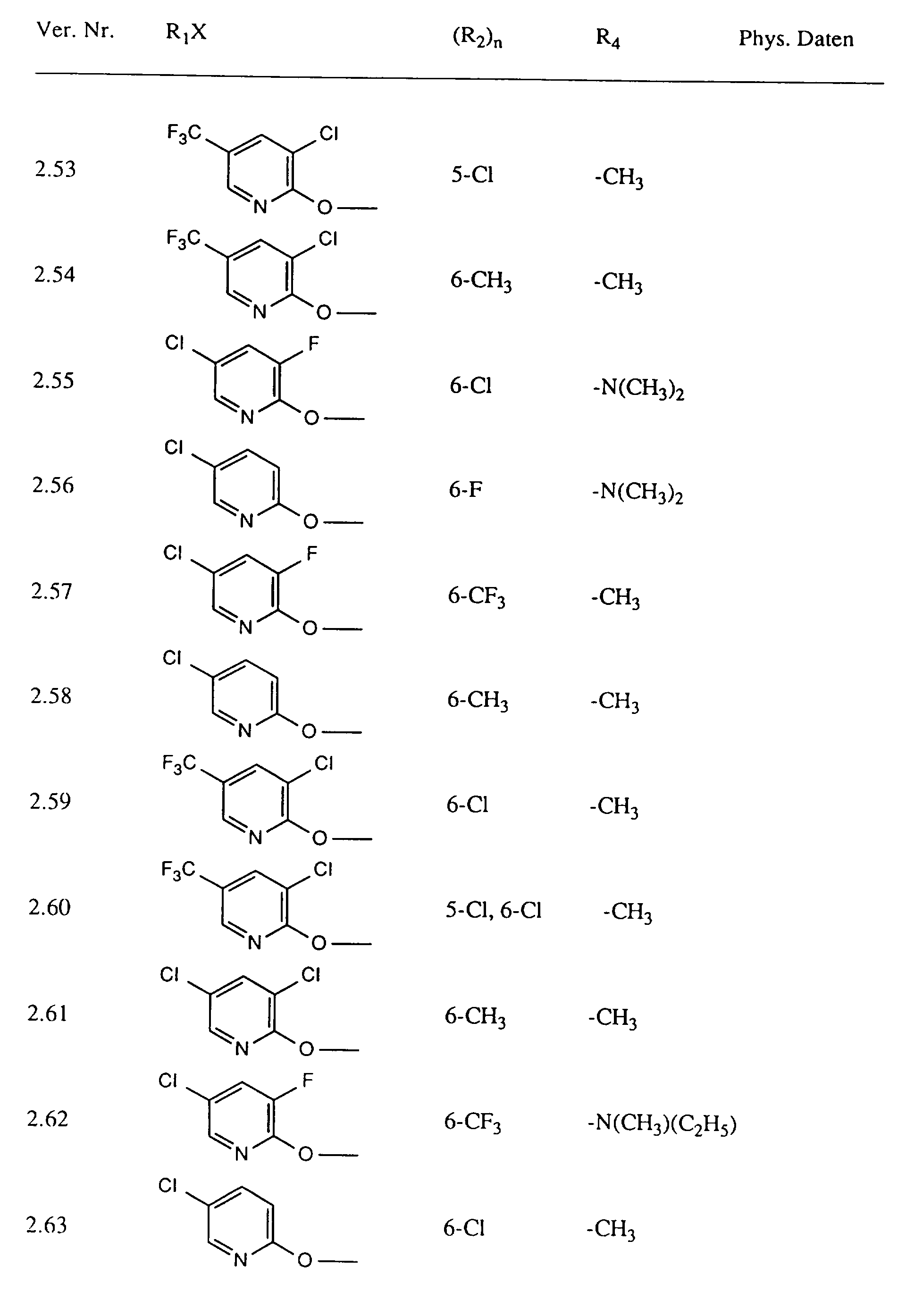

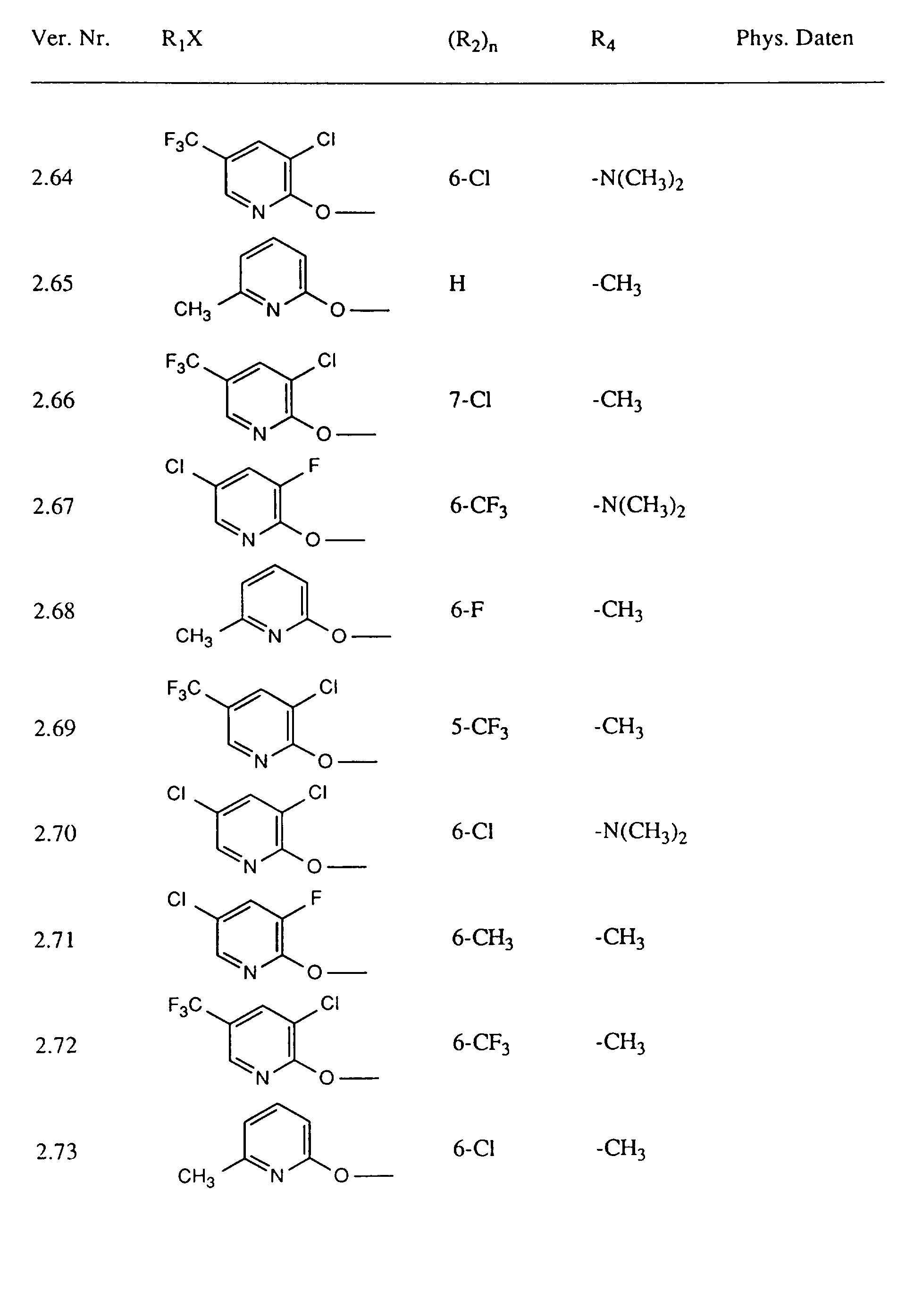

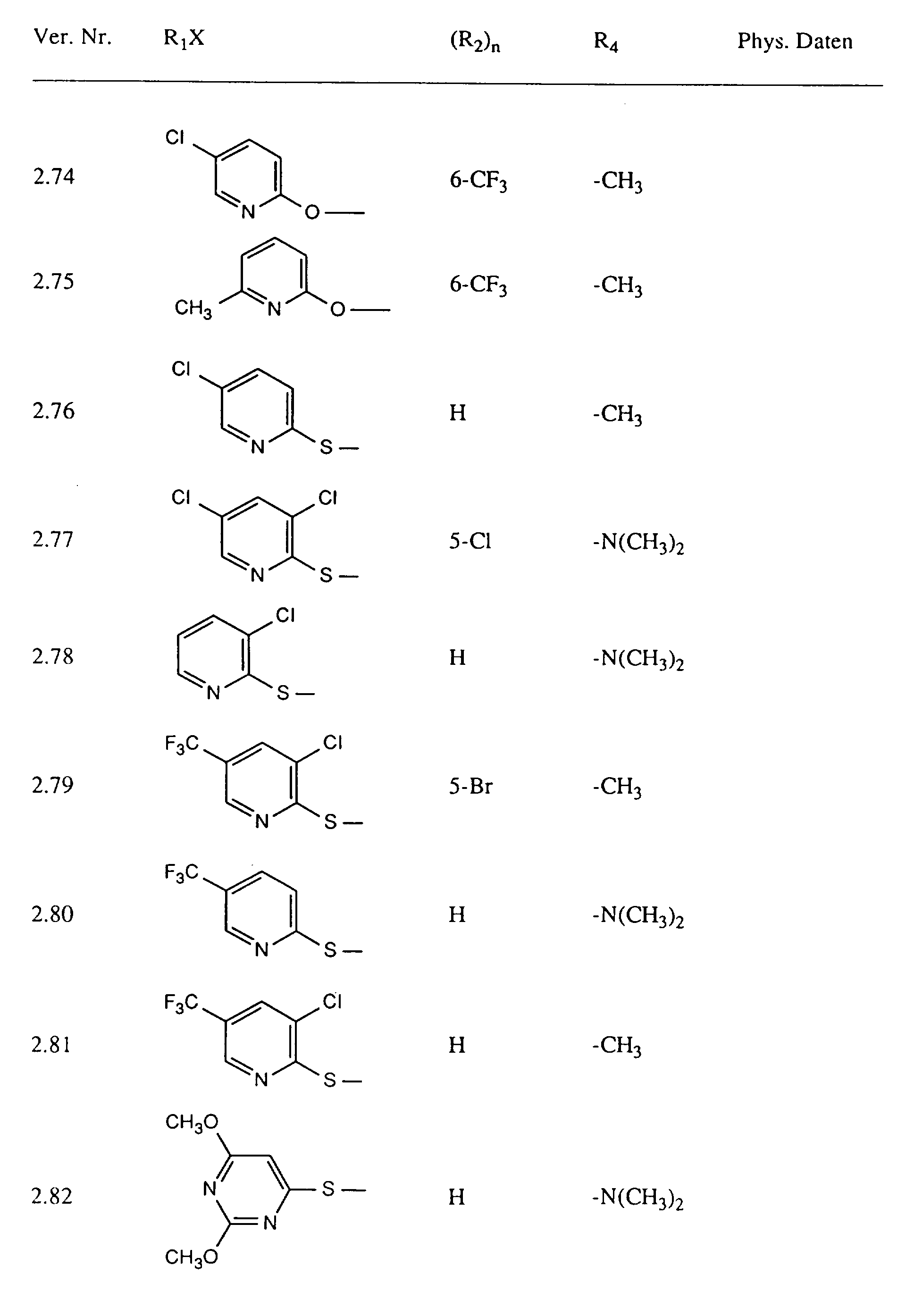

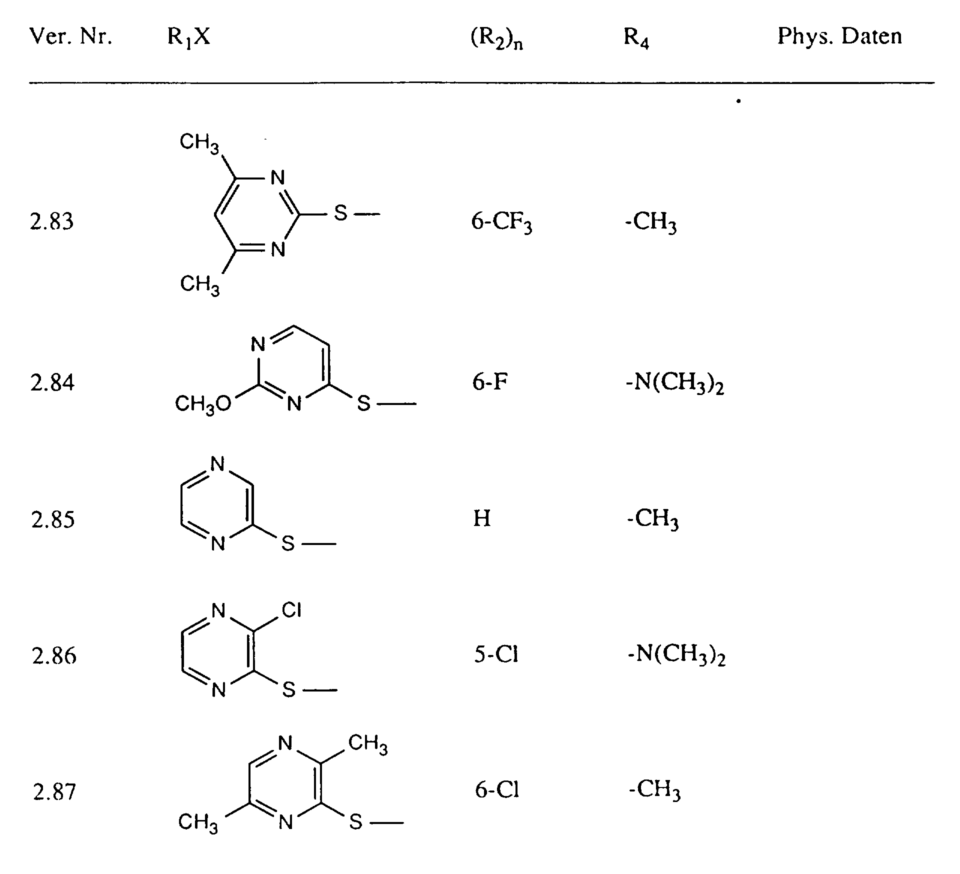

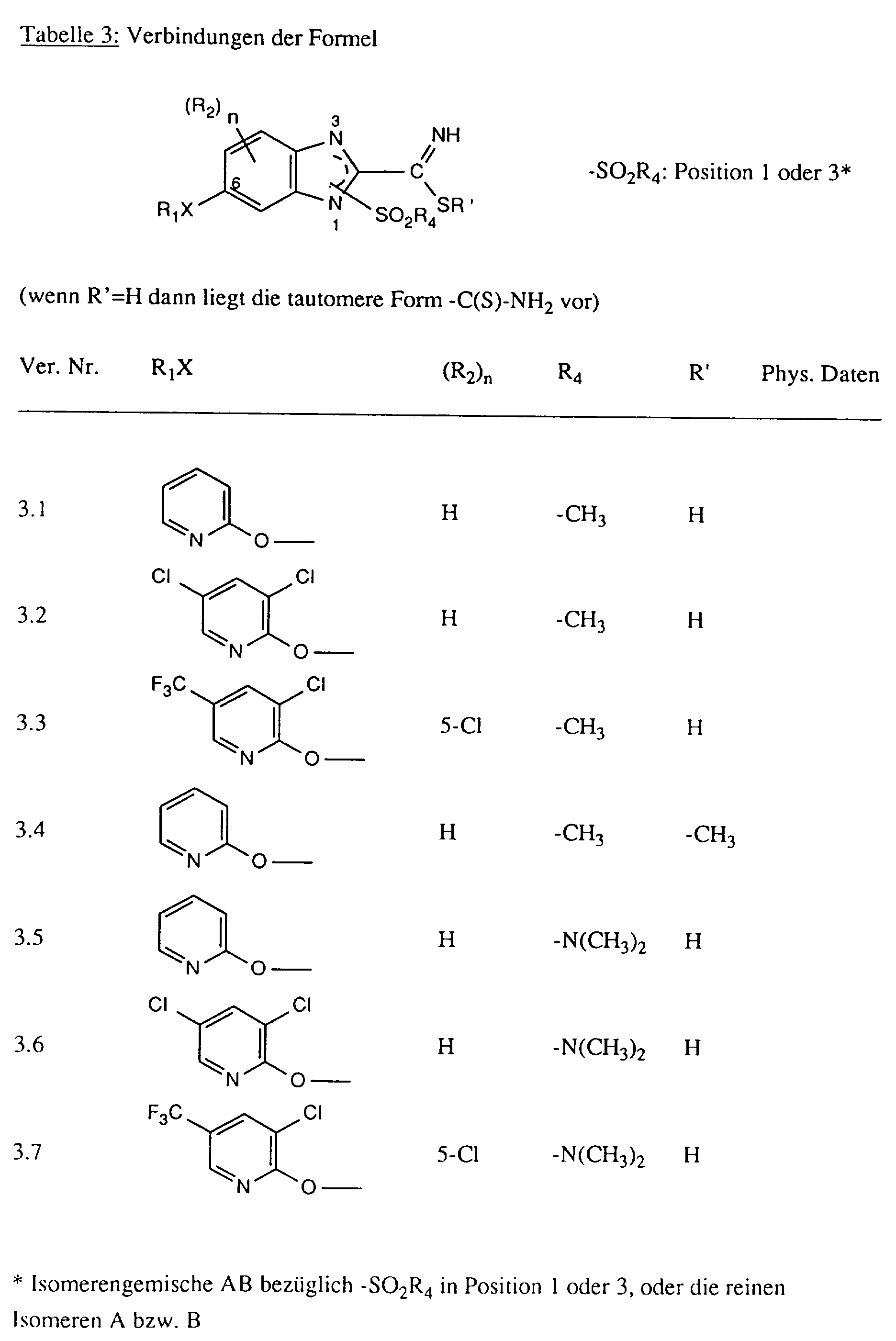

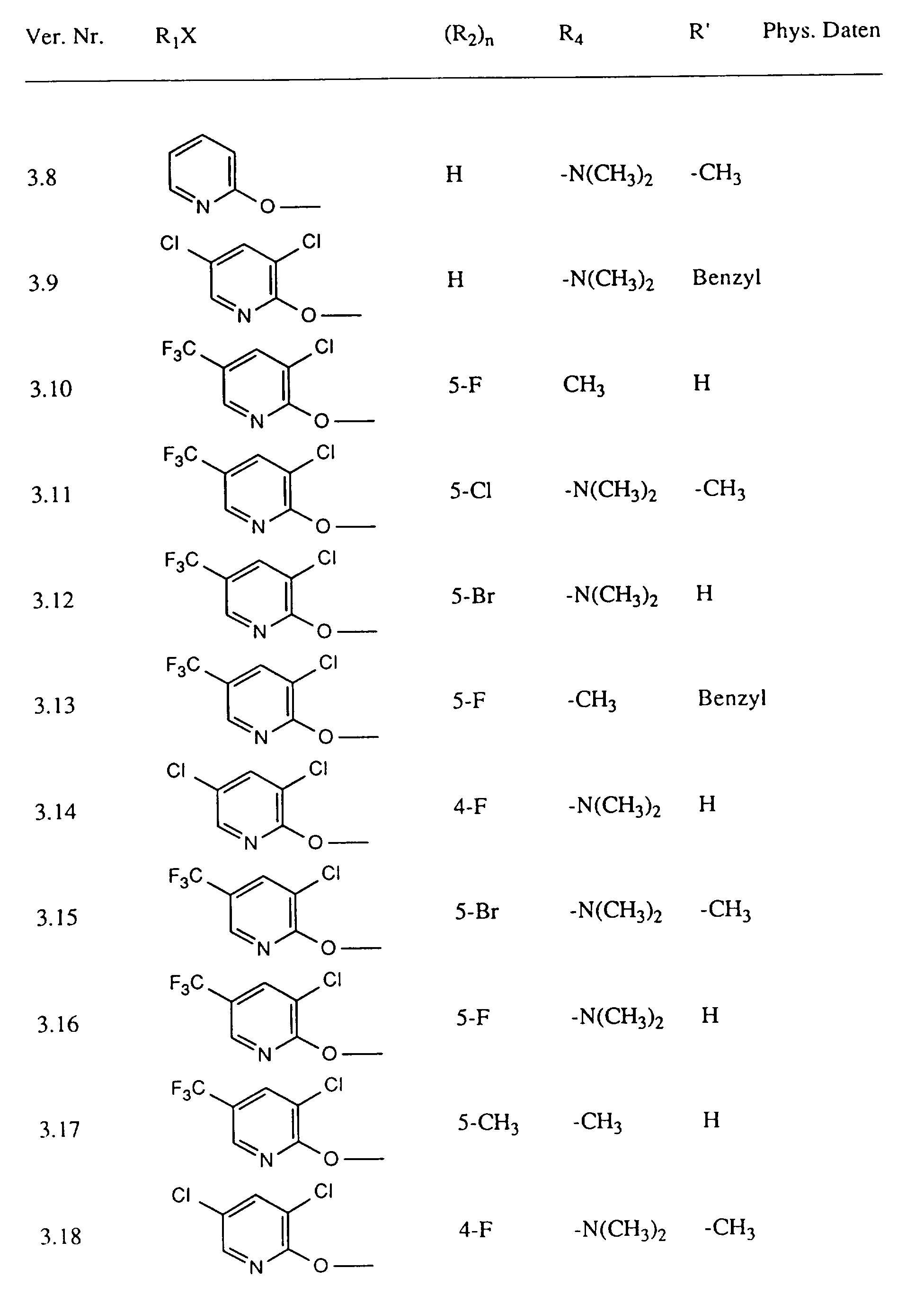

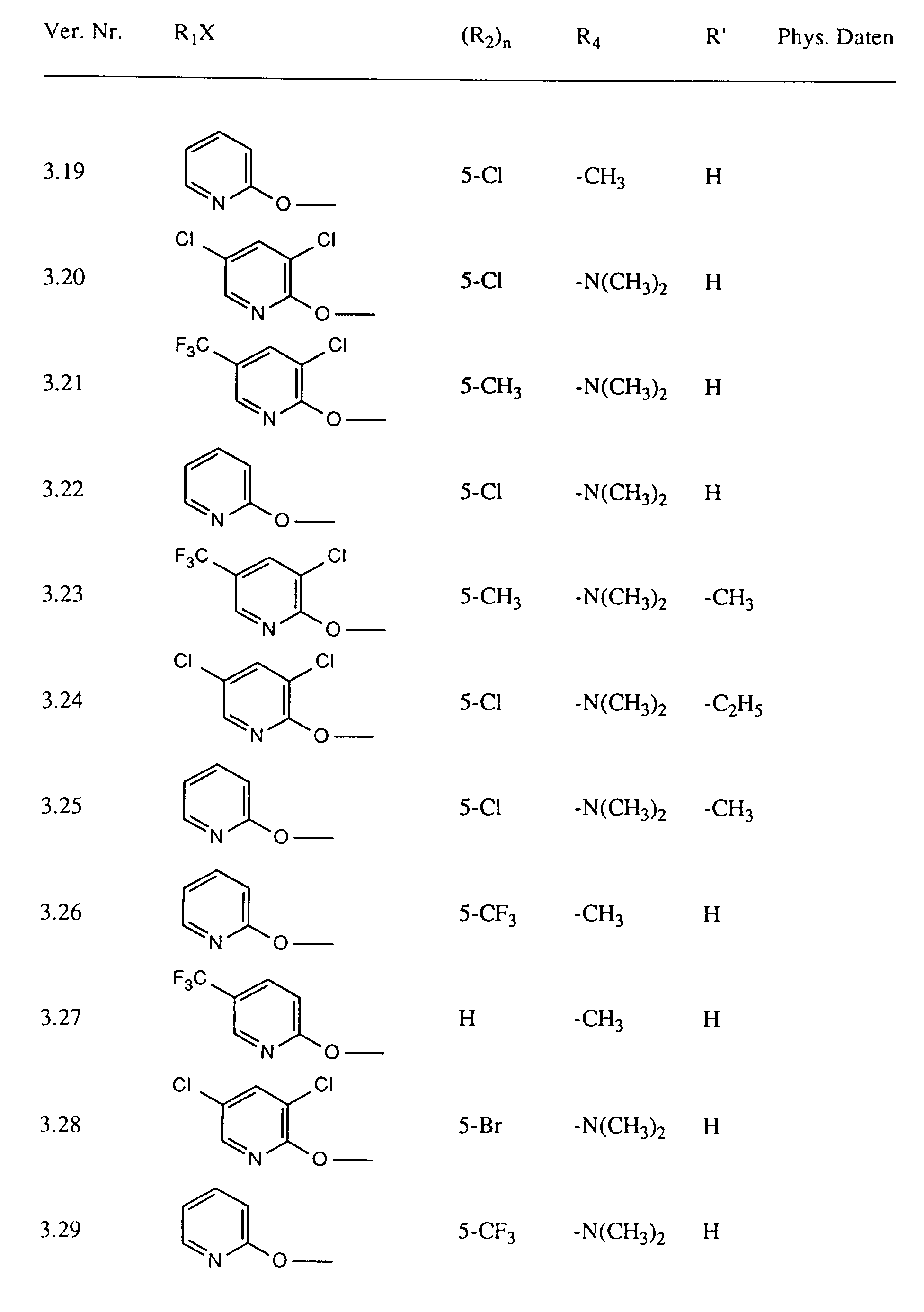

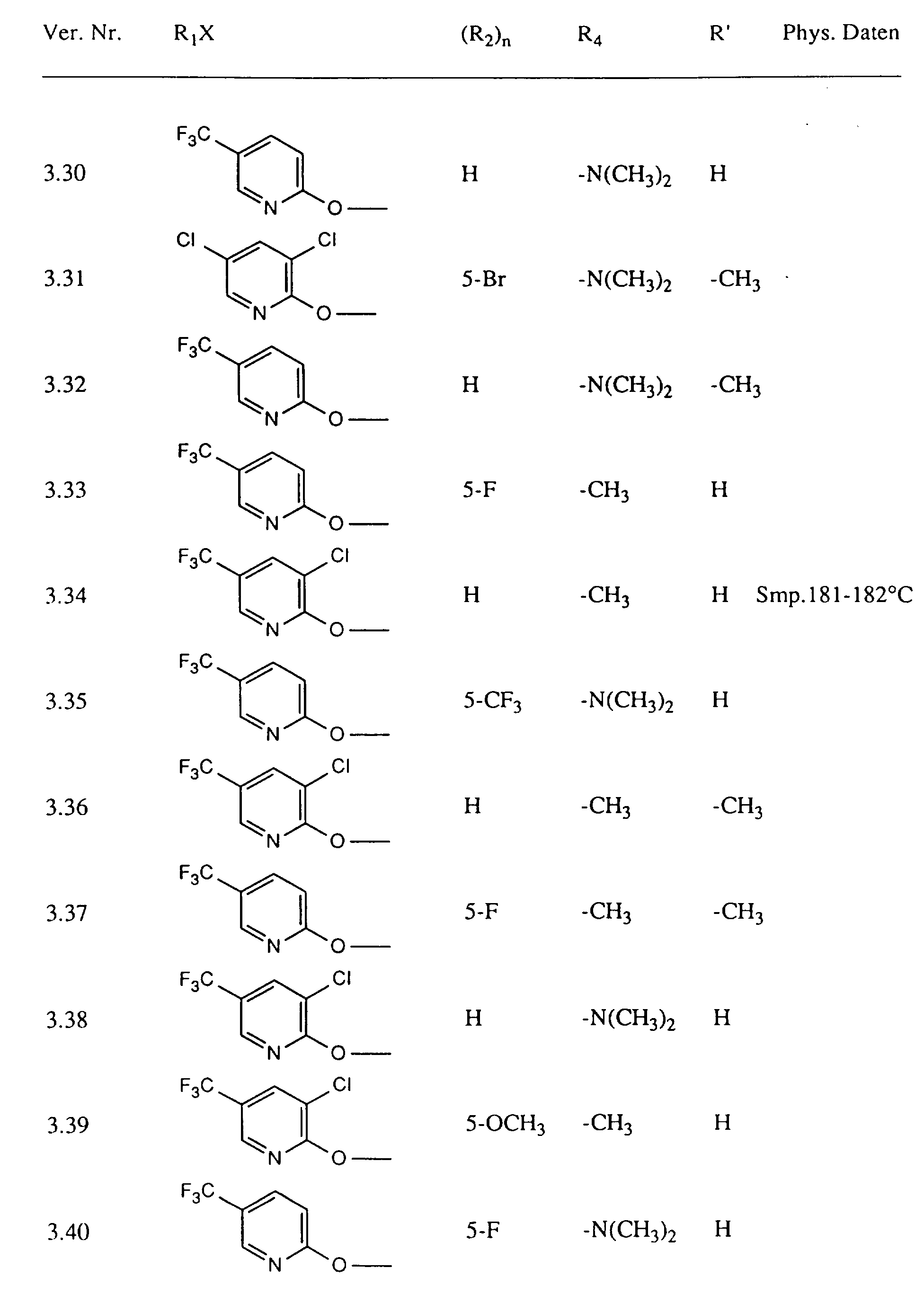

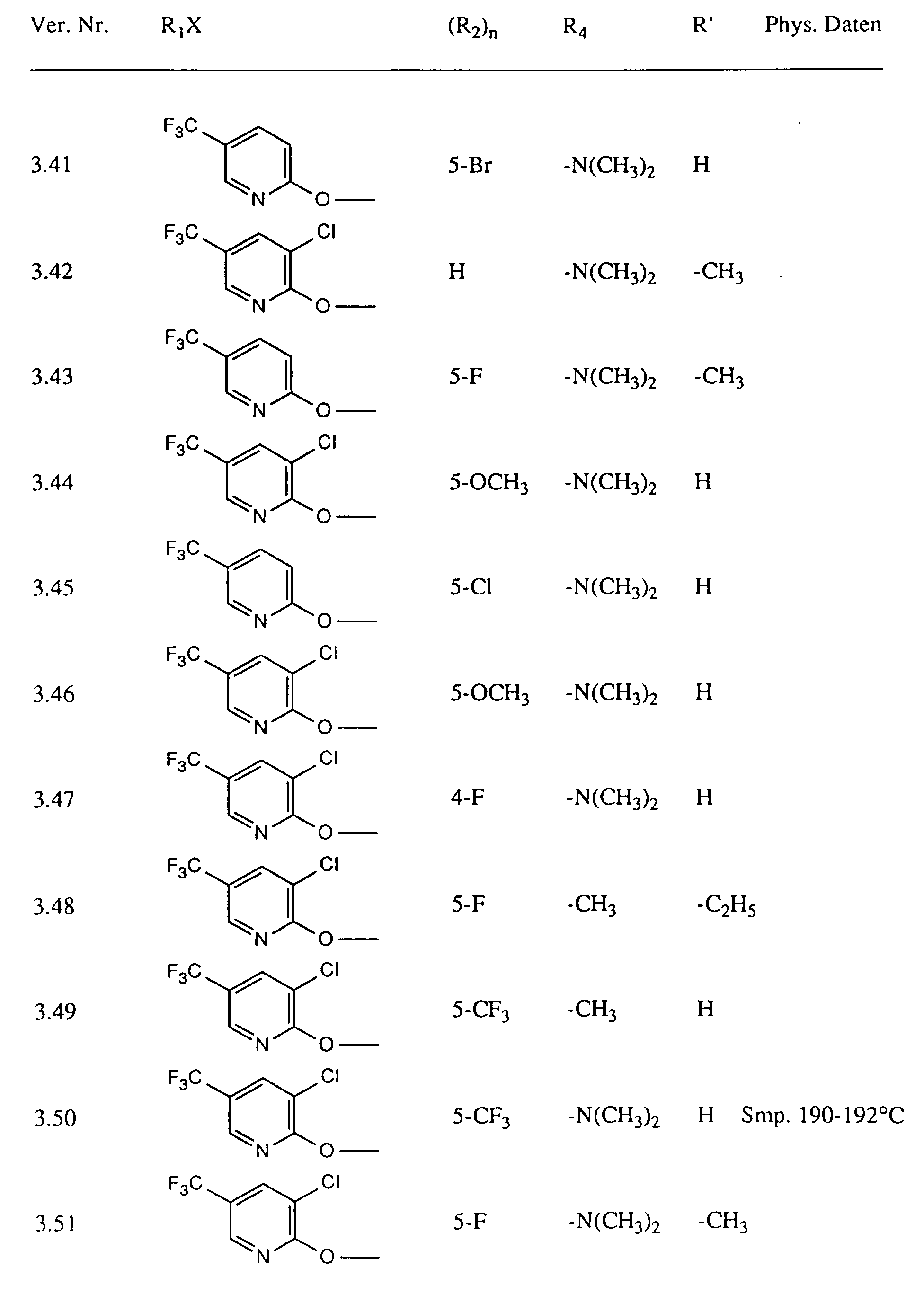

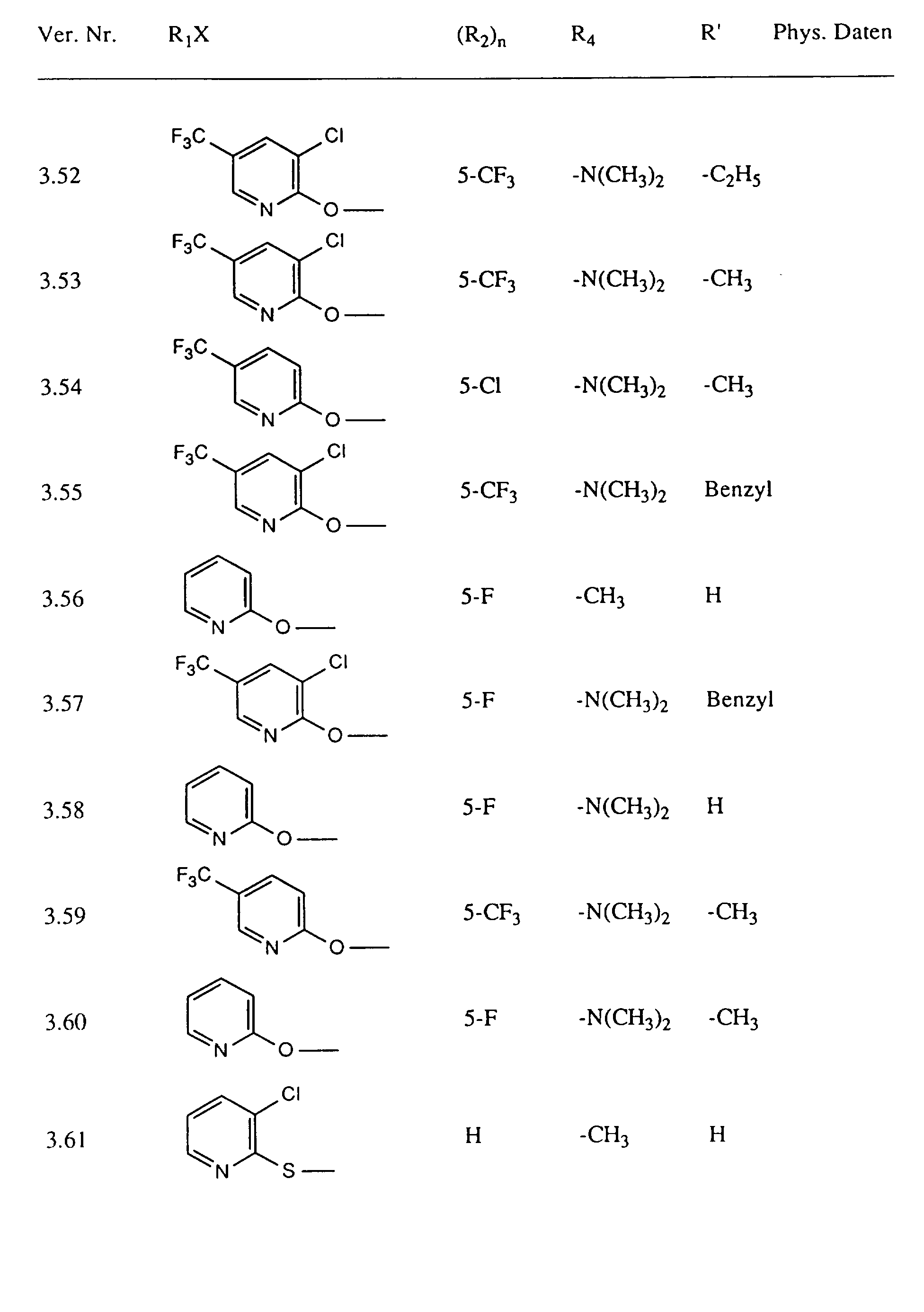

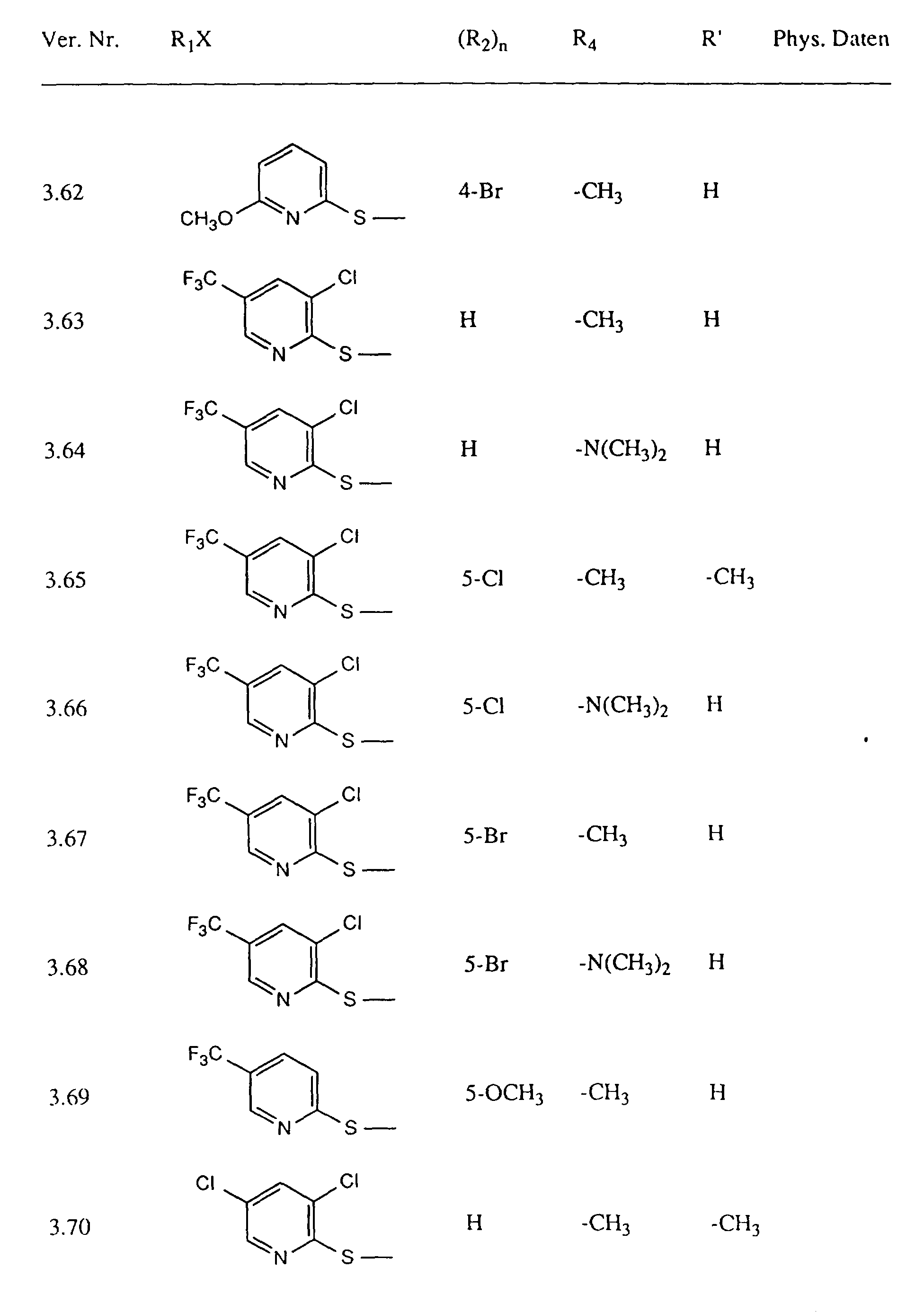

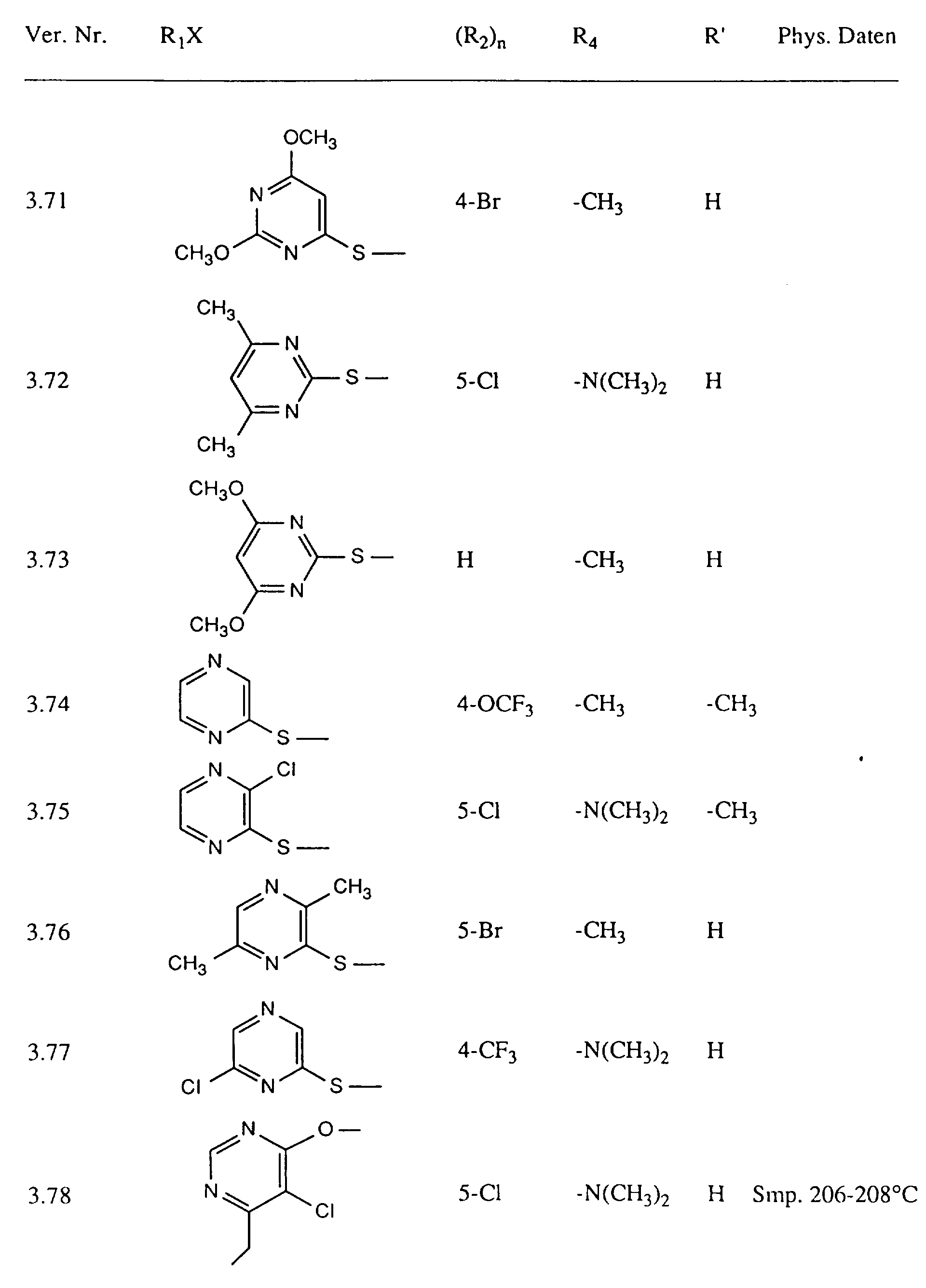

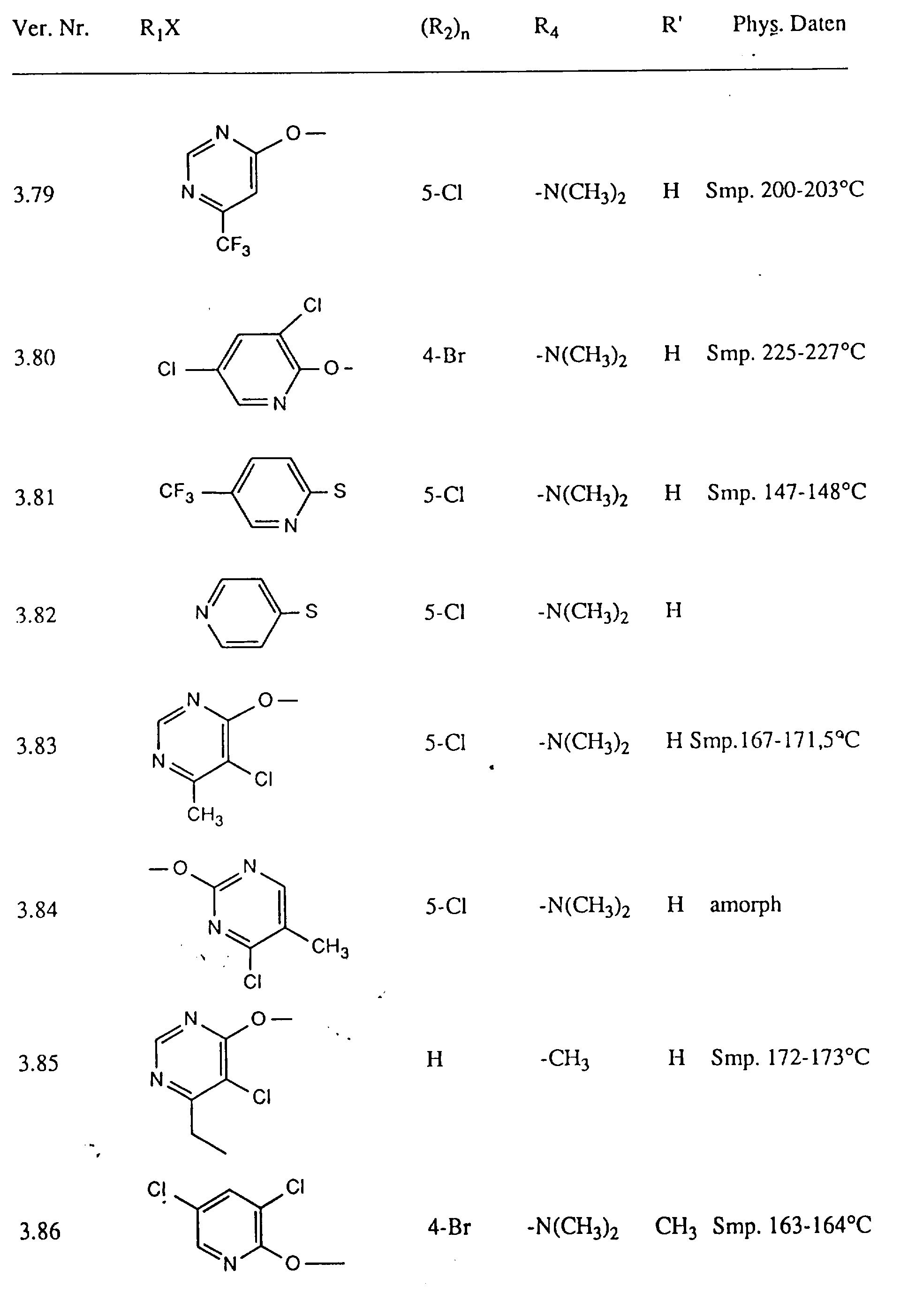

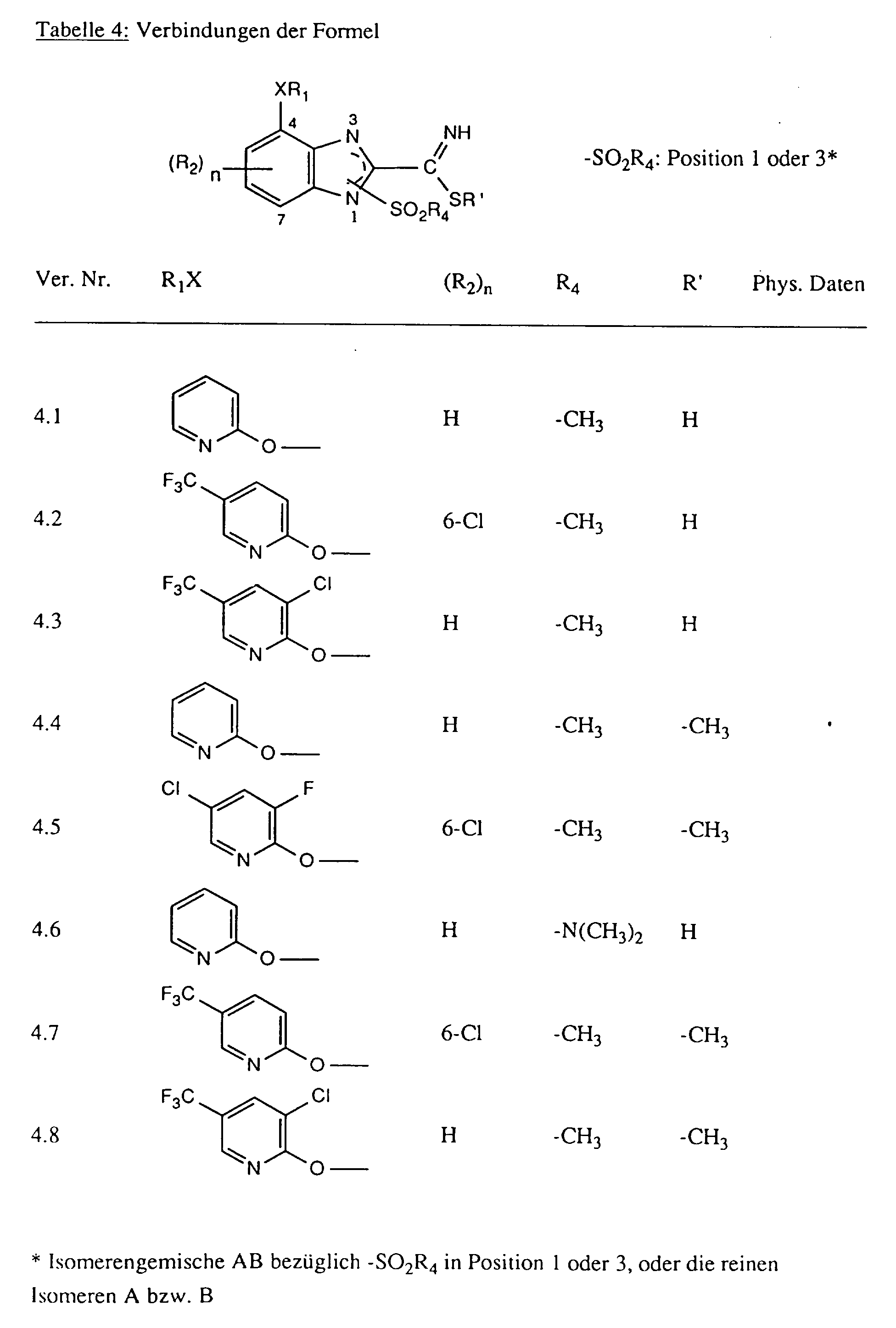

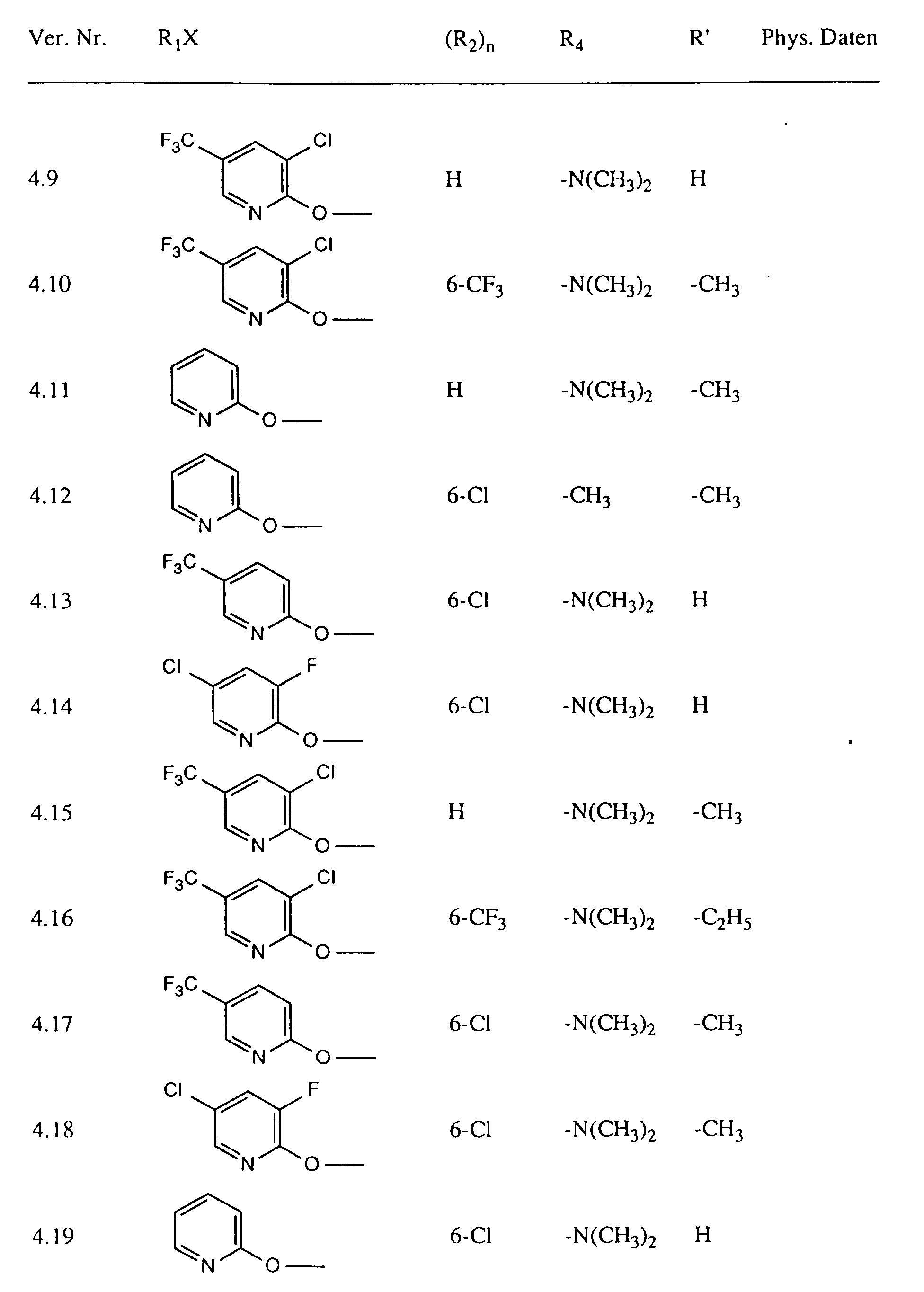

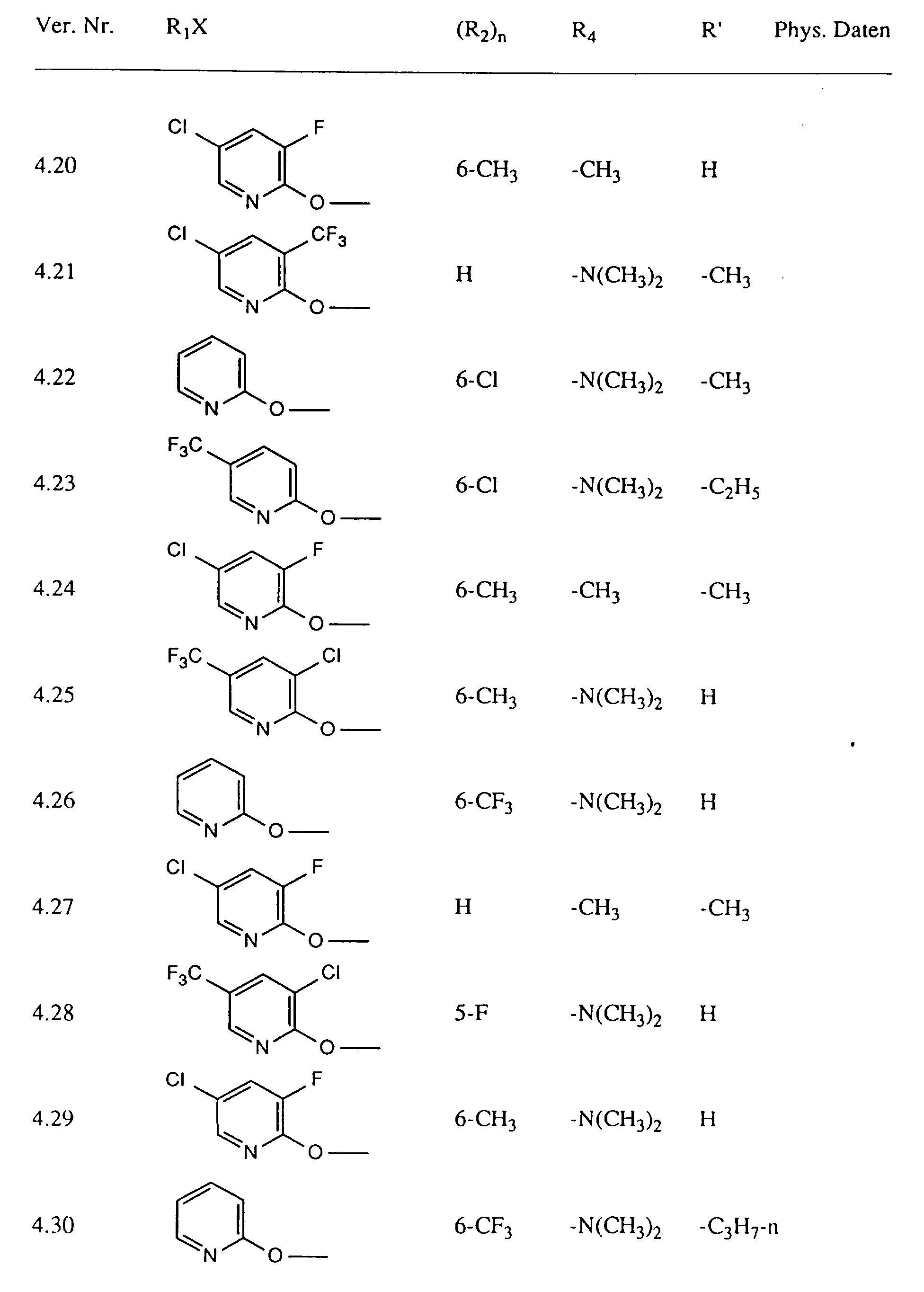

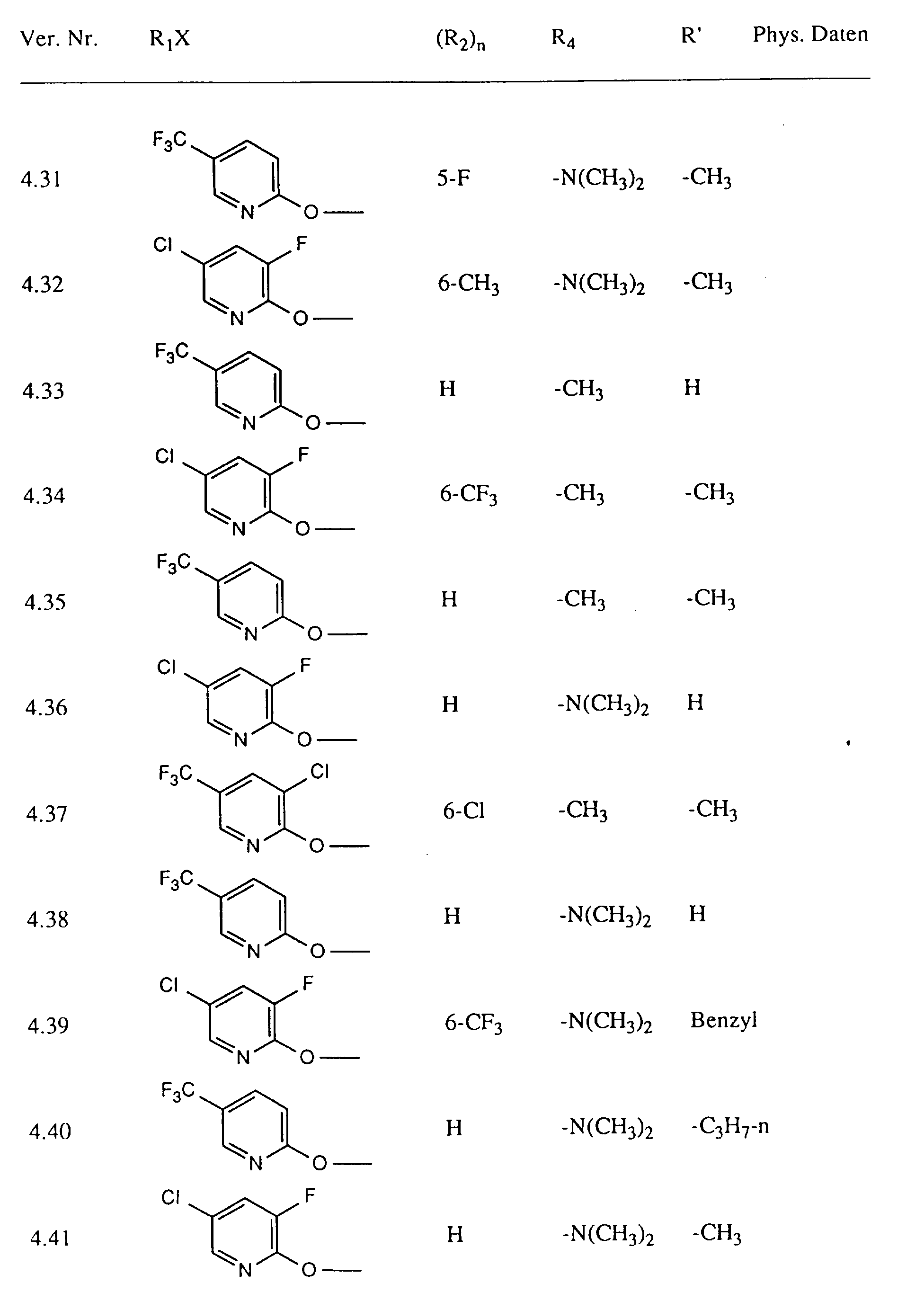

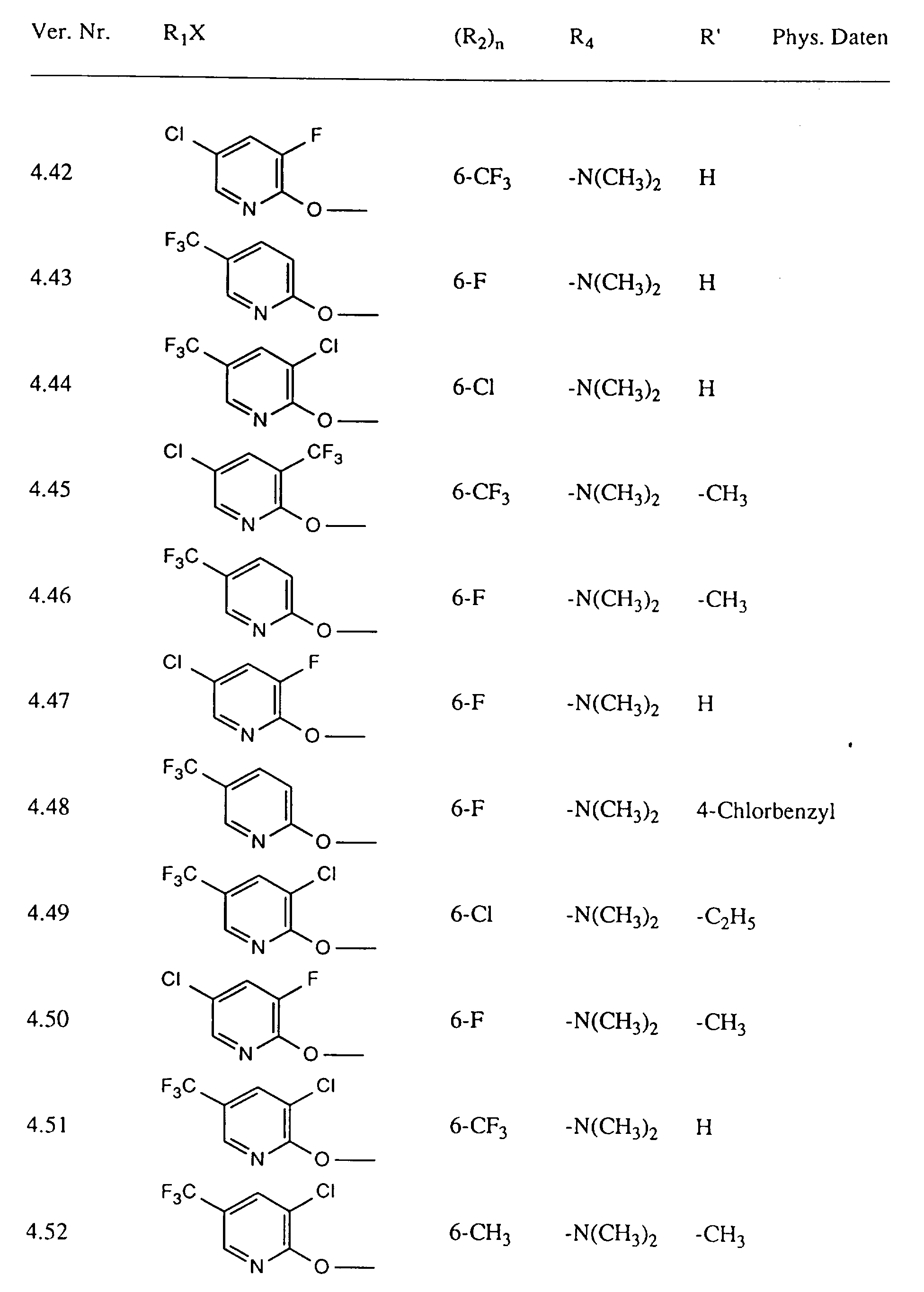

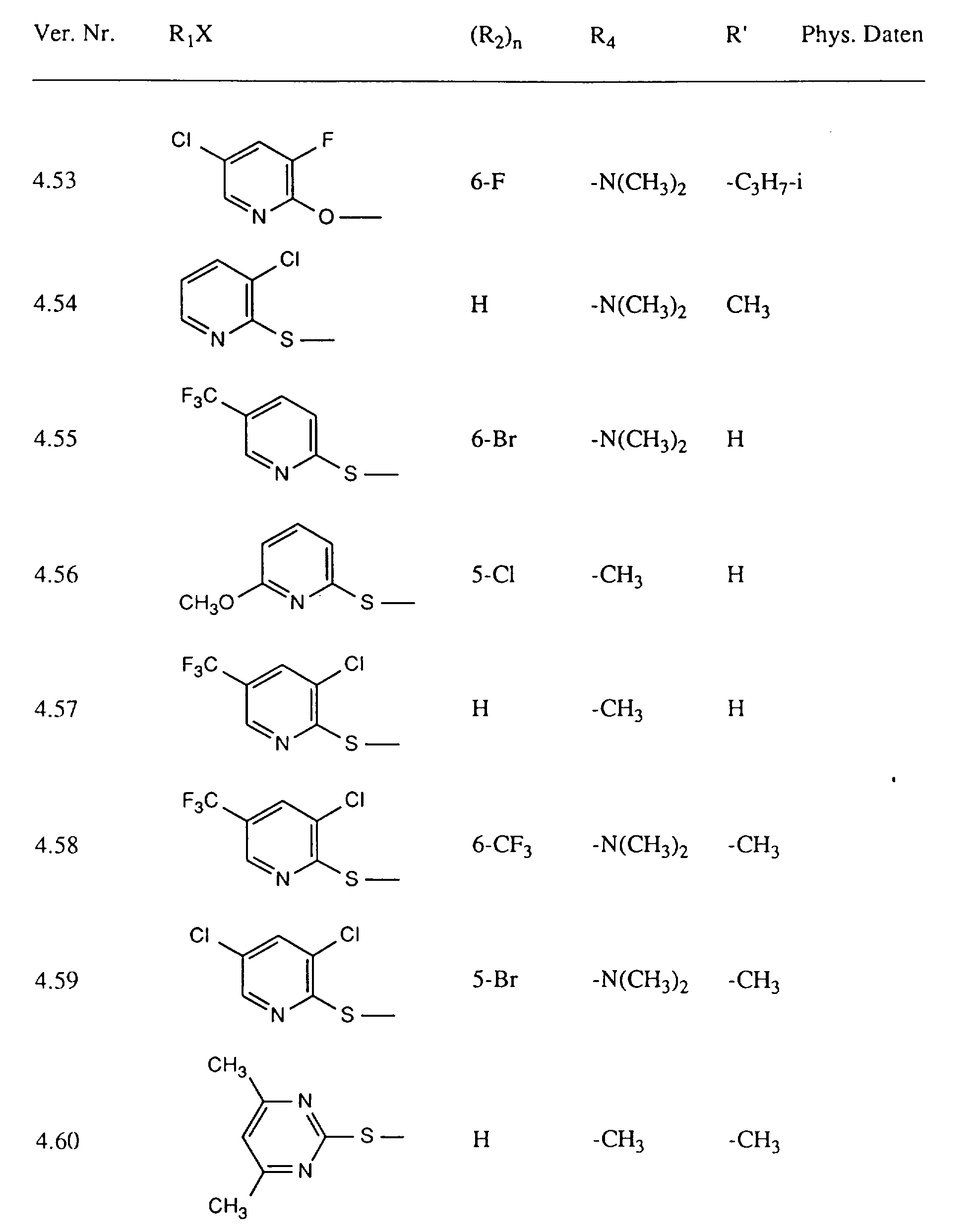

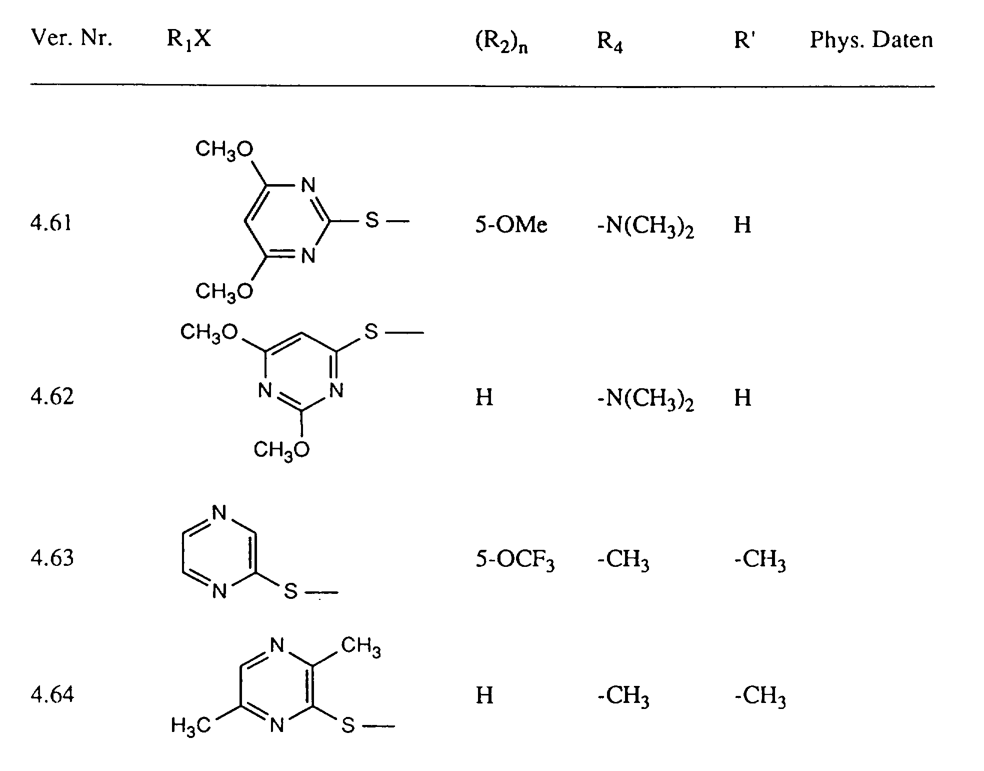

### Formulierungsbeispiele für Wirkstoffe der Formel I (% = Gewichtsprozent)

| 2.1. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2. Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Verdünnung hergestellt werden.

| 2.3. Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.4. Extruder Granulat | |
|---|---|
| Wirkstoff aus den Tabellen | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5. Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6. Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung Silikonöl in Form einer 75%igen | 0,2 % |
| wässrigen Emulsion | 0,8 % |
| Wasser | 32 % |

Der fein gemahlene Wirkstoff wird mit den Zusatzstoffen innig ver- mischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Plasmopara viticola auf Reben

### a) Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

### b) Residual-kurative Wirkung

Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen zeigen gegen Plasmopara viticola auf Reben eine sehr gute fungizide Wirkung, insbesondere die Wirkstoffe Nr. 1.2, 1.24, 1.66, 1.250, 1.276, 1.303, 1.322, 1.346, 3.79 und anderen bewirken eine vollständige Unterdrückung des Pilzbefalls (Restbefall 0 bis 5 %). Unbehandelte aber infizierte Kontrollpflanzen weisen dagegen einen Plasmopara-Befall von 100 % auf.

### Beispiel 3.2: Wirkung gegen Phytophthora auf Tomatenpflanzen

### Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.2, 1.12, 1.23, 1.24, 1.26, 1.54, 1.66, 1.146, 1.177, 1.199, 1.221, 1.230, 1.275, 1.308, 3.50, 3.79 und anderen wird ein Befall praktisch vollständig verhindert (0 bis 5 % Befall). Unbehandelte aber infizierte Kontrollpflanzen weisen dagegen einen Phytophthora-Befall von 100 % auf.

### Beispiel 3.3: Wirkung gegen Phytophthora auf Kartoffelpflanzen

### Residual-protektive Wirkung

2-3 Wochen alte Kartoffelpflanzen (Sorte Bintje) werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.23, 1.24, 1.66, 1.146 und anderen wird ein Befall praktisch vollständig verhindert (0 bis 5 % Befall). Unbehandelte aber infizierte Kontrollpflanzen weisen dagegen einen Phytophthora-Befall von 100 % auf.

### Beispiel 3.4: Wirkung gegen Pythium debaryanum auf Zuckerrüben (Beta vulgaris)

### a) Wirkung nach Bodenapplikation

Der Pilz wird auf sterilen Haferkörnern kultiviert und in einer Erde-Sand-Mischung beigegeben. Die so infizierte Erde wird in Blumentöpfe abgefüllt und mit Zuckerrübensamen besät. Gleich nach der Aussaat werden die als Spritzpulver formulierten Versuchspräparate als wässerige Suspension über die Erde gegossen /20 ppm Wirkstoff bezogen auf das Erdvolumen). Die Töpfe werden darauf während 2-3 Wochen im Gewächshaus bei 20-24°C aufgestellt. Die Erde wird ständig durch leichtes Besprühen mit Wasser gleichmässig feucht gehalten. Bei der Auswertung der Tests wird der Auflauf der Zuckerrübenpflanzen sowie der Anteil gesunder und kranker Pflanzen bestimmt.

### b) Wirkung nach Beizapplikation

Der Pilz wird auf sterilen Haferkörnern kultiviert und in einer Erde-Sand-Mischung beigegeben. Die so infizierte Erde wird in Blumentöpfe abgefüllt und mit Zuckerrübensamen besät, die mit den als Beizpulver formulierten Versuchspräparaten gebeizt worden waren (1000 ppm Wirkstoff bezogen auf das Samengewicht).Die besäten Töpfe werden darauf während 2-3 Wochen im Gewächshaus bei 20-24°C aufgestellt. Die Erde wird ständig durch leichtes Besprühen mit Wasser gleichmässig feucht gehalten. Bei der Auswertung der Tests wird der Auflauf der Zuckerrübenpflanzen sowie der Anteil gesunder und kranker Pflanzen bestimmt.

Nach der Behandlung mit Wirkstoffen der Formel I gemäss den Tabellen 1 und 3 laufen über 80% der Pflanzen auf und haben ein gesundes Aussehen. In den Kontrolltöpfen werden nur vereinzelt aufgelaufene Pflanzen mit kränklichem Aussehen beobachtet.

### Beispiel 3.5 : Direkte Wirkung gegen Peronospora tabacina

Formulierter Wirkstoff wird in verschiedenen Konzentrationen (10, 1,0,1 ppm) mit Wasseragar gemischt und in Petrischalen gegossen. Nach dem Abkühlen werden 100 µl einer Sporangiensuspension (10⁶ Sporen/ml) auf die Platte gestrichen. Die Platten werden 16 Stunden bei 18°C inkubiert.
Bei Verbindungen der Tabellen 1 und 3 wurde keine Hemmung der Keimung von Peronospora tabacina beobachtet.

## Patentansprüche

1. Benzimidazolsulfonsäure-Derivate der Formel I 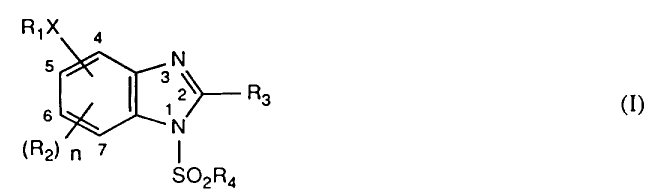 worin die R₄SO₂-Gruppe die 1- oder 3-Position besetzt und im Verhältnis zu den Substituenten R₁X- und R₂ reine oder gemischte Stellungsisomere bildet, und worin die Substituenten folgende Bedeutung haben:
R₁ = ungesättigter 5-gliedriger Heterocyclus mit maximal zwei Heteroatomen N und/oder S, oder ein ungesättigter 6-gliedriger Heterocyclus mit maximal zwei N-Atomen, wobei jeder der Heterocyclen unsubstituiert oder durch mindestens einen der Substituenten Halogen, C₁-C₃-Alkyl, C₁-C₃-Alkoxy, C₁-C₃-Haloalkyl, C₁-C₃-Haloalkoxy, COOC₁-C₃-Alkyl, C₃-C₆-Cycloalkyl, CN und Nitro substituiert sein kann;
R₂ = gleich oder verschieden Halogen, C₁-C₃-Alkyl, C₁-C₃-Alkoxy, C₁-C₃-Haloalkyl, C₁-C₃-Haloalkoxy, Nitro;
R₃ = Cyano, -CS-NH₂ oder -C(SR')=NH, wobei R'C₁-C₄-Alkyl, C₁-C₄-Haloalkyl, unsubstituiertes oder durch Halogen und/oder CF₃ substituiertes Benzyl darstellt;
R₄ = C₁-C₄-Alkyl, C₃-C₆-Cycloalkyl, -N(R'')(R'''), wobei R'' und R''' gleiches oder verschiedenes C₁-C₃-Alkyl darstellt;
X = Sauerstoff oder Schwefel; und
n = eine ganze Zahl 0, 1 oder 2.

2. Benzimidazolsulfonsäure-Derivate der Formel I, gemäss Anspruch 1,worin die R₄SO₂-Gruppe die 1- oder 3-Position besetzt und im Verhältnis zu den Substituenten R₁X- und R₂ reine oder gemischte Stellungsisomere bildet, und worin die Substituenten folgende Bedeutung haben:
R₁ = ungesättigter 5-gliedriger Heterocyclus mit maximal zwei Heteroatomen N und/oder S, oder ein ungesättigter 6-gliedriger Heterocyclus mit maximal zwei N-Atomen, wobei jeder der Heterocyclen unsubstituiert oder durch mindestens einen der Substituenten Halogen, C₁-C₃-Alkyl, C₁-C₃-Alkoxy, C₁-C₃-Haloalkyl, C₁-C₃-Haloalkoxy und Nitro substituiert sein kann;
R₂ = gleich oder verschieden Halogen, C₁-C₃-Alkyl, C₁-C₃-Alkoxy, C₁-C₃-Haloalkyl, C₁-C₃-Haloalkoxy, Nitro;
R₃ = Cyano, -CS-NH₂ oder -C(SR')=NH, wobei R'C₁-C₄-Alkyl, C₁-C₄-Haloalkyl, unsubstituiertes oder durch Halogen und/oder CF₃ substituiertes Benzyl darstellt;
R₄ = C₁-C₄-Alkyl, C₃-C₆-Cycloalkyl, -N(R'')(R'''), wobei R'' und R''' gleiches oder verschiedenes C₁-C₃-Alkyl darstellt;
X = Sauerstoff oder Schwefel; und
n = eine ganze Zahl 0, 1 oder 2.

3. Verbindungen gemäss Anspruch 2, worin R₁ einen unsubstituierten oder substituierten Pyridin-, Pyrimidin-, Pyrazin- oder Pyridazinring bedeutet.

4. Verbindungen gemäss Anspruch 3, worin der 6-gliedrige Heterocyclus durch ein bis drei Substituenten ausgewählt aus Halogen, Methyl, Ethyl, Isopropyl, Methoxy, C₁-C₂-Haloalkyl mit F und/oder Cl als Halogen, CF₃O, CHF₂O, Nitro, substituiert ist.

5. Verbindungen gemäss Anspruch 4, worin der 6-gliedrige Heterocyclus unsubstituierter oder ein- bis dreifach substituiertes Pyridin ist und R₂ gleich oder verschieden Fluor, Chlor, Brom, Methyl, Methoxy, CF₃, CF₃O, CHF₂O bedeutet, mit n = 0, 1 oder 2.

6. Verbindungen gemäss Anspruch 5, worin R₂ Halogen oder C₁-C₂-Haloalkyl bedeutet.

7. Verbindungen gemäss Anspruch 6, worin der Pyridinring durch CF₃ substituiert ist.

8. Verbindungen gemäss Anspruch 4, worin der 6-gliedrige Heterocyclus Pyrimidin ist, das unsubstituiert oder mit Halogen, C₁-C₄-Alkyl, C₁-C₄-Alkoxy oder C₁-C₂-Haloalkyl substituiert ist, und R₂ Fluor, Chlor, Brom, Methyl, Methoxy, Trifluormethyl oder Trifluormethoxy bedeutet, während n = 0, 1 oder 2 ist.

9. Verbindungen gemäss Anspruch 8, worin der 6-gliedrige Heterocyclus unsubstituiertes oder halo- oder haloalkyl-substituiertes Pyrimidin ist und R₂ Fluor, Chlor, Brom, Methyl, Methoxy, CF₃ bedeutet, mit n = 0, 1 oder 2.

10. Verbindungen gemäss Anspruch 9, worin der Pyrimidinring in 4-Stellung verknüpft ist.

11. Verbindungen gemäss Anspruch 4, worin R₄ Methyl, Dimethylamino, Cyclopropyl, Cyclopentyl oder Cyclohexyl bedeutet.

12. Verbindungen gemäss Anspruch 11, worin R₄ Methyl bedeutet.

13. Verbindungen gemäss Anspruch 11, worin R₄ N,N-Dimethylamino bedeutet.

14. Verbindungen gemäss Anspruch 2, worin R₃ Cyano oder C(S)NH₂ bedeutet.

15. Verbindungen gemäss Anspruch 2, worin XR₁ die Stellung 5/6 einnimmt.

16. Verbindungen gemäss Anspruch 2, worin R₁ einen unsubstituierten oder substituierten Pyrrol-, Thiophen-, Thiazol-, Isothiazol-, Imidazol- oder Pyrazol-Ring bedeutet.

17. Verbindungen gemäss Anspruch 16, worin der 5-gliedrige Heterocyclus durch Halogen und/oder Methyl substituiert ist.

18. Verbindungen gemäss Anspruch 16, worin R₂ gleich oder verschieden Halogen, Methyl, Methoxy, CF₃, CF₃O, CHF₂O bedeutet, mit n = 0, 1 oder 2.

19. Verbindungen gemäss Anspruch 2, worin X Sauerstoff bedeutet.

20. Verbindungen gemäss Anspruch 2, worin X Schwefel bedeutet.

21. Verbindungen der Formel II' 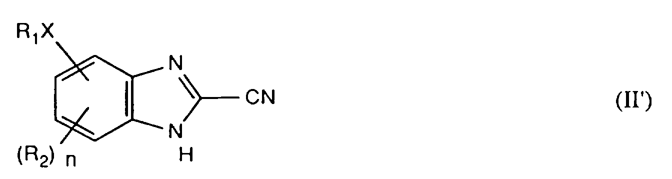 worin R₁, R₂, X und n die Bedeutungen gemäss Anspruch 2 haben.

22. Verfahren zur Herstellung von Verbindungen der Formel I gemäss Anspruch 2 durch Reaktion einer Verbindung der Formel II bei -30°C bis +180°C 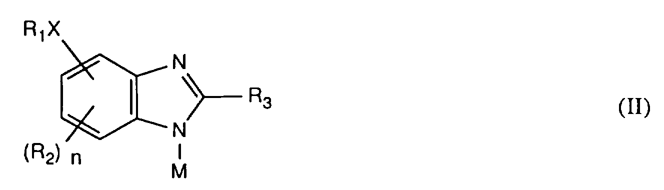 mit einer Verbindung der Formel III
Q-SO₂-R₄ (III)
worin R₁, R₂, R₃, X und n die für Formel I angegebenen Bedeutungen besitzen und M Wasserstoff oder ein Alkalimetall ist, während Q ein Halogenatom oder den Rest O-SO₂-R₄ darstellt, in einem inerten Lösungsmittel in Anwesenheit oder Abwesenheit einer Base.

23. Mittel zur Bekämpfung und Verhütung eines Befalls von Pflanzen durch Mikroorganismen, dadurch gekennzeichnet, dass es als Wirkstoff eine Verbindung der Formel I gemäss Anspruch 2 zusammen mit einem geeigneten Trägermaterial enthält.

24. Mittel gemäss Anspruch 23, dadurch gekennzeichnet, dass es als Wirkstoff eine Verbindung gemäss Anspruch 3 enthält.

25. Mittel gemäss Anspruch 23, dadurch gekennzeichnet, dass es als Wirkstoff eine Verbindung gemäss einem der Ansprüche 4 bis 19 enthält.

26. Verfahren zur Bekämpfung und Verhütung eines Befalls von Pflanzen durch Mikroorganismen, dadurch gekennzeichnet, dass man eine Verbindung der Formel I auf die Pflanze, auf Pflanzenteile oder auf den Nährboden der Pflanze appliziert.

27. Verwendung einer Verbindung gemäss Anspruch 2 als Mikrobizid.

## Claims

1. A benzimidazolesulfonic acid derivative of the formula I 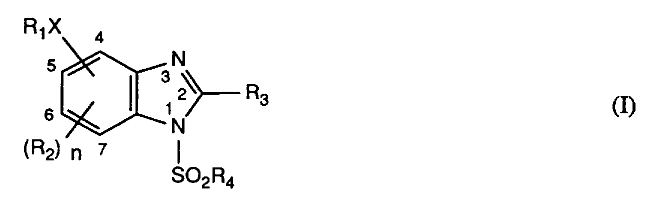 in which the R₄SO₂ group occupies the 1- or 3-position and, relative to the substituents R₁X- and R₂, forms pure or mixed positional isomers, and in which the substituents are defined as follows:
R₁ is an unsaturated 5-membered heterocycle having not more than two hetero atoms N and/or S, or an unsaturated 6-membered heterocycle having not more than two N atoms, it being possible for each of the heterocycles to be unsubstituted or substituted by at least one of the substituents halogen, C₁-C₃alkyl, C₁-C₃alkoxy, C₁-C₃haloalkyl, C₁-C₃haloalkoxy, COCC₁-C₃alkyl, C₃-C₆cycloalkyl, CN and nitro;
R₂ radicals, identical or different, are halogen, C₁-C₃alkyl, C₁-C₃alkoxy, C₁-C₃haloalkyl, C₁-C₃haloalkoxy, nitro;
R₃ is cyano, -CS-NH₂ or -C(SR')=NH, where R' is C₁-C₄alkyl, C₁-C₄haloalkyl, or benzyl which is unsubstituted or substituted by halogen and/or CF₃;
R₄ is C₁-C₄alkyl, C₃-C₆cycloalkyl, -N(R")(R"'), in which R" and R"' are identical or different C₁-C₃alkyl radicals;
X is oxygen or sulfur; and
n is an integer 0, 1 or 2.

2. A benzimidazolesulfonic acid derivative of the formula I according to claim 1, in which the R₄SO₂ group is in the 1- or 3-position and, relative to the substituents R₁X- and R₂, forms pure or mixed positional isomers, and in which the substituents are defined as follows:
R₁ is an unsaturated 5-membered heterocycle having not more than two hetero atoms N and/or S, or an unsaturated 6-membered heterocycle having not more than two N atoms, it being possible for each of the heterocycles to be unsubstituted or substituted by at least one of the substituents halogen, C₁-C₃alkyl, C₁-C₃alkoxy, C₁-C₃haloalkyl, C₁-C₃haloalkoxy, and nitro;
R₂ radicals, identical or different, are halogen, C₁-C₃alkyl, C₁-C₃alkoxy, C₁-C₃haloalkyl, C₁-C₃haloalkoxy, nitro;
R₃ is cyano, -CS-NH₂ or -C(SR')=NH, where R' is C₁-C₄aAyl, C₁-C₄haloalkyl, or benzyl which is unsubstituted or substituted by halogen and/or CF₃;
R₄ is C₁-C₄alkyl, C₃-C₆cycloalkyl, -N(R")(R"'), in which R" and R"' are identical or different C₁-C₃alkyl radicals;
X is oxygen or sulfur; and
n is an integer 0, 1 or 2.

3. A compound according to claim 2, in which R₁ is an unsubstituted or substituted pyridine, pyrimidine, pyrazine or pyridazine ring.

4. A compound according to claim 3, in which the 6-membered heterocycle is substituted by one to three substituents selected from amongst halogen, methyl, ethyl, isopropyl, methoxy, C₁-C₂halodkyl where halogen is F and/or Cl, CF₃O, CHF₂O or nitro.

5. A compound according to claim 4, in which the 6-membered heterocycle is unsubstituted or mono- to trisubstituted pyridine and R₂ radicals, identical or different, are fluorine, chlorine, bromine, methyl, methoxy, CF₃, CF₃O or CHF₂O, where n = 0, 1 or 2.

6. A compound according to claim 5, in which R₂ is halogen or C₁-C₂haloalkyl.

7. A compound according to claim 6, in which the pyridine ring is substituted by CF₃.

8. A compound according to claim 4, in which the 6-membered heterocycle is pyrimidine which is unsubstituted or substituted by halogen, C₁-C₄alkyl, C₁-C₄alkoxy or C₁-C₂haloalkyl, and R₂ is fluorine, chlorine, bromine, methyl, methoxy, trifluoromethyl or trifluoromethoxy, while n is 0, 1 or 2.

9. A compound according to claim 8, in which the 6-membered heterocycle is unsubstituted or halo- or haloalkyl-substituted pyrimidine and R₂ is fluorine, chlorine, bromine, methyl, methoxy or CF₃, n being 0, 1 or 2.

10. A compound according to claim 9, in which the pyrimidine ring is linked in the 4-position.

11. A compound according to claim 4, in which R₄ is methyl, dimethylamino, cyclopropyl, cyclopentyl or cyclohexyl.

12. A compound according to claim 11, in which R₄ is methyl.

13. A compound according to claim 11, in which R₄ is N,N-dimethylamino.

14. A compound according to claim 2, in which R₃ is cyano or C(S)NH₂.

15. A compound according to claim 2, in which XR₁ is in the 5/6-position.

16. A compound according to claim 2, in which R₁ is an unsubstituted or substituted pyrole, thiophene, thiazole, isothiazole, imidazole or pyrazole ring.

17. A compound according to claim 16, in which the 5-membered heterocycle is substituted by halogen and/or methyl.

18. A compound according to claim 16, in which R₂ radicals, identical or different, are halogen, methyl, methoxy, CF₃, CF₃O or CHF₂O, n being 0, 1 or 2.

19. A compound according to claim 2, in which X is oxygen.

20. A compound according to claim 2, in which X is sulfur.

21. A compound of the formula II' 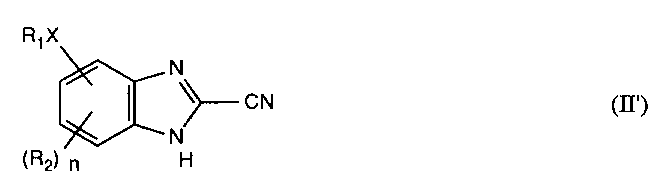 in which R₁, R₂, X and n are as defined in claim 2.

22. A process for the preparation of a compound of the formula I according to claim 2, by reacting a compound of the formula II at -30°C to +180°C 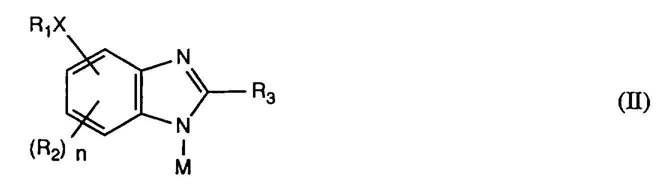 with a compound of the formula III
Q-SO₂-R₄ (III)
in which R₁, R₂, R₃, X and n are as defined for formula I and M is hydrogen or an alkali metal while Q is a halogen atom or the radical O-SO₂-R₄, in an inert solvent in the presence or absence of a base.

23. A composition for controlling and preventing attack of plants by microorganisms, which comprises, as active ingredient, a compound of the formula I according to claim 2 together with a suitable carrier material.

24. A composition according to claim 23, which comprises, as active ingredient, a compound according to claim 3.

25. A composition according to claim 23, which comprises, as active ingredient, a compound according to one of claims 4 to 19.

26. A method of controlling and preventing attack of plants by microorganisms, which comprises applying a compound of the formula I to the plant, parts of the plant or the substrate of the plant.

27. The use of a compound according to claim 2 as microbicide.

## Revendications

1. Dérivés d'acide benzimidazole sulfonique de formule I: 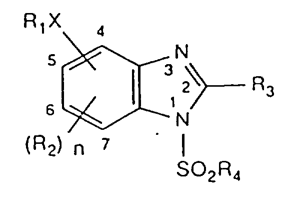 dans laquelle le groupe R₄SO₂ possède la position 1 ou 3, et forme par rapport aux substituts R₁X et R₂ des isomères de position purs ou mixtes et où les substituants ont les significations suivantes.
R₁ = hétérocycle à cinq chaînons insaturés ayant au maximum deux hétéréatomes N et/ou S, ou un hétérocycle à six chaînons saturés ayant au maximum deux atomes de N, où chacun des hétérocycles est non-substitué ou peut être substitué par au moins l'un des substituants halogènes, alkyle en C₁ à C₃, alcoxy en C₁ à C₃, haloalkyle en C₁ à C₃, haloalcoxy en C₁ à C₃, COO-alkyle en C₁ à C₃, cycloalkyle en C₃ à C₆, CN et Nitro;
R₂ identique ou différent, représente un halogène, alkyle en C₁ à C₃, alcoxy en C₁ à C₃, haloalkyle en C₁ à C₃, haloalcoxy en C₁ à C₃, nitro;
R₃ représente un cyano, -CS-NH₂ ou -C(SR)=NH, où R' représente un alkyle en C₁ à C₄, un haloalkyle en C₁ à C₄, un benzyle non-substitué ou substitué par un halogène et/ou CF₃;
R₄ représente un alkyle en C₁ à C₄, un cycloalkyle en C₃ à C₆, un -N(R")(R'''), où R" et R''' représentent des alkyles en C₁ à C₃, identiques ou différents;
X représente un oxygène ou un soufre; et
n est le nombre entier 0, 1 ou 2.

2. Dérivés d'acide benzimidazole sulfonique de formule I selon la revendication 1, dans lesquels le groupe R₄SO₂ possède la position 1 ou 3 et par rapport aux substituants R₁X et R₂ forment des isomères de position purs ou mixtes, et où les substituants ont les significations suivantes.
R₁ = hétérocycle à cinq chaînons non-saturés ayant au maximum deux hétéréatomes N et/ou S, ou un hétérocycle à six chaînons insaturés ayant au maximum deux atomes de N, où chacun des hétérocycles est non-substitué ou peut être substitué par au moins l'un des substituants halogènes, alkyle en C₁ à C₃, alcoxy en C₁ à C₃, haloalkyle en C₁ à C₃, haloalcoxy en C₁ à C₃, et Nitro;
R₂ identique ou différent, représente un halogène, alkyle en C₁ à C₃, alcoxy en C₁ à C₃, haloalkyle en C₁ à C₃, haloalcoxy en C₁ à C₃, nitro;
R₃ représente un cyano, -CS-NH₂ ou -C(SR)=NH , où R' représente un alkyle en C₁ à C₄, un haloalkyle en C₁ à C₄, un benzyle non-substitué ou substitué par un halogène et/ou CF₃;
R₄ représente un alkyle en C₁ à C₄, un cycloalkyle en C₃ à C₆, un -N(R")(R'''), où R" et R''' représentent des alkyles en C₁ à C₃, identiques ou différents;
X représente un oxygène ou un soufre; et
n est le nombre entier 0, 1 ou 2.

3. Composés selon la revendication 2, où R₁ représente un noyau pyridine, pyrimidine, pyrazine ou pyridazine non substituée ou substituée.

4. Composés selon la revendication 3, où l'hétérocycle à six chaînons est substitué par 3 substituants parmi halogène, méthyle, éthyle, isopropyle, méthoxy, haloalkyle en C₁ à C₂, avec F et/ou Cl comme halogène, CF₃O, CHF₂O, nitro.

5. Composés selon la revendication 4, où l'hétérocycle à six chaînons est une pyridine non-substituée ou mono-à trisubstituée, R₂ représente de façon identique ou différente les substituants fluor, chlore, brome, méthyle, méthoxy, CF₃, CF₃O, CHF₂O, avec n = 0, 1 ou 2.

6. Composés selon la revendication 5, où R₂ représente un halogène ou un haloalkyle en C₁ à C₂.

7. Composés selon la revendication 6, où le noyau pyridine est substitué par CF₃.

8. Composés selon la revendication 4, où l'hétérocycle à six chaînons est une pyrimidine, qui est non substituée ou substituée par un halogène, un alkyle en C₁ à C₄, un alcoxy en C₁ à C₄, un haloalkyle en C₁ à C₂, et R₂ est un fluor, un chlore, un brome, un méthyle, un méthoxy, un trifluorométhyle ou un trifluorométhoxy, tandis que n = 0, 1 ou 2.

9. Composés selon la revendication 8, où l'hétérocycle à six chaînons est une pyrimidine substitué ou halo-, ou haloalkyle substituée et R₂ est un fluor, un chlore, un brome, un méthyle, un méthoxy, CF₃, avec n = 0, 1 ou 2.

10. Composés selon la revendication 9, où le noyau pyrimidine est relié en position 4.

11. Composés selon la revendication 4, où R₄ représente unméthyle, diméthylamino, cyclopropyle, cyclopentyle ou cyclohexyle.

12. Composés selon la revendication 11, où R₄ représente un méthyle.

13. Composés selon-la revendication 11, où R₄ représente un N,N-diméthylamino.

14. Composés selon la revendication 2, où R₃ représente un cyano ou C(S)NH₂.

15. Composés selon la revendication 2, où XR₁ est en position 5/6.

16. Composés selon la revendication 2, où R₁ représente un noyau pyrrole, thiophène, thiazol, isothiazol, imidazol, ou pyrazole non substitué ou substitué.

17. Composés selon la revendication 16, où l'hétérocycle à 5 chaînons est substitué par un halogène et/ou méthyle.

18. Composés selon la revendication 16, où R₂ représente des substituants identiques ou différents qui sont des halogène, méthyle, méthoxy, CF₃, CF₃O, CHF₂O, avec n = 0, 1 ou 2.

19. Composés selon la revendication 2, où X représente un oxygène.

20. Composés selon la revendication 2, où X représente un soufre.

21. Composés de formule II' 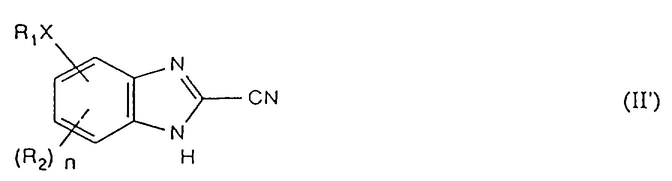 où R₁ , R₂, X et n ont la signification selon la revendication 2.

22. Procédé de préparation du composé de formule I selon la revendication 2 par réaction d'un composé de formule II entre -30°C et +180°C. 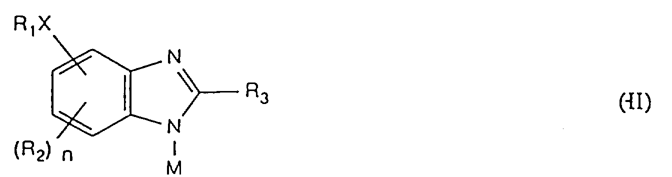 avec un composé de formule III
Q-SO₂-R₄ (III)
formules dans lesquelles R₁, R₂, R₃, X et n ont les significations données pour la revendication 1, et M est un hydrogène ou un métal alcalin, tandis que Q représente un atome d'halogène ou un radical O-SO₂-R₄, dans un solvant inerte en présence ou en l'absence d'une base.

23. Agent pour combattre ou prévenir une attaque des plantes par des microorganismes, caractérisé en ce qu'il contient comme matière active un composé de formule I selon la revendication 2, avec un support approprié.

24. Agent selon la revendication 23, caractérisé en ce qu'il contient comme matière active un composé selon la revendication 3.

25. Agent selon la revendication 23, caractérisé en ce qu'il contient comme matière active un composé selon l'une des revendications 4 à 19.

26. Procédé pour combattre ou prévenir une attaque de plantes par les microorganismes, caractérisé en ce qu'on applique un composé de formule I sur les plantes, sur des parties des plantes ou sur le sol nutritif des plantes.

27. Application d'un composé selon la revendication 2 comme microbicide.
